(19) **Europäisches Patentamt / European Patent Office / Office européen des brevets**

(11) **EP 3 218 762 B1**

(12) # EUROPÄISCHE PATENTSCHRIFT

(45) Veröffentlichungstag und Bekanntmachung des Hinweises auf die Patenterteilung:
**02.01.2019 Patentblatt 2019/01**

(21) Anmeldenummer: **15791668.5**

(22) Anmeldetag: **11.11.2015**

(51) Int Cl.:
**G02C 7/02** (2006.01)

(86) Internationale Anmeldenummer:
**PCT/EP2015/076344**

(87) Internationale Veröffentlichungsnummer:
**WO 2016/075198 (19.05.2016 Gazette 2016/20)**

(54) **OPTISCHE SEHHILFE MIT ZUSÄTZLICHEM ASTIGMATISMUS**

OPTICAL VISUAL AID WITH ADDITIONAL ASTIGMATISM

DISPOSITIF DE CORRECTION OPTIQUE POURVU D'UNE CORRECTION SUPPLÉMENTAIRE POUR L'ASTIGMATISME

(84) Benannte Vertragsstaaten:
**AL AT BE BG CH CY CZ DE DK EE ES FI FR GB GR HR HU IE IS IT LI LT LU LV MC MK MT NL NO PL PT RO RS SE SI SK SM TR**

(30) Priorität: **14.11.2014 DE 102014223341
10.04.2015 AT 502812015**

(43) Veröffentlichungstag der Anmeldung:
**20.09.2017 Patentblatt 2017/38**

(73) Patentinhaber: **Carl Zeiss Vision International GmbH
73430 Aalen (DE)**

(72) Erfinder:
 • **OHLENDORF, Arne
 72072 Tübingen (DE)**
 • **SESSNER, Rainer
 91154 Roth (DE)**

 • **KRATZER, Timo
 73434 Aalen (DE)**
 • **RIFAI, Katharina
 72072 Tübingen (DE)**
 • **LAPPE, Christian
 73557 Mutlangen (DE)**

(74) Vertreter: **Gauss, Nikolai et al
Pfiz/Gauss Patentanwälte PartmbB
Tübingerstraße 26
70178 Stuttgart (DE)**

(56) Entgegenhaltungen:
**EP-A1- 0 632 308        WO-A2-2010/083546
US-A1- 2009 210 054    US-A1- 2011 116 037**

 • **SAWUSCH, M.R. ET AL: "Optimal Astigmatism to Enhance Depth of Focus after Cataract Surgery", OPHTHALMOLOGY, Bd. 98, Nr. 7, 31. Juli 1991 (1991-07-31), Seiten 1025-1029, XP009187881,**

**Beschreibung**

Die Erfindung betrifft die Fertigung oder das computergestützte Bereitstellen oder die computergestützte Berechnung oder die computergestützte Auswahl einer optischen Sehhilfe mit wenigstens einem Brillenglas zur Verwendung von einer Beobachtungsperson für das Betrachten eines Objekts, wobei die optische Sehhilfe für wenigstens eine Blickrichtung eine auf ein Auge der Beobachtungsperson abgestimmte dioptrische Wirkung hat, die sich aus mehreren dioptrischen Wirkungskomponenten zusammensetzt. Darüber hinaus betrifft die Erfindung auch ein Verfahren für das Ermitteln einer Parametrisierung der aus mehreren dioptrischen Wirkungskomponenten zusammengesetzten dioptrischen Wirkung einer optischen Sehhilfe für ein Auge einer Beobachtungsperson, das eine auf das Auge der Beobachtungsperson abgestimmte dioptrische Wirkung hat. Außerdem betrifft die Erfindung ein Computerprogramm für das Durchführen des Verfahrens und ein System für das Bereitstellen oder die Fertigung einer optischen Sehhilfe mit wenigstens einem Brillenglas zur Verwendung von einer Beobachtungsperson für das Betrachten eines Objekts, in dem das Ermitteln einer gesuchten Parametrisierung der aus mehreren dioptrischen Wirkungskomponenten zusammengesetzten dioptrischen Wirkung einer optischen Sehhilfe erfolgen kann.

Unter einer optischen Sehhilfe versteht diese Erfindung insbesondere eine Brille mit einem vor dem Auge einer Beobachtungsperson positionierbaren Brillenglas. Eine optische Sehhilfe im Sinne der Erfindung ist jedoch auch eine vor dem Auge einer Beobachtungsperson positionierbare Anordnung von optischen Elementen, die wenigstens ein Brillenglas oder mehrere Brillengläser mit unterschiedlichen optischen Eigenschaften hat.

Unter einer dioptrischen Wirkungskomponente der dioptrischen Wirkung der Sehhilfe versteht diese Erfindung einen Beitrag der Sehhilfe für das Ausgleichen der Fehlsichtigkeit der Beobachtungsperson in Form von zumindest sphärischer Wirkung (Sphäre) und astigmatischer Wirkung (Zylinder und zugehörige Achslage).

Die sphärische Wirkung (sphärische Brechkraft) eines optischen Elements wird nachfolgend in der Einheit [D] = [1/m] (Dioptrie) angegeben, die astigmatische Wirkung (zylindrische Brechkraft) eines optischen Elements in der Einheit [DC] = [1/m] und die Achslage in Grad.

Im Sinne der Erfindung wird unter einer bestmöglich korrigierenden Wirkung einer dioptrischen Wirkungskomponente der dioptrischen Wirkung der Sehhilfe die Eigenschaft der dioptrischen Wirkungskomponente verstanden, dass der Beitrag der dioptrischen Wirkungskomponente zu der dioptrischen Wirkung der Sehhilfe insgesamt das sogenannte habituelle Refraktionsdefizit der Beobachtungsperson zumindest bis auf 1/5 D, bevorzugt bis auf 1/8 D der sphärischen Wirkung, zumindest bis auf 1/5 DC, bevorzugt bis auf 1/8 DC der astigmatischen Wirkung und $\pm$ 5° Achslage ausgleicht. Das habituelle Refraktionsdefizit kann z. B. durch eine Refraktion ermittelt werden.

Im Allgemeinen erstellt der Optiker oder Augenarzt aufgrund der Refraktion eine Verordnung (Brillenrezept) mit Rezeptwerten für eine bestmöglich korrigierende Wirkung der o.a. dioptrischen Wirkungskomponente der Sehhilfe.

Unter der teilweise korrigierenden Wirkung einer dioptrischen Wirkungskomponente der dioptrischen Wirkung der Sehhilfe versteht diese Erfindung die Eigenschaft dieser dioptrischen Wirkungskomponente, dass der Beitrag der dioptrischen Wirkungskomponente zu der dioptrischen Wirkung der Sehhilfe insgesamt das sogenannte habituelle Refraktionsdefizit der Beobachtungsperson zumindest teilweise korrigiert. Im Sinne der Erfindung teilweise korrigierend ist eine dioptrische Wirkungskomponente jedoch nur, wenn aufgrund der lediglich teilweise korrigierenden Wirkung die Sehschärfe der Beobachtungsperson um nicht mehr als 0,2 logMAR gegenüber der Sehschärfe reduziert ist, die mit einer dioptrischen Wirkungskomponente erzielt wird, die eine bestmöglich korrigierende Wirkung im Sinne der Erfindung hat.

Die Erfindung kombiniert eine oder mehrere dioptrische Wirkungskomponenten für eine bestmöglich korrigierende Wirkung und eine oder mehrere dioptrische Wirkungskomponenten für eine teilweise korrigierende Wirkung. Die teilweise korrigierende Wirkung kann dabei durch Kombination von unterschiedlichen optischen Elementen in einer Sehhilfe oder durch ein einzelnes optisches Element, z. B. ein Brillenglas mit einer dioptrischen Wirkung realisiert werden, die sich aus dioptrischen Wirkungskomponenten mit einer sphärischen Wirkung und/oder einer astigmatischen Wirkung und/oder einer prismatischen Wirkung zusammensetzt.

Damit eine Beobachtungsperson ein in einem bestimmten Entfernungsbereich angeordnetes Objekt scharf sehen kann, bedarf es einer scharfen Abbildung des Objekts auf der Netzhaut der Augen der Beobachtungsperson. Voraussetzung dafür ist, dass das auf der Netzhaut erzeugte Bild entweder exakt in einer zu der Objektebene konjugierten Bildebene liegt oder dass die Schärfentiefe der Abbildung des Objekts so groß ist, dass die mit einer Abbildung auf der Netzhaut von der zu der Objektebene konjugierten Bildebene verbundenen Unschärfe von der Beobachtungsperson nicht wahrgenommen werden kann.

Das Sehvermögen des menschlichen Auges wird auch als Visus V bezeichnet. Der Visus des menschlichen Auges ist als der in Winkelminuten gemessene Sehwinkel definiert, unter dem eine Beobachtungsperson mit dem Auge einen Gegenstand unter einem Öffnungswinkel $\alpha$ des Beobachtungsstrahlenbüschels gerade noch erkennen kann:

$$V := \alpha / 1'$$

Das Auge einer Beobachtungsperson mit Visus 1 kann also einen 1,5 mm großen Gegenstand in 5 m Entfernung gerade noch auflösen.

Für das Überprüfen der Sehschärfe finden sogenannte Sehprüfgeräte Verwendung, wie z. B. das Sehprüfgerät i.Polatest® oder das Sehprüfgerät Visuscreen 500 aus dem Hause Zeiss. Mit diesem Sehprüfgerät können den Augen einer Beobachtungsperson durch Projektion auf eine Anzeigefläche unterschiedliche Sehzeichen in Form von als ein Landolt-Ring oder als ein Snellen-E-Haken oder als Zahlen oder Buchstaben ausgebildeten Zeichen in unterschiedlichen Größen visualisiert werden. In der EP 1 880 663 A1 ist ein Sehprüfgerät mit einem Display für das Anzeigen von Sehzeichen beschrieben. Die unterschiedlichen Größen der Sehzeichen, die in den Sehprüfgeräten einer Beobachtungsperson angezeigt werden, entsprechen dabei verschiedenen Werten des Visus. Um die Sehschärfe zu überprüfen, werden die einer Beobachtungsperson angezeigten Sehzeichen solange verkleinert, bis die Beobachtungsperson die Sehzeichen nicht mehr deutlich erkennen, sondern nur noch erraten kann.

Das gesunde menschliche Auge hat die Fähigkeit, durch Verändern der Form der natürlichen Augenlinse für Objekte, die in unterschiedlichen Entfernungsbereichen liegen, eine scharfe Abbildung auf der Netzhaut sicherzustellen. Diese Fähigkeit wird als das sogenannte Akkommodationsvermögen bezeichnet. Mit zunehmendem Alter verliert das menschliche Auge die Fähigkeit, kleine Objekte in einer kurzen Objektentfernung deutlich zu erkennen. Dies liegt daran, dass die Akkommodationsfähigkeit des menschlichen Auges mit fortschreitendem Lebensalter abnimmt und deshalb für in der Nähe liegende Objekte fehlsichtig wird (Altersweitsichtigkeit).

Mittels optischer Sehhilfen können Fehlsichtigkeiten der menschlichen Augen oftmals vollständig oder zumindest teilweise korrigiert werden.

Eine Verwendung einer optischen Sehhilfe der eingangs genannten Art ist aus der EP 0 857 993 A2 bekannt. Dort ist ein als Gleitsichtglas ausgebildetes Brillenglas beschrieben, das eine auf das Auge einer Beobachtungsperson abgestimmte dioptrische Wirkung hat. Diese dioptrische Wirkung des Brillenglases setzt sich hier aus mehreren dioptrischen Wirkungskomponenten zusammen, die zu unterschiedlichen Sehzonen des Gleitsichtglases gehören. Damit wird einer Beobachtungsperson auch bei eingeschränktem Akkommodationsvermögen das scharfe Sehen in unterschiedlichen Entfernungsbereichen ermöglicht.

Die US 2009/0210054 A1 beschreibt eine optische Sehhilfe mit einem Brillenglas, das einen Astigmatismus für das Erhöhen der Schärfentiefe hat. Auch aus der US 2011/0116037 A1, der EP 0 632 308 A1 und der WO 2010/083546 A2 sind solche Brillengläser bekannt.

In der Publikation Sawusch M.R. et al., Optimal Astigmatism to Enhance Depth of Focus after Cataract Surgery, Ophtalmology 98, 1025 (1991) wird vorgeschlagen, bei Intraokularlinsen einen der sphärischen Brechkraft entsprechenden positiven Astigmatismus nach der folgenden Beziehung "Plus Zylinder = - Sphäre - 0,25" (plus cylinder = - sphere - 0,25) vorzusehen.

Aufgabe der Erfindung ist es, einer Beobachtungsperson das Beobachten von Objekten mit erhöhter Schärfentiefe zu ermöglichen und ein Verfahren und ein System für das Ermitteln einer Parametrisierung einer optischen Sehhilfe anzugeben, mit der eine Beobachtungsperson mit einer erhöhten Schärfentiefe Objekte beobachten kann.

Diese Aufgabe wird durch die in den Ansprüchen angegebene Erfindung gelöst.

Vorteilhafte Weiterbildungen der Erfindung sind in den abhängigen Ansprüchen angegeben.

Das Brillenglas einer erfindungsgemäß verwendeten optischen Sehhilfe kann z. B. als ein Multifokalglas, insbesondere als ein Gleitsichtglas ausgebildet sein. Das Brillenglas einer erfindungsgemäß verwendeten optischen Sehhilfe kann auch eine Rezeptfläche haben. Diese Rezeptfläche kann z. B. eine Brillenglasrückfläche sein.

Eine erfindungsgemäß verwendete Sehhilfe hat für wenigstens eine Blickrichtung eine auf ein Auge der Beobachtungsperson abgestimmte dioptrische Wirkung, die sich aus mehreren dioptrischen Wirkungskomponenten zusammensetzt. Dabei hat eine erste dioptrische Wirkungskomponente der mehreren dioptrischen Wirkungskomponenten für das Auge der Beobachtungsperson in einem definierten Entfernungsbereich eine für die Blickrichtung bestmöglich korrigierende Wirkung. Eine weitere dioptrische Wirkungskomponente der mehreren dioptrischen Wirkungskomponenten für das Auge der Beobachtungsperson in dem definierten Entfernungsbereich hat für die Blickrichtung eine zusätzliche astigmatische, teilweise korrigierende Wirkung. Anders ausgedrückt hat die für den Brillenträger vorgesehene Sehhilfe nicht genau die nach einem herkömmlichen Brillenrezept aufgrund einer Refraktionsbestimmung ermittelten Rezeptwerte für die sphärische Wirkung, die astigmatische Wirkung und deren Achslage und ggf. für die prismatische Wirkung und deren Basis, die eine für eine Blickrichtung bestmögliche korrigierende Wirkung bereitstellen, sondern sie hat eine zusätzliche astigmatische Wirkung mit ggf. gegenüber der Achslage nach dem Brillenrezept abweichender oder übereinstimmender Achslage für eine teilweise korrigierende Wirkung.

Die Erfinder haben nämlich erkannt, dass die natürliche Schärfentiefe des menschlichen Auges mit einer optischen Sehhilfe erhöht werden kann, die eine zusätzliche astigmatische Wirkung hat. Mit einer erfindungsgemäß verwendeten optischen Sehhilfe wird also für das Auge der Beobachtungsperson ein Astigmatismus induziert.

Unter einer Verwendung einer Sehhilfe ist vorliegend insbesondere zu verstehen, dass aus einem in virtueller oder gegenständlicher Form vorliegendem Lagerbestand die Sehhilfe ausgewählt wird, die die vorstehend angegebene dioptrische Wirkung für die Beobachtungsperson bereitstellt. Alternativ kann natürlich auch eine Sehhilfe individuell für die Beobachtungsperson berechnet und gefertigt werden. Man kann hier weiterhin von einer Rezeptfertigung sprechen, wobei die der Fertigung zugrunde gelegten Daten - wie oben beschrieben wurde - nicht genau die aus einer Refraktionsmessung bestimmten Rezeptwerte sind, sondern diese sind um die oben beschriebene zusätzliche astigmatische Wirkung mit zugehöriger Achslage für eine teilweise korrigierende Wirkung modifiziert.

Vor diesem Hintergrund haben die Erfinder insbesondere herausgefunden, dass, wenn die erste Wirkungskomponente eine das Auge der Beobachtungsperson für die Ferne bestmöglich korrigierende Wirkung und die von der ersten Wirkungskomponente verschiedene zweite Wirkungskomponente eine zusätzliche negative astigmatische Wirkung für das Auge der Beobachtungsperson hat, die natürliche Schärfentiefe des menschlichen Auges optimiert werden kann.

Insbesondere haben die Erfinder herausgefunden, dass wenn die erste Wirkungskomponente eine das Auge der Beobachtungsperson für eine Entfernung $A_S \geq 4m$ eines Objekts von dem Hornhautscheitel des Auges bestmöglich korrigierende Wirkung für die Blickrichtung hat und die weitere dioptrische Wirkungskomponente eine zusätzliche negative astigmatische Wirkung für das Auge der Beobachtungsperson für die Blickrichtung aufweist, ein besonders guter Seheindruck dadurch erzielt werden kann, indem die zusätzliche negative astigmatische Wirkung eine Zylinderbrechkraft BK ist, wobei für die Zylinderbrechkraft BK gilt: $-1,0\ DC \leq BK \leq -0,125\ DC$, vorzugsweise $-0,7\ DC \leq BK \leq -0,3\ DC$, besonders bevorzugt $BK \approx -0,5\ DC$, und wobei die zusätzliche negative astigmatische Wirkung eine in dem TABO-Schema angegebene Achslage $\varphi$ mit $70° \leq \varphi \leq 110°$, vorzugsweise $80° \leq \varphi \leq 100°$, besonders bevorzugt $\varphi \approx 90°$, oder eine in dem TABO-Schema angegebene Achslage $\varphi$ mit $-20° \leq \varphi \leq 20°$, vorzugsweise $-10° \leq \varphi \leq 10°$, besonders bevorzugt $\varphi \approx 0°$ hat.

Ein besonders guter Seheindruck kann auch dadurch erzielt werden, dass die erste dioptrische Wirkungskomponente eine das Auge der Beobachtungsperson für eine Entfernung $A_S \leq 1m$ eines Objekts von dem Hornhautscheitel des Auges bestmöglich korrigierende Wirkung für die Blickrichtung hat und die weitere dioptrische Wirkungskomponente eine zusätzliche negative astigmatische Wirkung für das Auge der Beobachtungsperson für die Blickrichtung aufweist, wobei die zusätzliche negative astigmatische Wirkung eine Zylinderbrechkraft BK mit $-1,0\ DC \leq BK \leq -0,125\ DC$, vorzugsweise $-0,7\ DC \leq BK \leq -0,3\ DC$, besonders bevorzugt $BK \approx -0,5\ DC$ und eine in dem TABO-Schema angegebene Achslage $\varphi$ mit $70° \leq \varphi \leq 110°$, vorzugsweise $80° \leq \varphi \leq 100°$, besonders bevorzugt $\varphi \approx 90°$ hat.

Die Erfinder haben außerdem herausgefunden, dass ein guter Seheindruck erzielt werden kann, indem die erste dioptrische Wirkungskomponente eine das Auge der Beobachtungsperson für eine Entfernung $A_S \leq 1m$ eines Objekts von dem Hornhautscheitel des Auges bestmöglich korrigierende Wirkung für die Blickrichtung hat und die weitere dioptrische Wirkungskomponente eine zusätzliche negative astigmatische Wirkung für das Auge der Beobachtungsperson für die Blickrichtung aufweist, wobei die zusätzliche negative astigmatische Wirkung eine Zylinderbrechkraft BK mit $-1,0\ DC \leq BK \leq -0,125\ DC$, vorzugsweise $-0,7\ DC \leq BK \leq -0,3\ DC$, besonders bevorzugt $BK \approx -0,5\ DC$ und eine in dem TABO-Schema angegebene Achslage $\varphi$ mit $-20° \leq \varphi \leq 20°$, vorzugsweise $-10° \leq \varphi \leq 10°$, besonders bevorzugt $\varphi \approx 0°$ hat. Von den Erfindern wurde nämlich festgestellt, dass sich dieser vorstehend angegebene Astigmatismus auf die von einer Beobachtungsperson wahrgenommene Schärfentiefe nicht nachteilhaft auswirkt.

Die Erfinder haben erkannt, dass sich die Addition bei Gleitsichtgläsern verringern lässt, wenn diese in der Nahbereichszone einen zusätzlichen Astigmatismus mit der vorstehend angegebenen Wirkung haben, der die Schärfentiefe erhöht, wobei die akkommodationsunterstützende Wirkung gleich bleibt.

Eine Idee der Erfindung ist es deshalb auch, dass bei der optischen Sehhilfe die erste dioptrische Wirkungskomponente in Bezug auf eine das Auge der Beobachtungsperson für eine Entfernung $25\ cm \leq A_S \leq 40\ cm$, bevorzugt $A_S \approx 33cm$ eines Objekts von dem Hornhautscheitel des Auges bestmöglich korrigierende Wirkung eine um den Wert $-1,0D \leq \Delta SBK \leq -0,1D$ reduzierte sphärische Brechkraft SBK für die Blickrichtung hat.

Bei einer erfindungsgemäßen optischen Sehhilfe kann sich die auf das Auge der Beobachtungsperson abgestimmte dioptrische Wirkung auch aus mindestens zwei ersten und zwei weiteren dioptrischen Wirkungskomponenten zusammensetzen. Eine der beiden ersten dioptrischen Wirkungskomponenten hat dann eine für das Auge der Beobachtungsperson für eine Entfernung $A_S \leq 1\ m$ eines Objekts von dem Hornhautscheitel des Auges bestmöglich korrigierende Wirkung für die Blickrichtung. Die andere der beiden ersten dioptrischen Wirkungskomponenten hat eine für das Auge der Beobachtungsperson für eine Entfernung $A_S \geq 4m$ des Objekts von dem Hornhautscheitel des Auges bestmöglich korrigierende Wirkung für eine weitere Blickrichtung. Dabei weist eine der beiden zweiten dioptrischen Wirkungskomponenten eine zusätzliche negative astigmatische, teilweise korrigierende Wirkung für das Auge der Beobachtungsperson mit einer Zylinderbrechkraft BK mit $-1,0\ DC \leq BK \leq -0,125$ DC, vorzugsweise $-0,7\ DC \leq BK \leq -0,3$ DC, besonders bevorzugt $BK \approx -0,5\ DC$ und einer in dem TABO-Schema angegebene Achslage $\varphi$ mit $70° \leq \varphi \leq 110°$, vorzugsweise $80° \leq \varphi \leq 100°$, besonders bevorzugt $\varphi \approx 90°$ für die Blickrichtung auf.

Zu bemerken ist allerdings, dass bei einer erfindungsgemäßen optischen Sehhilfe auch vorgesehen sein kann, dass sich die auf das Auge der Beobachtungsperson abgestimmte dioptrische Wirkung aus mindestens zwei ersten und zwei weiteren dioptrischen Wirkungskomponenten zusammensetzt, wobei eine der beiden ersten dioptrischen Wirkungskomponenten eine für das Auge der Beobachtungsperson für eine Entfernung $A_S \leq 1$ m eines Objekts von dem Hornhautscheitel des Auges bestmöglich korrigierende Wirkung für die Blickrichtung hat und die andere der beiden ersten dioptrischen Wirkungskomponenten eine für das Auge der Beobachtungsperson für eine Entfernung $A_S \geq 4$m des Objekts von dem Hornhautscheitel des Auges bestmöglich korrigierende Wirkung für eine weitere Blickrichtung hat und wobei eine der beiden zweiten dioptrischen Wirkungskomponenten dabei eine zusätzliche negative astigmatische, teilweise korrigierende Wirkung für das Auge der Beobachtungsperson mit einer Zylinderbrechkraft BK mit -1,0 DC $\leq$ BK $\leq$ -0,125 DC, vorzugsweise -0,7 DC $\leq$ BK $\leq$ -0,3 DC, besonders bevorzugt BK $\approx$ -0,5 DC und einer in dem TABO-Schema angegebene Achslage $\varphi$ mit -20° $\leq \varphi \leq$ 20°, vorzugsweise -10° $\leq \varphi \leq$ 10°, besonders bevorzugt $\varphi \approx$ 0° für die Blickrichtung aufweist.

Die andere der beiden zweiten dioptrischen Wirkungskomponenten bei einer vorstehend angegebenen optischen Sehhilfe hat dann eine zusätzliche negative astigmatische Wirkung für das Auge der Beobachtungsperson mit einer Zylinderbrechkraft BK mit -1,0 DC $\leq$ BK $\leq$ -0,125 DC, vorzugsweise -0,7 DC $\leq$ BK $\leq$ -0,3 DC, besonders bevorzugt BK $\approx$ -0,5 DC und mit einer in dem TABO-Schema angegebene Achslage $\varphi$ mit 70° $\leq \varphi \leq$ 110°, vorzugsweise 80° $\leq \varphi \leq$ 100°, besonders bevorzugt $\varphi \approx$ 90° für die weitere Blickrichtung oder mit einer Achslage $\varphi$ mit -20° $\leq \varphi \leq$ 20°, vorzugsweise -10° $\leq \varphi \leq$ 10°, besonders bevorzugt $\varphi \approx$ 0° für die weitere Blickrichtung.

Um die für eine Beobachtungsperson gesuchte Parametrisierung der aus mehreren dioptischen Wirkungskomponenten zusammengesetzten dioptischen Wirkung der optischen Sehhilfe zu ermitteln, schlägt die Erfindung als ein Verfahren vor, dass aus einer bestmöglichen Korrektur des Auges der Beobachtungsperson für wenigsten eine definierte Entfernung $A_S$ eines Objekts von dem Hornhautscheitel des Auges für eine Blickrichtung eine erste Parametrisierung der optischen Sehhilfe bestimmt wird. Die ermittelte erste Parametrisierung wird dann um eine zusätzliche dioptrische Wirkungskomponente korrigiert und die entsprechend korrigierte erste Parametrisierung darauf als die gesuchte Parametrisierung festgelegt.

Die erste Parametrisierung kann z. B. die Bestimmung der Rezeptwerte für die sphärische Wirkung, die astigmatische Wirkung und deren Achslage und gegebenenfalls für die prismatische Wirkung und deren Basis aus einer subjektiven und/oder objektiven Refraktionsmessung sein. Im Falle einer presbyopen Person kann die Refraktionsmessung nicht nur für einen Blick der Person in die Ferne (Fernpunktrefraktion), sondern auch oder alternativ eine oder mehrere Refraktionsmessungen bei unterschiedlichen Blickrichtungen und oder bei unterschiedlichen Objektentfernungen umfassen.

Bevorzugt ist dabei die bestmögliche Korrektur für das Auge der Beobachtungsperson eine bestmögliche Korrektur für eine Entfernung $A_S \leq$ 1m eines Objekts von dem Hornhautscheitel des Auges für die Blickrichtung, wobei die zusätzliche dioptrische Wirkungskomponente eine zusätzliche negative astigmatische Wirkung für das Auge der Beobachtungsperson aufweist, und wobei die zusätzliche negative astigmatische Wirkung eine Zylinderbrechkraft BK ist mit -1,0 DC $\leq$ BK $\leq$ -0,125 DC, vorzugsweise -0,7 DC $\leq$ BK $\leq$ -0,3 DC, besonders bevorzugt BK $\approx$ -0,5 DC und mit einer in dem TABO-Schema angegebenen Achslage $\varphi$ mit 70° $\leq \varphi \leq$ 110°, vorzugsweise 80° $\leq \varphi \leq$ 100°, besonders bevorzugt $\varphi \approx$ 90°.

Die Erfinder haben allerdings auch festgestellt, dass sich bei einer Korrektur für das Auge der Beobachtungsperson eine bestmögliche Korrektur für eine Entfernung $A_S \leq$ 1m eines Objekts von dem Hornhautscheitel des Auges für die Blickrichtung, wobei die zusätzliche dioptrische Wirkungskomponente eine zusätzliche negative astigmatische Wirkung für das Auge der Beobachtungsperson aufweist, und wobei die zusätzliche negative astigmatische Wirkung eine Zylinderbrechkraft BK ist mit -1,0 DC $\leq$ BK $\leq$ -0,125 DC, vorzugsweise -0,7 DC $\leq$ BK $\leq$ -0,3 DC, besonders bevorzugt BK $\approx$ -0,5 DC und mit einer in dem TABO-Schema angegebenen Achslage $\varphi$ mit -20° $\leq \varphi \leq$ 20°, vorzugsweise -10° $\leq \varphi \leq$ 10°, besonders bevorzugt $\varphi \approx$ 0°, die von der Beobachtungsperson wahrgenommene Schärfentiefe nicht spürbar verschlechtert. Dabei haben die Erfinder erkannt, dass sich dieser spezielle Astigmatismus bei einem Gleitsichtglas auf die Verteilung der Astigmatismen in einem Nahbereich sehr positiv auswirkt, weil damit eine Verbreiterung des Nahsehfeldes im Nahbereich verbunden ist.

Die ermittelte erste Parametrisierung kann dann auch um eine dioptrische Wirkungskomponente mit einer in Bezug auf eine das Auge der Beobachtungsperson für eine Entfernung 25 cm $\leq A_s \leq$ 40 cm, bevorzugt $A_S \approx$ 33 cm eines Objekts von dem Hornhautscheitel des Auges bestmöglich korrigierenden Wirkung für die Blickrichtung um den Wert -1,0 D $\leq \Delta$SBK $\leq$ -0,1 D reduzierten sphärischen Brechkraft SBK korrigiert werden.

Die bestmögliche Korrektur für das Auge der Beobachtungsperson kann dann zusätzlich auch eine bestmögliche Korrektur für eine Entfernung $A_S \geq$ 4 m eines Objekts von dem Hornhautscheitel des Auges für eine weitere Blickrichtung sein. Dann wird auch daraus die erste Parametrisierung der optischen Sehhilfe ermittelt und die so ermittelte erste Parametrisierung um eine zusätzliche dioptrische Wirkungskomponente korrigiert. Die korrigierte erste Parametrisierung wird dann als die gesuchte Parametrisierung festgelegt. Die zusätzliche dioptrische Wirkungskomponente für das Auge der Beobachtungsperson, um welche die erste Parametrisierung korrigiert ist, ist dabei für die weitere Blickrichtung eine negative astigmatische Wirkung mit der Zylinderbrechkraft BK mit -1,0 DC $\leq$ BK $\leq$ -0,125 DC, vorzugsweise -0,7 DC ≤ BK ≤ -0,3 DC, besonders bevorzugt BK ≈ -0,5 DC und mit einer in dem TABO-Schema angegebenen Achslage φ mit 70° ≤ φ ≤ 110°, vorzugsweise 80° ≤ φ ≤ 100°, besonders bevorzugt φ ≈ 90°, oder mit -20° ≤ φ ≤ 20°, vorzugsweise -10° ≤ φ ≤ 10°, besonders bevorzugt φ ≈ 0°.

Die bestmögliche Korrektur für das Auge der Beobachtungsperson kann auch eine bestmögliche Korrektur für eine Entfernung $A_S$ ≥ 4m eines Objekts von dem Hornhautscheitel des Auges für die Blickrichtung sein. Die zusätzliche dioptrische Wirkungskomponente kann dann eine zusätzliche negative astigmatische Wirkung für das Auge der Beobachtungsperson aufweisen, wobei die zusätzliche negative astigmatische Wirkung für die Blickrichtung eine Zylinderbrechkraft BK ist mit -1,0 DC ≤ BK ≤ -0,125 DC, vorzugsweise -0,7 DC ≤ BK ≤ -0,3 DC, besonders bevorzugt BK ≈ -0,5 DC und mit einer in dem TABO-Schema angegebenen Achslage φ mit 70° ≤ φ ≤ 110°, vorzugsweise 80° ≤ φ ≤ 100°, besonders bevorzugt φ ≈ 90°, oder mit -20° ≤ φ ≤ 20°, vorzugsweise -10° ≤ φ ≤ 10°, besonders bevorzugt φ ≈ 0°.

Die Erfindung erstreckt sich auch auf ein Computerprogramm mit Programmcodemitteln, um die Schritte der vorstehend angegebenen Verfahren durchzuführen, sowie auf einen Datenträger mit einem solchen Computerprogramm. Es ist dann ein Computer mit einem Prozessor und einem Speicher vorhanden. Das Computerprogramm mit Programmcode ist in dem Speicher abgespeichert. In dem Computer führt der Prozessor auf Basis des Programmcodes des in dem Speicher abgespeicherten Computerprogramms das erfindungsgemäße Verfahren aus.

Darüber hinaus erstreckt sich die Erfindung auch auf ein Computerprogrammprodukt mit Programmcodemitteln, die auf einem computerlesbaren Datenspeicher abgespeichert sind, um die Schritte der vorgenannten Verfahren durchzuführen. Außerdem erstreckt sich die Erfindung auf ein Computerprogrammprodukt, welches über das Internet oder vergleichbare Netze unabhängig vom Ort des Erfassens der bestmöglichen Korrektur des Auges der Beobachtungsperson die Schritte des vorgenannten Verfahrens durchführen kann.

Ein erfindungsgemäßes System für das Ermitteln einer gesuchten Parametrisierung einer optischen Sehhilfe für ein Auge einer Beobachtungsperson kann eine Messeinrichtung für das Bestimmen einer bestmöglichen Korrektur des auf eine vorgegebene Entfernung ($A_S$) akkommodierten Auges haben. Ein solches erfindungsgemäßes System enthält dann eine Rechnereinheit, der die mit der Messeinrichtung bestimmte bestmögliche Korrektur des auf eine vorgegebene Entfernung ($A_S$) akkommodierten Auges zuführbar ist. Die Rechnereinheit enthält ein Computerprogramm für das Ermitteln der gesuchten Parametrisierung ($P_E$) aus dem zugeführten bestmöglichen Korrektur mit einem vorstehend angegebenen Verfahren.

Ein erfindungsgemäßes System für das Ermitteln einer gesuchten Parametrisierung einer optischen Sehhilfe für ein Auge einer Beobachtungsperson kann auch eine Einrichtung für das Anzeigen von Sehzeichen in unterschiedlichen Entfernungen $A_S$ von dem Hornhautscheitel des Auges der Beobachtungsperson mit einer Einrichtung für das bestmögliche Korrigieren des Auges der Beobachtungsperson in den unterschiedlichen Entfernungen $A_S$ enthalten. Ein solches System enthält auch eine Messeinrichtung für das Bestimmen der Entfernung $A_S$ von der Beobachtungsperson angezeigten Sehzeichen zu dem Hornhautscheitel des Auges der Beobachtungsperson. Bevorzugt gibt es in dem System ein OLED-Display für das Anzeigen von unterschiedlich großen Sehzeichen für das Bestimmen der Sehstärke des Auges der Beobachtungsperson. Von Vorteil ist es, wenn das System ein Display hat, das die Sehzeichen in Form von zu Wörtern oder Sätzen aneinandergereihten Buchstaben anzeigt. Insbesondere ist es von Vorteil, wenn das System ein von der Beobachtungsperson betätigbares Schaltelement aufweist, das mit einer Rechnereinheit wirkungsgekoppelt ist und das für das Erzeugen eines der Rechnereinheit zugeführten Informationssignals über die von der Beobachtungsperson wahrgenommene Schärfentiefe (ST) dient.

Die Erfindung betrifft insbesondere auch die Verwendung einer optischen Sehhilfe (6) mit wenigstens einem Brillenglas (10) von einer Beobachtungsperson (28) für das Betrachten eines Objekts (15), wobei die optische Sehhilfe (6) für wenigstens eine Blickrichtung (A, B) eine auf ein Auge (11, 11') der Beobachtungsperson (28) abgestimmte dioptrische Wirkung hat, die sich aus mehreren dioptrischen Wirkungskomponenten ($K_1$, $K_2$, $K_3$, $K_4$) zusammensetzt, die **dadurch gekennzeichnet** ist, dass

eine erste dioptrische Wirkungskomponente ($K_1$, $K_3$) der mehreren dioptrischen Wirkungskomponenten ($K_1$, $K_2$, $K_3$, $K_4$) für das Auge (11, 11') der Beobachtungsperson (28) in einer definierten Entfernung $A_S$ des Objekts (15) von dem Hornhautscheitel des Auges (11, 11') eine für die Blickrichtung (A, B) bestmöglich korrigierende Wirkung; und

eine weitere dioptrische Wirkungskomponente ($K_2$, $K_4$) der mehreren dioptrischen Wirkungskomponenten ($K_1$, $K_2$, $K_3$, $K_4$) für das Auge (11, 11') der Beobachtungsperson (28) in der definierten Entfernung $A_S$ eine für die Blickrichtung (A, B) zusätzliche astigmatische, teilweise korrigierende Wirkung hat,

wobei unter der bestmöglich korrigierenden Wirkung einer dioptrischen Wirkungskomponente der dioptrischen Wirkung der Sehhilfe die Eigenschaft der dioptrischen Wirkungskomponente verstanden wird, dass der Beitrag der dioptrischen Wirkungskomponente zu der dioptrischen Wirkung der Sehhilfe insgesamt das sogenannte habituelle Refraktionsdefizit der Beobachtungsperson zumindest bis auf 1/5 D oder bis auf 1/8 D der sphärischen Wirkung und zumindest bis auf 1/5 DC oder bis auf 1/8 DC der astigmatischen Wirkung und ± 5° Achslage ausgleicht, und

wobei unter der teilweise korrigierenden Wirkung einer dioptrischen Wirkungskomponente der dioptrischen Wirkung der Sehhilfe die Eigenschaft dieser dioptrischen Wirkungskomponente verstanden wird, dass der Beitrag der dioptrischen Wirkungskomponente zu der dioptrischen Wirkung der Sehhilfe insgesamt das sogenannte habituelle Refraktionsdefizit der Beobachtungsperson zumindest teilweise korrigiert, wobei aufgrund der lediglich teilweise korrigierenden Wirkung die Sehschärfe der Beobachtungsperson um nicht mehr als 0,2 logMAR gegenüber der Sehschärfe reduziert ist, die mit einer dioptrischen Wirkungskomponente erzielt wird, die eine bestmöglich korrigierende Wirkung hat.

Diese Verwendung kann **dadurch gekennzeichnet** sein, dass die erste dioptrische Wirkungskomponente ($K_1$) eine das Auge (11, 11') der Beobachtungsperson (28) für eine Entfernung $A_S \geq 4m$ des Objekts (15) von dem Hornhautscheitel des Auges (11, 11') bestmöglich korrigierende Wirkung für die Blickrichtung (A) hat und die weitere dioptrische Wirkungskomponente ($K_2$) eine zusätzliche negative astigmatische Wirkung für das Auge (11, 11') der Beobachtungsperson (28) für die Blickrichtung (A) aufweist, wobei die zusätzliche negative astigmatische Wirkung eine Zylinderbrechkraft BK ist mit $-1,0 \, DC \leq BK \leq -0,125 \, DC$ oder $-0,7 \, DC \leq BK \leq -0,3 \, DC$ oder $BK \approx -0,5 \, DC$ und mit einer in dem TABO-Schema angegebenen Achslage $\varphi$ mit $70° \leq \varphi \leq 110°$ oder $80° \leq \varphi \leq 100°$ oder $\varphi \approx 90°$, oder mit einer in dem TABO-Schema angegebenen Achslage $\varphi$ mit $-20° \leq \varphi \leq 20°$ oder $-10° \leq \varphi \leq 10°$ oder $\varphi \approx 0°$.

Diese Verwendung kann auch **dadurch gekennzeichnet** sein, dass die erste dioptrische Wirkungskomponente ($K_3$) eine das Auge (11, 11') der Beobachtungsperson (28) für eine Entfernung $A_S \leq 1m$ des Objekts (15) von dem Hornhautscheitel des Auges (11, 11') bestmöglich korrigierende Wirkung für die Blickrichtung (B) hat und die weitere dioptrische Wirkungskomponente ($K_4$) eine zusätzliche negative astigmatische Wirkung für das Auge (11, 11') der Beobachtungsperson (28) für die Blickrichtung (B) aufweist, wobei die zusätzliche negative astigmatische Wirkung eine Zylinderbrechkraft BK mit $-1,0 \, DC \leq BK \leq -0,125 \, DC$ oder $-0,7 \, DC \leq BK \leq -0,3 \, DC$ oder $BK \approx -0,5 \, DC$ und eine in dem TABO-Schema angegebene Achslage $\varphi$ mit $-20° \leq \varphi \leq 20°$ oder $-10° \leq \varphi \leq 10°$ oder $\varphi \approx 0°$ hat.

In einer Weiterbildung kann diese Verwendung **dadurch gekennzeichnet** sein, dass die erste dioptrische Wirkungskomponente ($K_3$) eine in Bezug auf eine das Auge (11, 11') der Beobachtungsperson (28) für eine Entfernung $25 \, cm \leq A_S \leq 40 \, cm$ oder $A_S \approx 33 \, cm$ eines Objekts (15) von dem Hornhautscheitel des Auges (11, 11') bestmöglich korrigierende Wirkung um den Wert $-1,0 \, D \leq \Delta SBK \leq -0,1 \, D$ reduzierte sphärische Brechkraft SBK für die Blickrichtung (B) hat.

Die Verwendung kann auch **dadurch gekennzeichnet** sein, dass sich die auf das Auge (11, 11') der Beobachtungsperson (28) abgestimmte dioptrische Wirkung aus mindestens zwei ersten und zwei weiteren dioptrischen Wirkungskomponenten ($K_1$, $K_2$, $K_3$, $K_4$) zusammensetzt, wobei eine der beiden ersten dioptrischen Wirkungskomponenten ($K_1$) eine für das Auge (11, 11') der Beobachtungsperson (28) für eine Entfernung $A_S \leq 1 \, m$ des Objekts (15) von dem Hornhautscheitel des Auges (11, 11') bestmöglich korrigierende Wirkung für die Blickrichtung (B) hat und die andere der beiden ersten dioptrischen Wirkungskomponenten ($K_3$) eine für das Auge (11, 11') der Beobachtungsperson (28) für eine Entfernung $A_S \geq 4 \, m$ des Objekts (15) von dem Hornhautscheitel des Auges (11, 11') bestmöglich korrigierende Wirkung für eine weitere Blickrichtung (A) hat, und wobei eine der beiden zweiten dioptrischen Wirkungskomponenten ($K_2$) eine zusätzliche negative astigmatische, teilweise korrigierende Wirkung für das Auge (11, 11') der Beobachtungsperson (28) mit einer Zylinderbrechkraft BK mit $-1,0 \, DC \leq BK \leq -0,125 \, DC$ oder $-0,7 \, DC \leq BK \leq -0,3 \, DC$ oder $BK \approx -0,5 \, DC$ und einer in dem TABO-Schema angegebene Achslage $\varphi$ mit $-20° \leq \varphi \leq 20°$ oder $-10° \leq \varphi \leq 10°$ oder $\varphi \approx 0°$ für die Blickrichtung (B) hat, und wobei die andere der beiden zweiten dioptrischen Wirkungskomponenten ($K_4$) eine zusätzliche negative astigmatische Wirkung für das Auge (11, 11') der Beobachtungsperson (28) mit einer Zylinderbrechkraft BK mit $-1,0 \, DC \leq BK \leq -0,125 \, DC$ oder $-0,7 \, DC \leq BK \leq -0,3 \, DC$ oder $BK = -0,5 \, DC$ und einer in dem TABO-Schema angegebene Achslage $\varphi$ mit $70° \leq \varphi \leq 110°$ oder $80° \leq \varphi \leq 100°$ oder $\varphi \approx 90°$ für die weitere Blickrichtung (A) hat.

Die Verwendung kann weiter **dadurch gekennzeichnet** sein, dass die eine der beiden ersten dioptrischen Wirkungskomponenten ($K_1$) mit der für das Auge (11, 11') für die Entfernung $A_S \leq 1m$ bestmöglich korrigierenden Wirkung eine in Bezug auf eine das Auge (11, 11') der Beobachtungsperson (28) für eine Entfernung $25 \, cm \leq A_S \leq 40 \, cm$, bevorzugt $A_S \approx 33 \, cm$ des Objekts (15) von dem Hornhautscheitel des Auges (11, 11') bestmöglich korrigierende Wirkung um den Wert $-1,0 \, D \leq \Delta SBK \leq -0,1 \, D$ reduzierte sphärische Brechkraft SBK für die Blickrichtung (B) hat.

Die Erfindung betrifft außerdem insbesondere ein Verfahren für das Ermitteln einer gesuchten Parametrisierung ($P_E$) einer optischen Sehhilfe (6) für ein Auge (11, 11') einer Beobachtungsperson (28),

das **dadurch gekennzeichnet** ist, dass

aus einer bestmöglichen Korrektur des Auges (11, 11') der Beobachtungsperson (28) für wenigstens eine definierte Entfernung $A_S$ eines Objekts (15) von dem Hornhautscheitel des Auges (11, 11') für eine Blickrichtung (A, B) eine erste Parametrisierung ($P_A$) der dioptrischen Wirkung der optischen Sehhilfe (6) entsprechend einer ersten Wirkungskomponente ($K_1$, $K_2$) bestimmt wird, die eine bestmögliche korrigierende Wirkung hat,

die ermittelte erste Parametrisierung ($P_A$) um eine zusätzliche weitere dioptrische Wirkungskomponente ($K_2$, $K_4$) korrigiert wird, die in der definierten Entfernung (A3) eine für die Blickrichtung (A, B) zusätzliche astigmatische, teilweise korrigierende Wirkung hat, und

die korrigierte erste Parametrisierung ($P_A$) als die gesuchte Parametrisierung ($P_E$) festgelegt wird,

wobei unter der bestmöglich korrigierenden Wirkung einer dioptrischen Wirkungskomponente der dioptrischen Wirkung der Sehhilfe die Eigenschaft der dioptrischen Wirkungskomponente verstanden wird, dass der Beitrag der dioptrischen Wirkungskomponente zu der dioptrischen Wirkung der Sehhilfe insgesamt das sogenannte habituelle Refraktionsdefizit der Beobachtungsperson zumindest bis auf 1/5 D oder bis auf 1/8 D der sphärischen Wirkung und zumindest bis auf 1/5 DC oder bis auf 1/8 DC der astigmatischen Wirkung und ± 5° Achslage ausgleicht, und

wobei unter der teilweise korrigierenden Wirkung einer dioptrischen Wirkungskomponente der dioptrischen Wirkung der Sehhilfe die Eigenschaft dieser dioptrischen Wirkungskomponente verstanden wird, dass der Beitrag der dioptrischen Wirkungskomponente zu der dioptrischen Wirkung der Sehhilfe insgesamt das sogenannte habituelle Refraktionsdefizit der Beobachtungsperson zumindest teilweise korrigiert, wobei aufgrund der lediglich teilweise korrigierenden Wirkung die Sehschärfe der Beobachtungsperson um nicht mehr als 0,2 logMAR gegenüber der Sehschärfe reduziert ist, die mit einer dioptrischen Wirkungskomponente erzielt wird, die eine bestmöglich korrigierende Wirkung hat.

Dieses Verfahren kann **dadurch gekennzeichnet** sein, dass die bestmögliche Korrektur für das Auge (11, 11') der Beobachtungsperson (28) für die Blickrichtung (B) eine bestmögliche Korrektur für eine Entfernung $A_S \leq 1$ m oder 25 cm $\leq A_S \leq 40$ cm oder $A_S \approx 25$ cm oder $A_S \approx 33$ cm oder $A_S \approx 40$ cm eines Objekts (15) von dem Hornhautscheitel (11, 11') des Auges ist und die zusätzliche dioptrische Wirkungskomponente ($K_4$) eine zusätzliche negative astigmatische Wirkung für das Auge (11, 11') der Beobachtungsperson (28) aufweist, wobei die zusätzliche negative astigmatische Wirkung eine Zylinderbrechkraft BK ist mit -1,0 DC $\leq$ BK $\leq$ -0,125 DC, se -0,7 DC $\leq$ BK $\leq$ -0,3 DC, besonders bevorzugt BK $\approx$ -0,5 DC und mit einer in dem TABO-Schema angegebenen Achslage $\varphi$ mit $70° \leq \varphi \leq 110°$ oder $80° \leq \varphi \leq 100°$, besonders bevorzugt $\varphi \approx 90°$, oder einer Achslage $\varphi$ mit $-20° \leq \varphi \leq 20°$ oder $-10° \leq \varphi \leq 10°$ oder $\varphi \approx 0°$.

Das Verfahren kann weiter **dadurch gekennzeichnet** sein, dass die ermittelte erste Parametrisierung ($P_A$) um eine dioptrische Wirkungskomponente mit einer in Bezug auf eine das Auge (11, 11') für eine Entfernung 25 cm $\leq A_S \leq$ 40 cm oder $A_S \approx 33$ cm eines Objekts (15) von dem Hornhautscheitel des Auges (11, 11') bestmöglich korrigierenden Wirkung um den Wert -1,0 D $\leq \Delta$SBK $\leq$ -0,1D reduzierten sphärischen Brechkraft SBK korrigiert wird.

Das Verfahren kann außerdem **dadurch gekennzeichnet** sein, dass die bestmögliche Korrektur für das Auge (11, 11') der Beobachtungsperson (28) zusätzlich eine bestmögliche Korrektur für eine Entfernung $A_S \geq 4$ m eines Objekts (15) von dem Hornhautscheitel (11, 11') des Auges für eine weitere Blickrichtung (B) ist und auch daraus die erste Parametrisierung ($P_A$) der optischen Sehhilfe (10) ermittelt wird, und die so ermittelte erste Parametrisierung ($P_A$) auch um eine zusätzliche dioptrische Wirkungskomponente ($K_3$) korrigiert wird und die korrigierte erste Parametrisierung ($P_A$) als die gesuchte Parametrisierung ($P_E$) festgelegt wird, wobei die zusätzliche dioptrische Wirkungskomponente ($K_3$) für das Auge (11, 11') der Beobachtungsperson (28) eine negative astigmatische Wirkung ist mit der Zylinderbrechkraft BK mit -1,0 DC $\leq$ BK $\leq$ -0,125 DC oder -0,7 DC $\leq$ BK $\leq$ -0,3 DC oder BK $\approx$ -0,5 DC und mit einer in dem TABO-Schema angegebenen Achslage $\varphi$ mit $70° \leq \varphi \leq 110°$ oder $80° \leq \varphi \leq 100°$ oder $\varphi \approx 90°$, oder mit $-20° \leq \varphi \leq 20°$ oder $-10° \leq \varphi \leq 10°$ oder $\varphi \approx 0°$.

Dabei kann das Verfahren auch **dadurch gekennzeichnet** sein, dass die bestmögliche Korrektur für das Auge (11, 11') der Beobachtungsperson (28) für die Blickrichtung (A) eine bestmögliche Korrektur für eine Entfernung $A_S \geq 4$ m eines Objekts (15) von dem Hornhautscheitel (11, 11') des Auges ist und die zusätzliche dioptrische Wirkungskomponente ($K_3$) eine zusätzliche negative astigmatische Wirkung für das Auge (11, 11') der Beobachtungsperson (28) aufweist, wobei die zusätzliche negative astigmatische Wirkung eine Zylinderbrechkraft BK ist mit -1,0 DC $\leq$ BK $\leq$ -0,125 DC, vorzugsweise -0,7 DC $\leq$ BK $\leq$ -0,3 DC, besonders bevorzugt BK $\approx$ -0,5 DC und mit einer in dem TABO-Schema angegebenen Achslage $\varphi$ mit $70° \leq \varphi \leq 110°$, vorzugsweise $80° \leq \varphi \leq 100°$, besonders bevorzugt $\varphi \approx 90°$.

Die Erfindung betrifft auch ein Computerprogramm mit Programmcodemitteln, um alle Schritte eines vorstehend angegebenen Verfahrens durchzuführen.

Darüber hinaus betrifft die Erfindung ein System (92) für das Ermitteln einer gesuchten Parametrisierung ($P_E$) einer optischen Sehhilfe (10) für ein Auge (11, 11') einer Beobachtungsperson (28),

mit einer Messeinrichtung (94) für das Bestimmen einer bestmöglichen Korrektur des auf eine vorgegebene Entfernung ($A_S$) akkommodierten Auges (11); und

mit einer Rechnereinheit (98), der die mit der Messeinrichtung (94) bestimmte bestmögliche Korrektur des auf eine vorgegebene Entfernung ($A_S$) akkommodierten Auges (11) zuführbar ist;

das **dadurch gekennzeichnet** ist, dass

die Rechnereinheit ein Computerprogramm für das Ermitteln der gesuchten Parametrisierung ($P_E$) aus dem zugeführten bestmöglichen Korrektur mit einem vorstehend angegebenen Computerprogramm enthält.

Die Erfindung betrifft auch ein System für das Ermitteln einer gesuchten Parametrisierung einer optischen Sehhilfe (10) für ein Auge (11, 11') einer Beobachtungsperson (28),

mit einer eine Einrichtung (30) für das bestmögliche Korrigieren des Auges (11, 11') der Beobachtungsperson (28) in den unterschiedlichen Entfernungen $A_S$; und

mit einer Messeinrichtung für das Bestimmen der Entfernung $A_S$ von der Beobachtungsperson (28) angezeigten Sehzeichen zu dem Hornhautscheitel des Auges (11, 11') der Beobachtungsperson (28).

Dieses System kann dabei **gekennzeichnet** sein **durch** ein Display (38) für das Anzeigen von unterschiedlich großen Sehzeichen (36) für das Bestimmen der Sehstärke des Auges (11, 11') der Beobachtungsperson (28) und/oder ein Sehzeichen in Form von zu Wörtern oder Sätzen aneinandergereihten Buchstaben anzeigendes Display (38) und/oder ein von der Beobachtungsperson betätigbares Schaltelement (28), das mit einer Rechnereinheit (42) wirkungsgekoppelt ist und das für das Erzeugen eines der Rechnereinheit (42) zugeführten Informationssignals über die von der Beobachtungsperson (28) wahrgenommene Schärfentiefe (ST) dient.

Nachfolgend werden vorteilhafte Ausführungsbeispiele der Erfindung beschrieben, die in den Zeichnungen schematisch dargestellt sind.

Es zeigen:

Fig. 1      ein erstes System für das für das Ermitteln einer Parametrisierung von einer optischen Sehhilfe mit einer in Bezug auf den Refraktionsausgleich in einer definierten Entfernung zusätzlichen astigmatischen Wirkung;

Fig. 2      einen Graph mit experimentellen Werten der von einer Beobachtungspersonen für verschiedene Entfernungen durch ein Brillenglas mit einer in Bezug auf den Refraktionsausgleich zusätzlichen astigmatischen Wirkung wahrgenommenen Schärfentiefe;

Fig. 3      einen Graph mit experimentellen Werten der von unterschiedlichen Beobachtungspersonen für verschiedene Entfernungen durch ein Brillenglas mit einer in Bezug auf den Refraktionsausgleich zusätzlichen astigmatischen Wirkung wahrgenommenen Schärfentiefe;

Fig. 4a      einen ersten Graph mit experimentellen Werten zu der Veränderung der von einer Beobachtungsperson wahrgenommenen Veränderung der Schärfentiefe beim Blick durch ein Brillenglas mit einem zum Refraktionsausgleich zusätzlichen Astigmatismus, wenn die Sehschärfe um 0.1logMAR reduziert wird;

Fig. 4b      einen weiteren Graph mit experimentellen Werten zu der Veränderung der von einer Beobachtungsperson wahrgenommenen Veränderung der Sehschärfe beim Blick durch ein Brillenglas mit einem zum Refraktionsausgleich zusätzlichen Astigmatismus, wenn die Schärfentiefe um 1D erhöht wird;

Fig. 5      ein zweites System für das Ermitteln einer Parametrisierung von einer optischen Sehhilfe mit einer in Bezug auf den Refraktionsausgleich in einem definierten Entfernungsbereich zusätzlichen astigmatischen Wirkung;

Fig. 6      ein drittes System für das Ermitteln einer Parametrisierung von einer optischen Sehhilfe mit einer in Bezug auf den Refraktionsausgleich in einem definierten Entfernungsbereich zusätzlichen astigmatischen Wirkung;

Fig. 7      ein auf eine Beobachtungsperson abgestimmte optische Sehhilfe mit einem Brillenglas in einer Seitenansicht mit einer zusätzlichen astigmatischen Wirkung;

Fig. 8      das Brillenglas der optischen Sehhilfe in einer Frontansicht mit einem Nahbezugspunkt und einem Fernbezugspunkt;

Fig. 9      die Achslage der zusätzlichen astigmatischen Wirkung in dem Nahbezugspunkt nach TABO-Schema;

Fig. 10      die Achslage der zusätzlichen astigmatischen Wirkung in dem Fernbezugspunkt nach TABO-Schema; und

Fig. 11a bis Fig. 11e      den Einfluss einer zusätzlichen astigmatischen Wirkung auf den Verlauf der Flächenastigmatismen bei einem Gleitsichtglas.

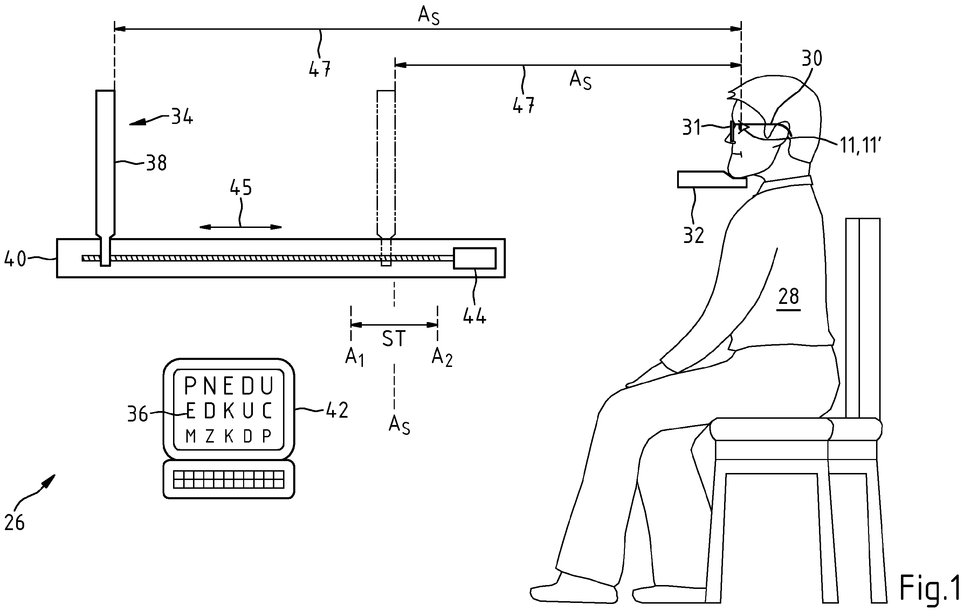

Die Fig. 1 zeigt ein erstes System 26 für das Ermitteln einer Parametrisierung von einer optischen Sehhilfe mit einer in Bezug auf den Refraktionsausgleich in eine definierten Entfernung $A_S$ eines Objekts von dem Hornhautscheitel der Augen 11, 11' einer Beobachtungsperson zusätzlichen astigmatischen Wirkung für die Augen 11, 11' der Beobachtungsperson 28.

Das System 26 umfasst eine Probierbrille 30 mit auswechselbaren Probiergläsern 31. Durch Einsetzen geeigneter Probiergläser 31 in die Probierbrille 30 kann für das linke und rechte Auge 11, 11' der Beobachtungsperson 28 eine sphärische Brechkraft und eine Zylinderbrechkraft sowie eine prismatische Wirkung eingestellt werden, um das entsprechende Auge 11, 11' bestmöglich zu korrigieren. In dem System 26 gibt es eine Kinnstütze 32, um den Kopf der Beobachtungsperson 28 damit ortsfest zu halten. Das System 26 weist weiter eine Visualisierungsvorrichtung 34 auf, mit welcher dem linken und rechten Auge 11, 11' der Beobachtungsperson 28 unterschiedliche Sehzeichen 36 an einem OLED-Display 38 zur Anzeige gebracht werden können. Das System 26 enthält eine mit dem OLED-Display 38 verbundene Rechnereinheit 42, mit dem die Art und Größe von Optotypen an dem OLED-Display 38 einstellbar ist.

Das OLED-Display 38 in dem System 26 ist an einer Schiene 40 aufgenommen. Es ist dort in Bezug auf die Kinnstütze 32 linearbeweglich geführt und kann mittels der Rechnereinheit 42 mit einem Schrittmotor 44 entsprechend dem Doppelpfeil 45 in mehreren unterschiedlichen Abständen 47 von den Augen 11, 11' der Beobachtungperson 28 positioniert werden. Für die an dem OLED-Display 38 angezeigten Sehzeichen können damit verschiedene Entfernungen $A_S$ der Optotypen von dem Hornhautscheitel der Augen 11, 11' der Beobachtungsperson angezeigt werden.

Das System 26 ermöglicht damit eine Bestimmung der Schärfentiefe ST des Seheindrucks einer Beobachtungsperson 28 für unterschiedliche Entfernungen $A_S \approx 25$ cm, $A_S \approx 33$ cm, $A_S \approx 40$ cm, $A_S \approx 55$ cm, $A_S \approx 66{,}7$ cm, $A_S \approx 100$ cm, $A_S \approx 4$ m, indem diesem an dem OLED-Display 38 entsprechende Optotypen in verschiedenen Entfernungen $A_S$ bei unterschiedlichen Parametrisierungen der Probierbrille 30 angezeigt werden. Die Schärfentiefe ST ist dabei die Differenz $ST = A_1 - A_2$ eines ersten Abstandes $A_1$ von einem zweiten Abstand $A_2 < A_1$ des OLED-Displays 38 von den Augen 11, 11' der Beobachtungsperson 28, bei denen die Beobachtungsperson auf dem OLED-Display 38 angezeigte Sehzeichen, deren Größe gegenüber dem maximalen Visus der Beobachtungsperson um 0,1 logMAR erhöht ist, wobei dann die Sehschärfe um 0.1 logMAR reduziert ist, gerade noch erkennen kann. Zu bemerken ist, dass bei gesunden Menschen der maximale Visus normalerweise mindestens den Wert 0,0 logMAR hat.

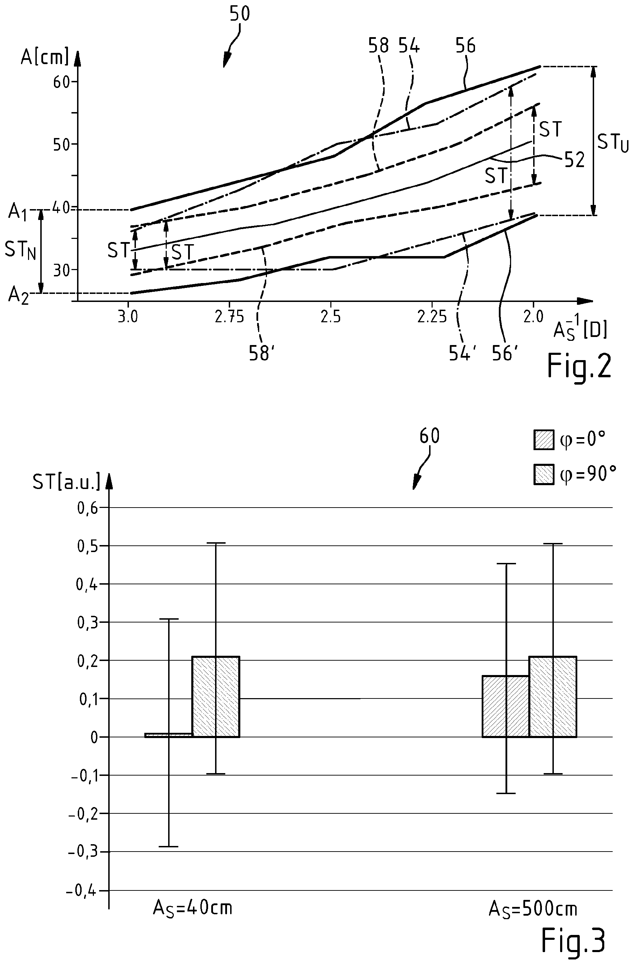

Die Fig. 2 zeigt einen Graph 50 mit experimentellen Werten für die von einer Beobachtungsperson 28 durch die Probierbrille 30 wahrgenommene Schärfentiefe ST bei unterschiedlicher sphärischer Wirkung der darin angeordneten Gläser. Für eine der Kurve 52 entsprechende Entfernung des OLED-Displays 38 von den Augen 11, 11' der Beobachtungsperson 28 bewirken die Gläser der Probierbrille 30 aufgrund ihrer Parametrisierung mit der auf der Abszisse angegebenen sphärischen Wirkung einen vollständigen Refraktionsausgleich. Die Kurven 54, 54' entsprechen dabei der von der Beobachtungsperson 28 wahrgenommene Schärfentiefe ST ohne den zusätzlichen Astigmatismus der Gläser. Die Kurven 56, 56' zeigen die von der Beobachtungsperson 28 wahrgenommene Schärfentiefe bei einer der Kurve 52 zugrundeliegenden dioptrischen Wirkung der Gläser der Probierbrille 30 mit einem zusätzlichen Astigmatismus, der die Zylinderbrechkraft -0,5 DC und die auf das TABO-Schema bezogene Achslage $\varphi = 0°$ hat. Die Kurven 58, 58' entsprechen der von der Beobachtungsperson 28 wahrgenommenen Schärfentiefe bei einer der Kurve 52 zugrundeliegenden dioptrischen Wirkung der Gläser der Probierbrille 30 mit einem zusätzlichen Astigmatismus der Zylinderbrechkraft -0,5 DC und der auf das TABO-Schema bezogenen Achslage $\varphi = 90°$.

Wie der Graph 50 zeigt, kann mit dem zusätzlichen Astigmatismus der Gläser der Probierbrille 30 die von der Beobachtungsperson 28 wahrgenommene Schärfentiefe ST in dem in der Nähe der Augen der Beobachtungsperson 28 liegenden Entfernungsbereich erhöht werden, wenn der zusätzliche Astigmatismus die Zylinderbrechkraft -0,5 DC und die auf das TABO-Schema bezogenen Achslage $\varphi = 90°$ hat. Bei großen Entfernungen A von den Augen der Beobachtungsperson 28, d. h. $A \geq 1$ m, wird die von der Beobachtungsperson 28 wahrgenommene Schärfentiefe ST mit einem zusätzlichen Astigmatismus der Probierbrille 30 mit der Zylinderbrechkraft -0,5 DC und der auf das TABO-Schema bezogenen Achslage $\varphi = 90°$ vergrößert.

Die Fig. 3 zeigt einen Graph 60 mit experimentellen Werten der von unterschiedlichen Beobachtungspersonen 28 bei den Entfernungen $A_S = 40$ cm und $A_S = 500$ cm durch eine Probierbrille 30 wahrgenommenen Schärfentiefe ST. Zusätzlich zu einer für den Refraktionsausgleich der Beobachtungspersonen 28 erforderlichen Parametrisierung wurden dabei in der Probierbrille 30 Gläser eingesetzt, die eine astigmatische Wirkung mit der Zylinderbrechkraft -0,5 DC und der auf das TABO-Schema bezogenen Achslage $\varphi = 0°$ bzw. $\varphi = 90°$ haben. Wie aus dem Graph 60 hervorgeht, ist die mit dem zusätzlichen Astigmatismus verbundene Erhöhung der Schärfentiefe ST für unterschiedliche Beobachtungspersonen zwar verschieden. Aus dem Graph 60 ergibt sich aber, dass sich im Mittel mit dem zusätzlichen Astigmatismus der Zylinderbrechkraft -0,5 DC und der Achslage $\varphi = 90°$ in der Nähe, d. h. kleine Entfernungen $A_S$, und der Achslage $\varphi = 90°$ für die Ferne, d. h. große Entfernungen $A_S$, die wahrnehmbare Schärfentiefe ST einer Beobachtungsperson erheblich vergrößert.

Mit dem System 26 kann eine für einer Beobachtungsperson 28 günstige Parametrisierung von auf die Augen 11, 11' der Beobachtungsperson 28 abgestimmten Brillengläsern 10 für das linke und/oder rechte Auge 11, 11', um die von der Beobachtungsperson wahrgenommene Schärfentiefe für eine bestimmte Blickrichtung zu erhöhen, z. B. wie folgt ermittelt werden:

Zunächst wird der Kopf der Beobachtungsperson 28 in der Kinnstütze 32 positioniert. Darauf wird das OLED-Display 38 in eine definierte Zieldistanz bewegt, in der das OLED-Display 38 einen von einer Beobachtungsperson 28 an der Rechnereinheit 42 festgelegten ersten Abstand $A_{S1} \approx 33$ cm von dem Hornhautscheitelpunkt der Augen 11, 11' hat, der in dem Entfernungsbereich Nähe liegt. Der Beobachtungsperson 28 wird dann in einem ersten Schritt die Korrektion, mit der für diesen Abstand eine bestmögliche Korrektion erreicht wird, in die Probierbrille eingesetzt. In einem zweiten Schritt wird die von der Beobachtungsperson 28 wahrgenommene Schärfentiefe für den Abstand $A_S \approx 33$ cm bestimmt, indem das OLED-Display 38 zu der Beobachtungsperson 28 hin bewegt wird, bis diese auf dem OLED-Display zur Anzeige gebrachten Sehzeichen nicht mehr erkennen kann, deren Größe gegenüber dem maximalen Visus der Beobachtungsperson um 0,1 logMAR erhöht ist, wobei dann die Sehschärfe um 0.1 logMAR reduziert ist. Der damit verbundene Verschiebeweg des OLED-Displays 38 wird dann in der Rechnereinheit 42 gespeichert. Darauf wird der zweite Schritt für Entfernungen $A_S \approx 36$ cm, $A_S \approx 40$ cm, $A_S \approx 44$ cm und $A_S \approx 50$ cm wiederholt. Auch die dabei ermittelten Werte für die Schärfentiefe werden in der Rechnereinheit 42 abgespeichert. Die bestmögliche korrigierende Korrektion der Augen der Beobachtungsperson für 33 cm mit der Probierbrille 30 wird dabei jeweils wie folgt abgeschwächt: bei der Entfernung $A_S \approx 36$ cm um 0,25 D, bei $A_S \approx 40$ cm um 0,5 D, bei $A_S \approx 44$ cm um 0,75 D und bei $A_S \approx 50$ cm um 1 D.

In einem vierten Schritt wird dann der Beobachtungsperson 28 in der Probierbrille 30 zusätzlich zu der in dem ersten Schritt ermittelten Korrektion ein Astigmatismus -0,5 DC in 0° und ein sphärisches Glas von 0,25 D in die Probierbrille eingesetzt, so dass das sphärische Äquivalent zu der in dem ersten Schritt ermittelten bestmöglichen korrigierenden Korrektion gleich bleibt.

In einem fünften Schritt werden der zweite und dritte Schritt darauf wiederholt und es werden dann in der Rechnereinheit 42 die Daten der Schärfentiefe abgespeichert. Im Anschluss daran wird der Beobachtungsperson 28 in einem sechsten Schritt neben der Korrektion aus dem ersten Schritt ein Astigmatismus -0,5 DC in 90° und ein sphärisches Glas von 0,25 D in die Probierbrille 30 eingesetzt, so dass das sphärische Äquivalent zu der bestmöglich korrigierenden Korrektion aus dem ersten Schritt gleich bleibt. Darauf werden der zweite und dritte Schritt in einem siebten Schritt wiederholt und es werden dann die Daten der Schärfentiefe wiederum in der Rechnereinheit 42 abgespeichert.

In einem achten Schritt werden die so ermittelten Daten darauf in einem dem Graphen 50 aus der Fig. 2 entsprechenden Graphen dargestellt. In einem neunten Schritt wird dann aus diesem Graphen 50 eine Additionsreduzierung der Korrektion in der Probierbrille 30 wie folgt festgelegt:

Die Addition der Korrektion wird um denjenigen Betrag reduziert, bei dem die Schärfentiefe ST des Auges 11, 11' für die Beobachtungsperson 28 eine deutliche Abbildung der Sehzeichen in einer Entfernung von $A_S \approx 33$ cm auf der Abszisse des Grafen noch ermöglicht.

Alternativ hierzu kann mit dem System 26 eine für eine Beobachtungsperson 28 günstige Parametrisierung von auf die Augen 11, 11' der Beobachtungsperson 28 abgestimmten Brillengläsern 10 auch wie folgt ermittelt werden, um die von der Beobachtungsperson wahrgenommene Schärfentiefe für das linke und/oder rechte Auge 11, 11' zu erhöhen:

Es wird zunächst der Kopf der Beobachtungsperson 28 in der Kinnstütze 32 positioniert. Darauf wird das OLED-Display 38 in eine definierte Zieldistanz bewegt, in der das OLED-Display 38 einen von einer Beobachtungsperson 28 an der Rechnereinheit 42 festgelegten ersten Abstand $A_S \approx 33$ cm von dem Hornhautscheitelpunkt der Augen 11, 11' hat, der in dem Entfernungsbereich Nähe liegt. Der Beobachtungsperson 28 wird dann in einem ersten Schritt die Korrektion, mit der für diesen Abstand eine bestmögliche Korrektion erreicht wird, in die Probierbrille eingesetzt.

Darauf werden in einem zweiten Schritt an dem OLED-Display 38 Sehzeichen in einer unterschiedlichen Größe zur Anzeige gebracht und damit die Augen 11, 11' der Beobachtungsperson 28 durch Einsetzen von unterschiedlichen optischen Gläsern 46, 48 mit einer sphärischen und/oder zylindrischen Brechkraft und/oder einer prismatischen Wirkung in die Probierbrille 30 für den Abstand $A_S$ korrigiert. Die Größe der Sehzeichen ist dabei so gewählt, dass deren Größe gegenüber dem maximalen Visus der Beobachtungsperson 28 um nicht mehr als 0,1 logMAR erhöht ist, wobei dann die Sehschärfe um 0.1 logMAR reduziert ist. Die dabei für ein Auge 11, 11' ermittelte für den Refraktionsausgleich erforderliche dioptrische Wirkung wird dann in einem Speicher der Rechnereinheit 42 als eine Ausgangsparametrisierung $P_A$ für ein auf das entsprechende Auge 11, 11' abgestimmtes Brillenglas definiert und in dem Speicher der Rechnereinheit 42 abgelegt.

In einem dritten Schritt wird dann zu optischen Gläsern 46, 48, die für ein entsprechendes Auge 11, 11' der Beobachtungsperson 28 bei einer bestimmten Entfernung A eine bestmöglich korrigierende Wirkung haben, die von der Beobachtungsperson 28 durch eine Probierbrille 30 mit diesen Gläsern 46, 48 zu der Entfernung $A_S$ wahrgenommenen Schärfentiefe ST bestimmt.

In einem vierten Schritt wird dann mit einem zusätzlichen optischen Element in der Probierbrille 30 der dioptrischen Wirkung der ermittelten Ausgangsparametrisierung $P_A$ ein Astigmatismus mit der Zylinderbrechkraft -0,5 DC und 90°-Achslage überlagert.

Darauf wird in einem fünften Schritt durch Verlagern des OLED-Displays 38 an der Schiene der Abstand A des OLED-Displays 38 von den Augen 11, 11' der Beobachtungsperson 28 variiert. Hierdurch werden die möglichen Ablagen des OLED-Displays 38 von dem Abstand $A_S \approx 5$ m bestimmt, bis zu denen die Beobachtungsperson 28 keine Veränderung ihres Seheindrucks mit einem Auge 11, 11' von an den OLED-Displays 38 zur Anzeige gebrachten Sehzeichen wahrnimmt. Auf diese Weise wird die Schärfentiefe $ST_U$ des Seheindrucks für die Ferne, d. h. den Entfernungsbereich Unendlich ermittelt.

Die ermittelte Schärfentiefe $ST_U$ wird dann als die Schärfentiefe des Seheindrucks des entsprechenden Auges 11, 11' der Beobachtungsperson 28 bei dem Entfernungsbereich Unendlich definiert und in dem Speicher der Rechnereinheit 42 abgelegt.

In einem sechsten Schritt wird das OLED-Display 38 dann in eine von der ersten Zieldistanz $A_S$ verschiedene

Zieldistanz $A_S \approx 30$ cm bewegt, in der das OLED-Display 38 einen von einer Beobachtungsperson 28 an der Rechnereinheit 42 festgelegten zweiten, dem Entfernungsbereich Nähe entsprechenden Abstand $A_S \approx 30$ cm von dem Hornhautscheitelpunkt der Augen 11, 11' der Beobachtungsperson 28 hat.

In einem siebten Schritt wird dann mit einem weiteren optischen Element in Form von einem Probierglas mit einer astigmatischen Wirkung in der Probierbrille 30 der dioptrischen Wirkung der ermittelten Ausgangsparametrisierung $P_A$ ein Astigmatismus mit der Zylinderbrechkraft -0,5 DC und 0°-Achslage oder 90°-Achslage überlagert.

Darauf wird in einem achten Schritt durch Verlagern des OLED-Displays 38 an der Schiene der Abstand A des OLED-Displays 38 von dem Hornhautscheitelpunkt der Augen 11, 11' der Beobachtungsperson 28 variiert, um durch Bestimmen der möglichen Ablagen des OLED-Displays 38 von dem Abstand $A_S \approx 30$ cm, bis zu denen die Beobachtungsperson 28 keine Veränderung ihres Seheindrucks mit einem Auge 11, 11' wahrnimmt, die Schärfentiefe dieses Seheindrucks für den Entfernungsbereich Nähe zu ermitteln.

Die ermittelte Schärfentiefe $ST_N$ wird dann als die Schärfentiefe des Seheindrucks des entsprechenden Auges 11, 11' der Beobachtungsperson 28 bei dem Entfernungsbereich Nähe definiert und in dem Speicher der Rechnereinheit 42 abgelegt.

In einem neunten Schritt wird dann als die finale Parametrisierung für ein auf das entsprechende Auge 11, 11' abgestimmtes Brillenglas wie folgt eine Parametrisierung von $P_F$ definiert: Die Parametrisierung $P_F$ ist in Bezug auf die Ausgangsparametrisierung $P_A$ zum einen um eine sphärische Wirkung für den Entfernungsbereich Unendlich korrigiert, die einem um die ermittelte Schärfentiefe $ST_U$ verringerten Objektabstand entspricht. Zum anderen ist die Ausgangs-Parametrisierung $P_A$ um eine sphärische Addition für den Entfernungsbereich Nähe korrigiert, die einem um die ermittelte Schärfentiefe $ST_N$ erhöhten Objektabstand entspricht. Darüber hinaus hat ein Brillenglas mit der finalen Parametrisierung $P_F$ in Bezug auf ein Brillenglas mit der Ausgangsparametrisierung $P_A$ einen zusätzlichen Astigmatismus mit der Zylinderbrechkraft -0,5 DC und 0°-Achslage oder 90°-Achslage für den Entfernungsbereich Nähe und einen zusätzlichen Astigmatismus mit der Zylinderbrechkraft -0,5 DC und 90°-Achslage oder 0°-Achslage für den Entfernungsbereich Unendlich.

In einem zehnten Schritt wird dann der Seheindruck der Beobachtungsperson 28 für die finale Parametrisierung $P_F$ überprüft, indem dieser entsprechende Sehzeichen mit dem OLED-Display 38 an beiden Augen 11, 11' in unterschiedlichen Entfernungsbereichen zur Anzeige gebracht werden.

Zum Bestimmten einer für eine Beobachtungsperson 28 günstige Parametrisierung $P_F$ von auf die Augen 11, 11' der Beobachtungsperson 28 abgestimmten Brillengläsern 10 für das linke und/oder rechte Auge 11, 11' in dem System 26 sind Sehzeichen in Form von zu Wörtern oder Sätzen aneinandergereihte Buchstaben von Vorteil. Die Erfinder haben herausgefunden, dass mit dieser Maßnahme auch der Einfluss des Lesevermögens berücksichtigt werden kann. Für das vorstehend beschriebene Verfahren kann damit eine hohe Reproduzierbarkeit einer für eine Beobachtungsperson 28 ermittelten Parametrisierung $P_F$ erreicht werden.

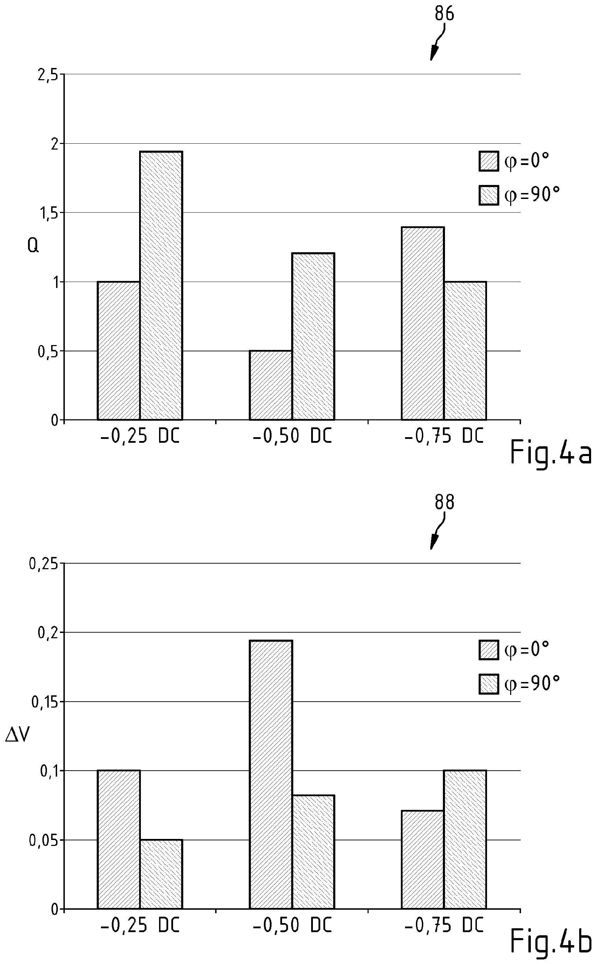

Die Fig. 4a zeigt einen ersten Graph 86 mit experimentellen Werten zu dem Verhältnis $Q := \frac{ST_M}{ST_O}$ der von einer Beobachtungsperson 28 wahrgenommenen Schärfentiefe $ST_M$ beim Beobachten eines Objekts in 500 cm Entfernung durch ein Brillenglas 10, das eine dioptrische Wirkung hat, die sich aus einer ersten und einer weiteren dioptrischen Wirkungskomponente zusammensetzt, und der von der Beobachtungsperson 28 wahrgenommenen Schärfentiefe $ST_O$ beim Beobachten des Objekts durch ein Brillenglas 10 mit einer dioptrischen Wirkung, die von der gleichen ersten dioptrischen Wirkungskomponente hervorgerufen wird und bei dem es die weitere dioptrische Wirkungskomponente nicht gibt. Die erste dioptrische Wirkungskomponente hat für die Beobachtungsperson 28 in einem definierten Entfernungsbereich eine bestmöglich korrigierende Wirkung. Die weitere dioptrische Wirkungskomponente entspricht einem Astigmatismus mit der Zylinderbrechkraft BK =-0,25 DC bzw. BK = -0,50 DC bzw. BK = -0,75 DC und der auf das TABO-Schema bezogenen Achslage $\varphi = 0°$ bzw. $\varphi = 90°$. Der Graph 86 zeigt, wie die von einer Beobachtungsperson wahrgenommene Schärfentiefe ansteigt, wenn sich bei einem zusätzlichen Astigmatismus mit der Zylinderbrechkraft BK = -0,50 DC und der auf das TABO-Schema bezogenen Achslage $\varphi = 90°$ die Sehschärfe um 0,1 logMAR verringert.

Die Fig. 4b zeigt einen weiteren Graph 88 mit experimentellen Werten zu der von einer Beobachtungsperson wahrgenommenen Veränderung $\Delta V$ des Visus V beim Beobachten eines Objekts in 500 cm Entfernung durch ein Brillenglas 10, das eine dioptrische Wirkung hat, die sich aus einer ersten und einer weiteren dioptrischen Wirkungskomponente zusammensetzt, wenn sich die Schärfentiefe um 1 D erhöht. Die erste dioptrische Wirkungskomponente hat für die Beobachtungsperson 28 in einem definierten Entfernungsbereich eine bestmöglich korrigierende Wirkung. Die weitere dioptrische Wirkungskomponente entspricht einem Astigmatismus mit der Zylinderbrechkraft BK = -0,25 DC bzw. BK = -0,50 DC bzw. BK = -0,75 DC und der auf das TABO-Schema bezogenen Achslage $\varphi = 0°$ bzw. $\varphi = 90°$. Der Graph 88 belegt, dass sich die von einer Beobachtungsperson erreichte Sehschärfe bei Erhöhung der Schärfentiefe mit einem Astigmatismus von -0,5 DC und der auf das TABO-Schema bezogenen Achslage $\varphi = 90°$ lediglich geringfügig verschlechtert.

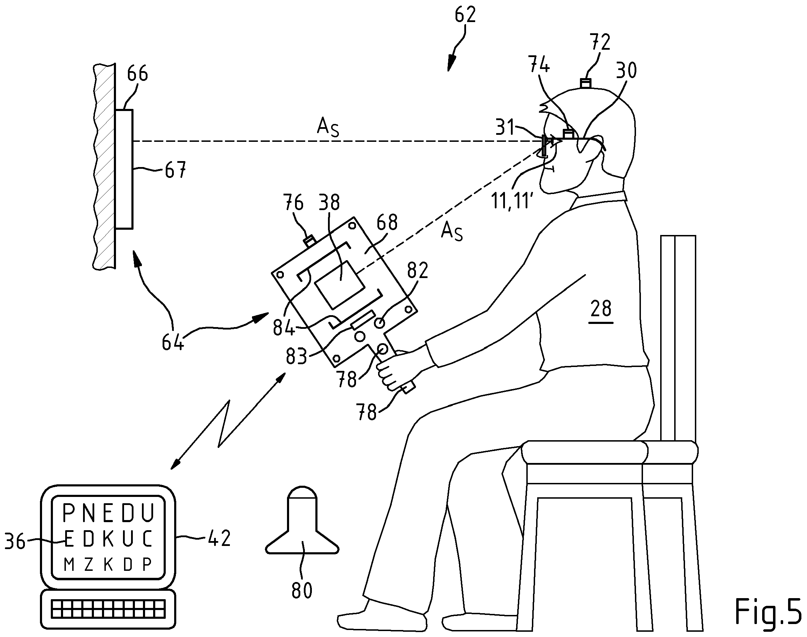

Die Fig. 5 zeigt ein zweites System 62 für das Ermitteln einer Parametrisierung von einer optischen Sehhilfe mit einer in Bezug auf den Refraktionsausgleich in einem definierten Entfernungsbereich zusätzlichen astigmatischen Wirkung für die Augen 11, 11' einer Beobachtungsperson 28.

Das System 62 umfasst ebenfalls eine Probierbrille 30. In dem System 62 gibt es eine Visualisierungsvorrichtung 64 mit einer ersten Anzeigeeinrichtung 66, die eine Anzeigefläche 67 für das Anzeigen von Sehzeichen in dem Entfernungsbereich unendlich bei einem Abstand $A_S \approx 5$ m von den Augen 11, 11' der Beobachtungsperson 28 aufweist. Die Visualisierungsvorrichtung 64 umfasst weiter eine zweite Anzeigeeinrichtung 68 mit einem OLED-Display 38 für das Anzeigen von Sehzeichen 36 in der Nähe der Augen der Beobachtungsperson 28 bei einem Abstand $A_S \approx 30$ cm. Das OLED-Display 38 hat eine Pixeldichte, die in dem Abstand $A_S \approx 30$ cm von dem Hornhautscheitelpunkt der Augen der Beobachtungsperson 28 das Anzeigen von Sehzeichen 36 in einer Größe ermöglicht, die das Bestimmen einer Sehschärfe mit dem Visus -0,3 logMAR zulässt. Das OLED-Display 38 ist deshalb z. B. ein OLED des Typs eMAgine SVGA + Rev2, das 800 x 600 Pixel hat. Die Anzeigeeinrichtung 68 in dem System 62 ist als ein Lesetafelpanel gestaltet. Die Anzeigeeinrichtung 68 hat einen Griff 70, mit dem es von der Beobachtungsperson 28 mit einer Hand gehalten werden kann. In dem System 62 gibt es einen Positionssensor 72, der an dem Kopf der Beobachtungsperson 28 angebracht ist. An der Probierbrille 30 ist ein Positionssensor 74 festgelegt. An der Anzeigeeinrichtung 68 ist ein Positionssensor 76 vorgesehen. Das System 62 enthält eine Rechnereinheit 42, die mit der Anzeigevorrichtung 66 und der Anzeigeeinrichtung 68 sowie den Positionssensoren 72, 74 und 76 drahtlos kommuniziert, z. B. mittels WLAN oder Bluetooth. An dem Griff 70 der Anzeigeeinrichtung 68 gibt es einen als Druckknopf 78 ausgebildeten Responseknopf. Der Druckknopf 78 ist mit der Rechnereinheit 42 wirkungsverbunden.

Die Rechnereinheit 42 ist eine Messeinrichtung für das Bestimmen der Entfernung $A_S$ von der Beobachtungsperson 28 angezeigten Sehzeichen zu dem Hornhautscheitel des Auges 11, 11' der Beobachtungsperson 28. Sie berechnet aus den von den Positionssensoren 72, 74, 76 übermittelten Positionssignalen die Relativposition der Anzeigefläche 67 der ersten Anzeigeeinrichtung 66 und die Relativposition des OLED-Displays 38 der zweiten Anzeigeeinrichtung 68 zu den Augen 11, 11' der Beobachtungsperson 28. Der Druckknopf 78 dient dabei für das Erfassen der Information der von der Beobachtungsperson 28 für eine bestimmte Parametrisierung der Gläser der Probierbrille 30 wahrgenommenen Schärfentiefe seines Seheindrucks von dem mit dem OLED-Display angezeigten Sehzeichen 36. Der Rechnereinheit 42 kann der Beobachtungsperson 28 hierfür mit dem Druckknopf 78 den Abstand $A_S$ kommunizieren, bei dem er bei einer bestimmten Parametrisierung der Gläser der Probierbrille 30 an dem OLED-Display 38 angezeigte Optotypen nicht mehr deutlich wahrzunehmen vermag.

Das System 62 kann grundsätzlich von der Beobachtungsperson 28 oder auch einer dritten Person bedient werden. Es ermöglicht damit berührungslos, automatisch und schnell ohne Betreuung oder Anleitung von Technikern bzw. Ingenieuren insbesondere das Vermessen der Schärfentiefe und Sehschärfe der Augen 11, 11' der Beobachtungsperson 28 im Nahbereich. Das System 62 kann z. B. von einem Augenoptiker eingesetzt werden. Es eignet sich aber auch für die Verwendung in Kliniken, Forschungsinstituten und Arztpraxen. Mit dem System 62 können die Augen einer Beobachtungsperson 28 sowohl monokular als auch binokular vermessen werden. Bevorzugt enthält das System 62 auch eine Dockingstation 80 für die Anzeigeeinrichtung 68, die z. B. für das Aufladen eines Akkumulators 83 in der Anzeigeeinrichtung 68 dient.

Es sei bemerkt, dass die Positionssensoren 72, 74, 76 in dem System 62 z. B. als Ultraschallsensoren ausgebildet sein können. Alternativ oder zusätzlich ist es auch möglich, für das Bestimmen der Relativposition der Augen der Beobachtungsperson zu der Anzeigefläche 67 der Anzeigeeinrichtung 66 und dem Display 28 der Anzeigeeinrichtung 68 optische Sensoren vorzusehen, die für das Erfassen der entsprechenden Abstände mittels Bildauswertung in der Rechnereinheit 42 ausgelegt sind. Mit den Positionssensoren 72, 74, 76 ist es möglich, beim Blick der Beobachtungsperson 28 auf das OLED-Display 38 der Anzeigeeinrichtung 68 und beim Blick der Beobachtungsperson 28 auf die Anzeigefläche 67 der Anzeigeeinrichtung 66 sowohl die Kopfposition- und Orientierung als auch Lage und Orientierung der Probierbrille 30 in einem gemeinsamen Koordinatensystem aufzunehmen. Dies ermöglicht das Erstellen von individuellen Haltungsprofilen für Beobachtungspersonen 28, wenn diese durch ein Brillenglas blicken.

Die Anzeigeeinrichtung 68 kann auch eine Kamera 82 enthalten, die unterhalb des Lesefeldes angeordnet ist und ein Erfassen von Bewegungen der Augen einer Beobachtungsperson 28 beim Lesen ermöglicht. Zu bemerken ist, dass die Kamera 82 auch oberhalb des Lesefeldes angeordnet sein kann. Hier kann der Bildsensor der Kamera 82 dann besonders vorteilhaft auch für das Bestimmen der Pupillengröße eingesetzt werden oder als sogenannter Eyetracker fungieren.

Von Vorteil ist es, wenn die Anzeigeeinrichtung 68 einen Einschubrahmen 84 für Lesetexttafeln enthält, mittels derer das reale Leseverhaltens einer Beobachtungsperson 28 ausgetestet werden kann. Die Funktion eines vorstehend angegebenen Eyetrackers ermöglicht dabei das Überprüfen des Blickverhaltens. Von Vorteil ist es auch, bei der Anzeigeeinrichtung 68 Start-Stop-Taster vorzusehen, die für das quantitative Erfassen des Lesevermögens der Beobachtungsperson 28 dienen. Günstig ist es außerdem, wenn die Anzeigeeinrichtung 68 einen Einschub für ein oder mehrere Farbfilter enthält. Damit wird es der Beobachtungsperson 28 ermöglicht, die an dem OLED-Display 38 zur Anzeige gebrachten Sehzeichen bzw. Texte durch ein Farbfilter hindurch zu betrachten.

Darüber hinaus ist zu bemerken, dass die Anzeigeeinrichtung 68 optional auch IR Beleuchtungs-LEDs aufweisen kann, die in dem Randbereichen der Anzeigeeinrichtung 68 angeordnet sind, um damit das reflexfreie Ausleuchten der Augen des Probenden zu ermöglichen.

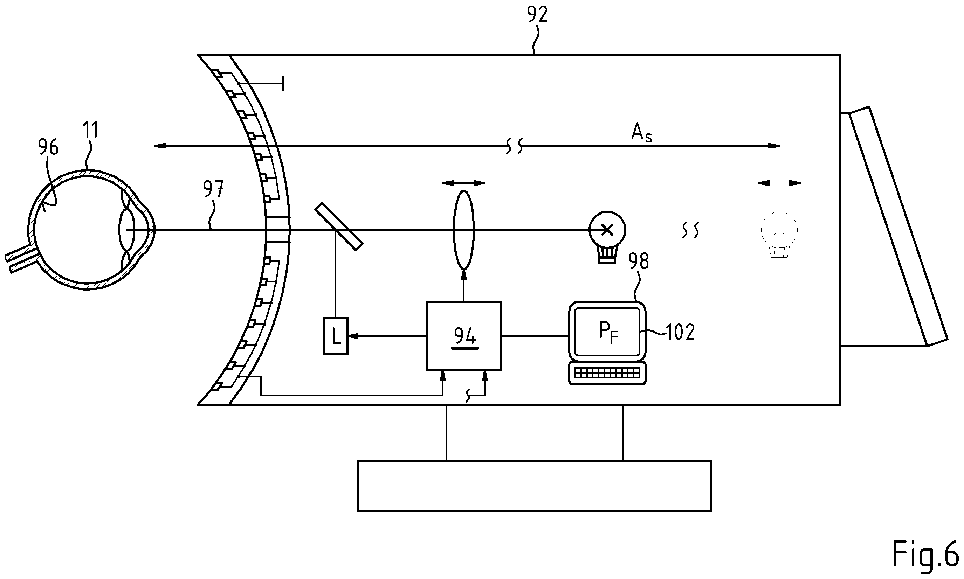

Die Fig. 6 zeigt ein drittes System 92 für das Ermitteln einer Parametrisierung von einer optischen Sehhilfe mit einer in Bezug auf den Refraktionsausgleich in einem definierten Entfernungsbereich zusätzlichen astigmatischen Wirkung für die Augen 11, 11' einer Beobachtungsperson 28 für eine Blickrichtung.

Das System 92 enthält eine Messeinrichtung 94, wie es sie z. B. in dem ophthalmologischen Messsystem i.Profiler® aus dem Hause Zeiss gibt, mit dem, wie in der DE 10 2007 032 001 B4 detailliert beschrieben ist, für das Auge 11 einer Beobachtungsperson 28 in einem gegebenen Akkommodationszustand ein Laserlichtstrahl 97 auf die Netzhaut projiziert wird. Mit einem Wellenfrontmessgerät 94 wird hier die Wellenfront des von der Netzhaut 96 reflektierten Lichts des Laserlichtstrahls 97 erfasst und daraus das objektive habituelle Refraktionsdefizit des Auges 11 bestimmt.

Die mit dem Wellenfrontmessgerät 94 gemessene Abweichung des Verlaufs der Wellenfront von einer Referenz für ein nicht mit Sehfehlern behaftetes Auge wird dann als die gesuchte Aberration, d. h. die Abweichung der Wellenfront vom idealen Fall bestimmt. Dieses Verfahren ist beispielsweise in der Dissertation von G. M. Spitzlberger "Änderung der optischen Aberrationen des menschlichen Auges durch Laser in situ Keratomileusis" aus dem Jahr 2004, auf die hiermit vollumfänglich Bezug genommen und deren Offenbarungsgehalt in die Beschreibung der Erfindung vollumfänglich mit einbezogen wird, im Einzelnen erläutert.

Es sei bemerkt, dass das System 92 auch eine Messeinrichtung 94 enthalten kann, die wie in der DE 10 2007 032 001 B4 in Absatz [0022] beschrieben ist, für das Bestimmen von Brechkraftfehlern des Auges 11 einer Beobachtungsperson 28 einen Laserstrahl bereitstellt, der auf die Netzhaut des Auges durch die Pupille hindurch auftrifft und mit dem die Netzhaut gescannt wird. Der mit dem Laserstrahl auf der Retina 96 erzeugte Lichtpunkt wird hier dann jeweils als ein Reflex auf der Makula des Auges 11 erfasst.

Mit der Messeinrichtung 94 in dem System 92 wird das habituelle Refraktionsdefizit des Auges in zwei Akkommodationszuständen bestimmt, die der Entfernung eines Objekts $A_S \approx 30$ cm und $A_S \approx 5$ m von dem Hornhautscheitel des Auges 11 entsprechen. Grundsätzlich sei bemerkt, dass mit der Messeinrichtung 94 in dem System 92 das habituelle Refraktionsdefizit des Auges 11 auch in mehr als zwei Akkommodationszuständen bestimmt werden kann, z. B. Akkommodationszuständen, die den unterschiedlichen Entfernungen $A_S \approx 25$ cm, $A_S \approx 33$ cm, $A_S \approx 40$ cm, $A_S \approx 55$ cm, $A_S \approx 66,7$ cm, $A_S \approx 100$ cm, $A_S \approx 4$ m von dem Hornhautscheitel des Auges entsprechen.

In dem System 92 gibt es eine mit der Messeinrichtung 94 verbundene Rechnereinheit 98 mit einem Computerprogramm, das aus dem der Objektdistanz $A_S \approx 30$ cm von dem Hornhautscheitelpunkt der Augen 11, 11' der Beobachtungsperson 28 entsprechenden Akkommodationszustand eine erste Parametrisierung $P_A$ als eine Ausgangsparametrisierung berechnet. Von dem Computerprogramm wird dann diese erste Parametrisierung $P_A$ um eine zusätzliche dioptrische Wirkungskomponente korrigiert, indem der Parametrisierung $P_A$ ein Astigmatismus mit der Zylinderbrechkraft -0,5 DC und 0°-Achslage oder 90°-Achslage überlagert wird. Entsprechend berechnet das Computerprogramm in der Rechnereinheit 98 aus dem der Objektdistanz $A_S \approx 5$ m von dem Hornhautscheitelpunkt der Augen 11, 11' der Beobachtungsperson 28 entsprechenden Akkommodationszustand eine weitere erste Parametrisierung $P_A$ als eine Ausgangsparametrisierung. Von dem Computerprogramm wird dann diese weitere erste Parametrisierung $P_A$ um eine zusätzliche dioptrische Wirkungskomponente korrigiert, indem der Parametrisierung $P_A$ ein Astigmatismus mit der Zylinderbrechkraft -0,5 DC und 90°-Achslage oder auch 0°-Achslage überlagert wird.

Als die gesuchte finale Parametrisierung $P_F$ der optischen Sehhilfe wird von dem Computerprogramm dann an einer Ausgabe-Schnittstelle 102 der Rechnereinheit 98 die korrigierte erste Parametrisierung $P_A$ und die korrigierte weitere Parametrisierung $P_A$ ausgegeben.

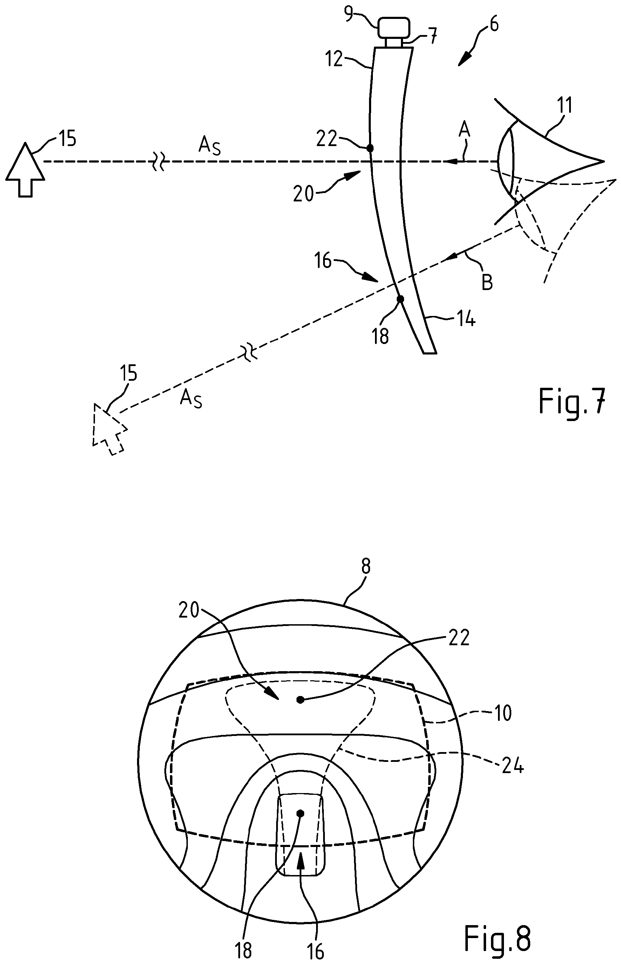

Die Fig. 7 zeigt eine als Brille ausgebildete optische Sehhilfe 6 für das Auge 11 einer Beobachtungsperson. Die optische Sehhilfe 6 enthält ein Brillenglas 10, das in einer Fassung 7 an einem Brillengestell 9 gehalten ist. Das Brillenglas 10 ist in der Fig. 7 in einer Seitenansicht gezeigt. Durch das Brillenglas 10 kann eine Beobachtungsperson ein Objekt 15 in dem Abstand $A_S$ von dem Hornhautscheitel des Auges 11 mit einer unterschiedliche Bereiche von dem Brillenglas 10 durchsetzenden Blickrichtung A, B scharf sehen. Bei dem Brillenglas 10 handelt es sich um ein Gleitsichtglas. Das Brillenglas 10 hat eine Brillenglasvorderfläche 12, die beim bestimmungsgemäßen Gebrauch einem Auge 11 einer Beobachtungsperson abgewandt ist, und sie hat eine beim bestimmungsgemäßen Gebrauch dem Auge der Beobachtungsperson zugewandte Brillenglasrückfläche 14. Die Brillenglasvorderfläche 12 ist hier als eine Gleitsichtfläche gestaltet. Die Brillenglasvorderfläche 12 hat eine Nahbereichszone 16 mit einem Nahbezugspunkt 18 und weist eine Fernbereichszone 20 mit einem Fernbezugspunkt 22 auf. Die Brillenglasrückfläche 14 ist vorliegend eine Rezeptfläche, d. h. Sphäre, Zylinder und Achslagen dieser Fläche wurden nach der Vorschrift eines entsprechend den vorstehenden Ausführungen modifizierten Brillenrezepts gefertigt.

Die Fig. 8 zeigt das Brillenglas 10 als ein Teil eines Brillenglasrohlings 8 in einer Frontansicht. Das Brillenglas 10 ist als ein linkes Brillenglas 10 für das Auge 11 der Beobachtungsperson ausgelegt. Es hat auf der Brillenglasvorderfläche 12 einen in einer Nahbereichszone 16 liegenden Nahbezugspunkt 18 und eine Fernbereichszone 20 mit einem Fernbezugspunkt 22. Zwischen der Nahbereichszone 16 und der Fernbereichszone 20 ist ein Progressionskanal 24 erstreckt.

Die dioptrische Wirkung des Brillenglases 10 lässt sich in dem Nahbezugspunkt 18 und in dem Fernbezugspunkt 22 in mehrere dioptrische Wirkungskomponenten $K_1$, $K_2$, $K_3$, $K_4$ mit einer unterschiedlichen Brechkraft $BKN_1$, $BKN_2$, $BKF_1$, $BKF_2$ zerlegen.

In dem Nahbezugspunkt 18 hat das Brillenglas 10 eine dioptrische Wirkung mit der Brechkraft $BKN = BKN_1 + BKN_2$. Dort ist die dioptrische Wirkung des Brillenglases 10 aus einer ersten dioptrischen Wirkungskomponente $K_2$ mit der sphärischen Brechkraft $BKN_1$ und einer zweiten dioptrischen Wirkungskomponente $K_4$ mit der Zylinderbrechkraft $BKN_2$ zusammengesetzt. Die erste Wirkungskomponente $K_1$ korrigiert das Auge 11 der Beobachtungsperson bei durch den Nahbezugspunkt 18 verlaufender Blickrichtung für die Nähe.

Entsprechend hat das Brillenglas in dem Fernbezugspunkt 22 eine dioptrische Wirkung mit der Brechkraft $BKF = BKF_1 + BKF_2$, die sich aus einer Wirkungskomponente $K_1$ und einer weiteren Wirkungskomponente $K_3$ zusammensetzt. Die Wirkungskomponente $K_1$ bewirkt in dem Fernbezugspunkt 22 das Korrigieren des entsprechenden Auges 11 der Beobachtungsperson für die Ferne.

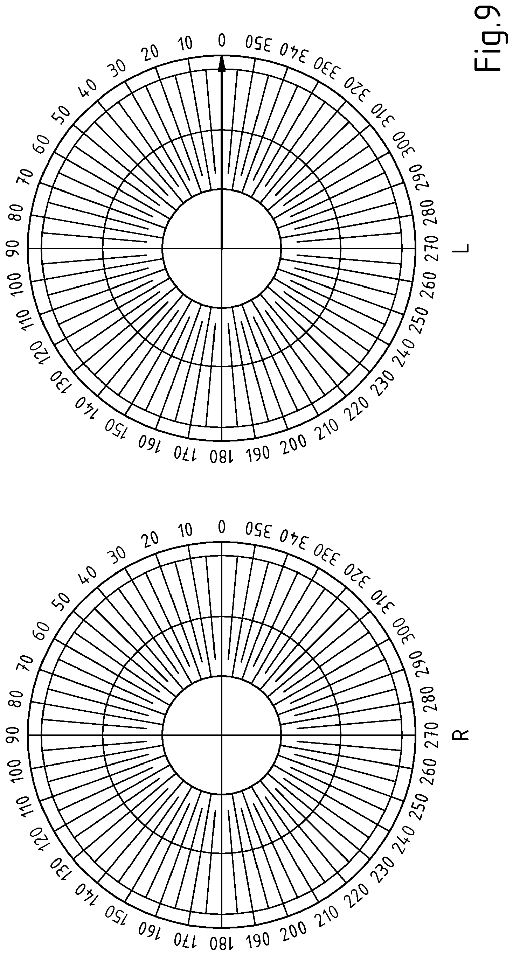

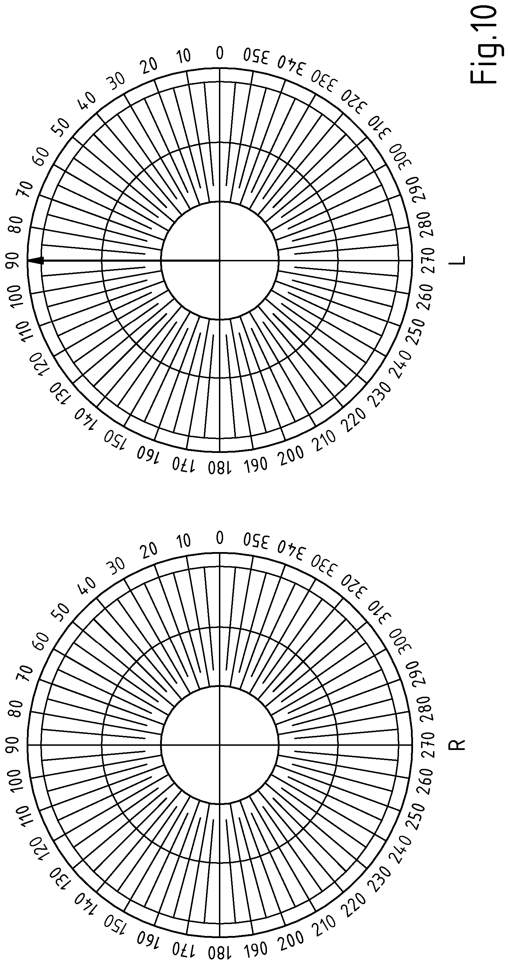

Die dioptrischen Wirkungskomponenten $K_2$, $K_4$ entsprechen jeweils einem Astigmatismus mit der Zylinderbrechkraft -0,5 DC. Die Achslage des Astigmatismus der dioptrischen Wirkungskomponenten $K_3$, $K_4$ ist allerdings verschieden. Die Fig. 9 zeigt das TABO-Schema des zusätzlichen Astigmatismus der dioptrischen Wirkungskomponente $K_4$ in dem Nahbezugspunkt 18. Die dioptrische Wirkungskomponente $K_4$ hat die Zylinderbrechkraft $BKN_2$ und die Achslage $\varphi = 0°$. In der Fig. 10 ist die Achslage des zusätzlichen Astigmatismus der dioptrischen Wirkungskomponente $K_3$ der dioptrischen Wirkung des Brillenglases 10 in dem Fernbezugspunkt 22 gezeigt. Die dioptrische Wirkungskomponente $K_3$ hat die Zylinderbrechkraft $BKF_2$ und die Achslage $\varphi = 90°$.

Die Erfinder haben erkannt, dass die von einer Beobachtungsperson wahrgenommene Schärfentiefe ST für die Nähe erhöht werden kann, indem der dioptrischen Wirkung eines das Auge 11 der Beobachtungsperson für die Nähe bestmöglich korrigierenden Brillenglases ein zusätzlicher Astigmatismus mit der Zylinderbrechkraft -0,5 DC und der auf das TABO-Schema bezogenen Achslage $\varphi = 90°$ überlagert wird.

Weiter haben die Erfinder erkannt, dass die von einer Beobachtungsperson wahrgenommene Schärfentiefe für die Ferne gesteigert werden kann, indem der dioptrischen Wirkung eines das Auge 11 der Beobachtungsperson für die Nähe bestmöglich korrigierenden Brillenglases ein zusätzlicher Astigmatismus mit der Zylinderbrechkraft -0,5 DC und der auf das TABO-Schema bezogenen Achslage $\varphi = 90°$ oder $\varphi = 0°$ überlagert wird.

Insbesondere haben die Erfinder erkannt, dass die von einer Beobachtungsperson wahrgenommene Schärfentiefe erhöht werden kann, indem der vorstehend angegebene zusätzliche Astigmatismus sowohl der dioptrischen Wirkung eines das linke Auge der Beobachtungsperson bestmöglich korrigierenden Brillenglases als auch der dioptrischen Wirkung eines das rechte Auge der Beobachtungsperson bestmöglich korrigierenden Brillenglases überlagert wird.

Die Brillenglasrückfläche 14 des Brillenglases 10, bei dem es sich um eine Rezeptfläche handelt, hat eine auf die Beobachtungsperson angepasste Sphäre, und einen Zylinder mit einer bestimmten Achslage, um den vorstehend angegebenen zusätzlichen Astigmatismus zu erzeugen. Im Allgemeinen weist die Brillenglasrückfläche 14 auch einen weiteren Zylinder mit einer bestimmten Achslage auf, um damit z. B. eine Hornhautverkrümmung auszugleichen.

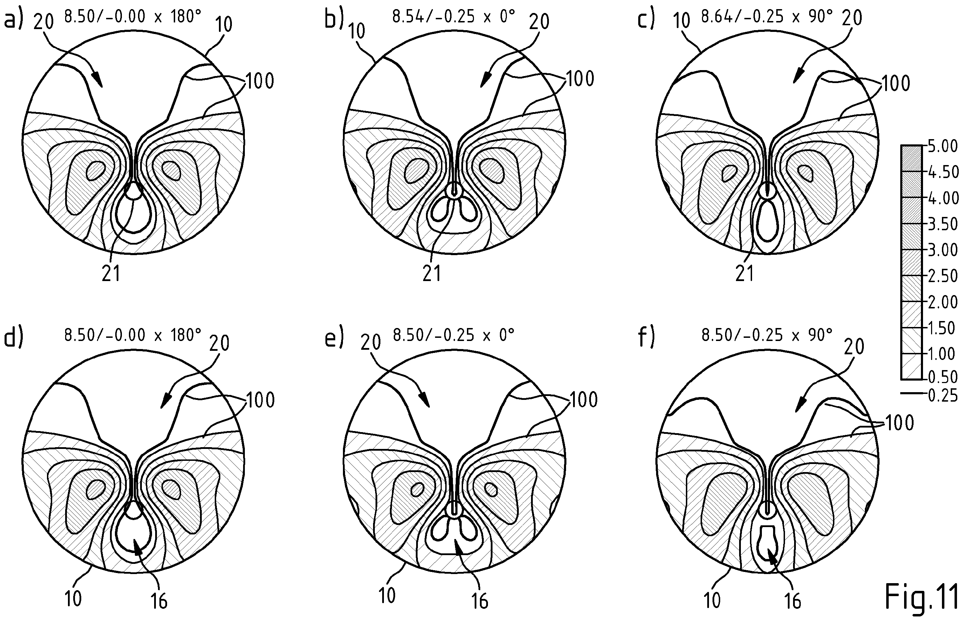

Die Fig. 11a bis Fig. 11e erläutern den Einfluss einer zusätzlichen astigmatischen Wirkung auf die Ausdehnung der Nah- und Fernbereichszone 16, 20 sowie des Progressionskanals 24 bei einem Gleitsichtglas 10.

Die Fig. 11a zeigt ein Gleitsichtglas 10, das eine auf ein Auge 11, 11' einer Beobachtungsperson 28 angepasste dioptrische Wirkung hat, die das Auge 11, 11' der Beobachtungsperson 28 bei dem Blick durch die Nahbereichszone 16 und die Fernbereichszone 20 exakt korrigiert. Der Astigmatismus des Gleitsichtglases 10 hat hier den mit den Isoastigmatismuslinien 100 angegebenen Verlauf. Die dioptrische Wirkung umfasst hier keinen zusätzlichen Astigmatismus.

Die Fig. 11b zeigt das Gleitsichtglas 10 mit einer auf das Auge 11, 11' der Beobachtungsperson 28 angepassten dioptrischen Wirkungskomponente $K_1$, $K_3$, die das Auge der Beobachtungsperson 28 bei dem Blick durch die Nahbereichszone 16 und die Fernbereichszone 20 bestmöglich korrigiert, wobei in der Nahbereichszone 16 eine weitere dioptrische Wirkungskomponente $K_4$ nämlich ein zusätzlicher negativer Astigmatismus mit der zylindrischen Brechkraft -0,25 DC und der Achslage $\varphi = 0°$ überlagert ist. Diese Maßnahme bewirkt eine vorteilhafte Verbreiterung der Nahbereichszone 16, wobei die Ausdehnung der Fernbereichszone 22 allerdings abnimmt. In der Fig. 11c ist das Gleitsichtglas 10 gezeigt, wenn der auf das Auge 11, 11' der Beobachtungsperson 28 angepassten dioptrischen Wirkungskomponente $K_2$ der dioptrischen Wirkung, die das Auge 11, 11' der Beobachtungsperson 28 bei dem Blick durch die Nahbereichszone 16 und die Fernbereichszone 20 vollständig korrigiert, in der Nahbereichszone 16 als eine weitere dioptrische Wirkungskomponente $K_4$ der zusätzliche negative Astigmatismus mit der zylindrischen Brechkraft -0,25 DC und der Achslage $\varphi = 90°$ überlagert ist. Gegenüber dem in der Fig. 11a gezeigten Gleitsichtglas 10 ist hier die Fernbereichszone 22 breiter und dafür die Nahbereichszone 16 etwas schmäler. Bei dem in der Fig. 11a, Fig. 11b und Fig. 11c gezeigten Gleitsichtglas 10 ist jeweils in dem mit der Kreislinie 21 kenntlich gemachten Gebiet die durchschnittliche dioptrisch vollkorrigierende Wirkung konstant.

Demgegenüber ist die sphärische Wirkung bei dem in Fig. 11d, Fig. 11e und Fig. 11f gezeigten Gleitsichtglas 10 in der Nahbereichszone 16 und in der Fernbereichszone 20 identisch. Bei dem Gleitsichtglas 10 in Fig. 11d ist der an eine Beobachtungsperson 28 angepassten dioptrischen Wirkungskomponente $K_2$ der dioptrischen Wirkung, die das Auge der Beobachtungsperson 28 bei dem Blick durch die Nahbereichszone 16 und die Fernbereichszone 20 bestmöglich korrigiert, kein zusätzlicher Astigmatismus überlagert.

Die Fig. 11e zeigt das Gleitsichtglas 10 mit einer der an die Beobachtungsperson 28 angepassten dioptrischen Wirkungskomponente $K_2$ der dioptrischen Wirkung als eine weitere dioptrische Wirkungskomponente $K_4$ überlagerten zusätzlichen negativen Astigmatismus mit der zylindrischen Brechkraft -0,25 DC und der Achslage $\varphi = 0°$.

Die Fig. 11f zeigt das Gleitsichtglas 10 mit einer der an die Beobachtungsperson 28 angepassten dioptrischen Wirkungskomponente $K_2$ der dioptrischen Wirkung als eine weitere dioptrische Wirkungskomponente $K_4$ überlagerten zusätzliche negativen Astigmatismus mit der zylindrischen Brechkraft -0,25 DC und der Achslage $\varphi = 90°$.

Die Fig. 11e zeigt, dass bei dem zusätzliche negativen Astigmatismus mit der Achslage $\varphi = 0°$ der Abstand zwischen den Isoastigmatismuslinien mit der zylindrischen Brechkraft +0,5 DC und +1,00 DC gegenüber dem Gleitsichtglas 10 aus Fig. 11e größer wird. Aus der Fig. 11f geht hervor, dass bei dem zusätzlichen negativen Astigmatismus mit der Achslage $\varphi = 90°$ der Abstand zwischen den Isoastigmatismuslinien mit der zylindrischen Brechkraft +0,5 DC und +1,00 DC gegenüber den dem Gleitsichtglas 10 aus Fig. 11e abnimmt.

Die Fig. 11a bis 11e zeigen, dass bei einem Gleitsichtglas 10, das in der Nahbereichszone 16 einen zusätzlichen negative Astigmatismus mit der zylindrischen Brechkraft -0,25 DC und der Achslage $\varphi = 0°$ hat und das in der Fernbereichszone 20 einen zusätzlichen negativen Astigmatismus mit der zylindrischen Brechkraft -0,25 DC und der Achslage $\varphi = 90°$ aufweist, eine vorteilhafte Ausdehnung der Nahbereichszone 16 und der Fernbereichszone 20 ermöglicht wird.

Aus den Fig. 11a bis 11e geht hervor, dass bei einem Gleitsichtglas somit mit einem zusätzlichen negativen Astigmatismus mit der zylindrischen Brechkraft -0,25 DC und der Achslage $\varphi = 0°$ für die Nähe und einem zusätzlichen negativen Astigmatismus mit der Achslage $\varphi = 90°$ für die Ferne einer Beobachtungsperson nicht nur das Betrachten des Objektbereichs mit einer entsprechend höheren Schärfentiefe ST bewirkt werden kann, sondern auch, dass ein solches Gleitsichtglas aufgrund der größeren Ausdehnung der Nah- und Fernbereichszone 16, 20 bei gleichen Verlauf der sphärischen Brechkraft auch einen verbesserten Sehkomfort bietet.

Darüber hinaus sei bemerkt, dass ein Gleitsichtglas 10, das einen vorstehend beschriebenen zusätzlichen Astigmatismus in der Nah- und Fernbereichszone 16, 20 hat, für eine Beobachtungsperson 28 wegen der entsprechend erhöhten Schärfentiefe auch mit einer Progression zwischen Fernbezugspunkt und Nahbezugspunkt sowie mit reduzierter sphärischer Brechkraft im Nahbezugspunkt ausgebildet sein kann. Auch diese Maßnahme hat eine entsprechend größere Ausdehnung der Nah- und Fernbereichszone 86, 88 zur Folge.

**Bezugszeichenliste**

| | |
|---|---|
| A, B | Blickrichtung |
| 6 | optische Sehhilfe |
| 7 | Fassung |
| 8 | Brillenglasrohling |
| 9 | Brillengestell |
| 10 | Brillenglas |
| 11, 11' | Auge |
| 12 | Brillenglasvorderfläche |
| 14 | Brillenglasrückfläche |
| 15 | Objekt |
| 16 | Nahbereichszone |
| 18 | Nahbezugspunkt |
| 20 | Fernbereichszone |
| 21 | Kreislinie |
| 22 | Fernbezugspunkt |
| 24 | Progressionskanal |
| 26 | System |
| 28 | Beobachtungsperson |
| 30 | Probierbrille |
| 31 | Probierglas |
| 32 | Kinnstütze |
| 34 | Visualisierungsvorrichtung |
| 36 | Sehzeichen |
| 38 | OLED-Display |
| 40 | Schiene |

| 42 | Rechnereinheit |
|---|---|
| 44 | Schrittmotor |
| 45 | Doppelpfeil |
| 46 | optische Gläser |
| 47 | Abstand |
| 48 | optische Gläser |
| 50 | Graph |
| 52 | Kurve |
| 54, 54' | Kurve |
| 56, 56' | Kurve |
| 58, 58' | Kurve |
| 60 | Graph |
| 62 | zweites System |
| 64 | Visualisierungsvorrichtung |
| 66 | erste Anzeigeeinrichtung |
| 67 | Anzeigefläche |
| 68 | zweite Anzeigeeinrichtung |
| 70 | Griff |
| 72 | Positionssensor |
| 74 | Positionssensor |
| 76 | Positionssensor |
| 78 | Druckknopf |
| 80 | Dockingstation |
| 82 | Kamera |
| 83 | Akkumulator |
| 84 | Einschubrahmen |
| 86,88 | Graph |
| 92 | System |
| 94 | Messeinrichtung |
| 96 | Retina |
| 97 | Laserlichtstrahl |
| 98 | Rechnereinheit |
| 100 | Isoastigmatismuslinie |
| 102 | Schnittstelle |

## Patentansprüche

1. Fertigung oder computergestütztes Bereitstellen oder computergestützte Berechnung oder computergestützte Auswahl einer optischen Sehhilfe (6) mit wenigstens einem Brillenglas (10) zur Verwendung von einer Beobachtungsperson (28) für das Betrachten eines Objekts (15),
wobei die optische Sehhilfe (6) für wenigstens eine Blickrichtung (A, B) eine auf ein Auge (11, 11') der Beobachtungsperson (28) abgestimmte dioptrische Wirkung hat, die sich aus mehreren dioptrischen Wirkungskomponenten ($K_1$, $K_2$, $K_3$, $K_4$) zusammensetzt,
**dadurch gekennzeichnet, dass**
eine erste dioptrische Wirkungskomponente ($K_1$, $K_3$) der mehreren dioptrischen Wirkungskomponenten ($K_1$, $K_2$, $K_3$, $K_4$) für das Auge (11, 11') der Beobachtungsperson (28) in einer definierten Entfernung As des Objekts (15) von dem Hornhautscheitel des Auges (11, 11') eine erste, für die wenigstens eine Blickrichtung (A, B) korrigierende Wirkung; und
eine weitere dioptrische Wirkungskomponente ($K_2$, $K_4$) der mehreren dioptrischen Wirkungskomponenten ($K_1$, $K_2$, $K_3$, $K_4$) für das Auge (11, 11') der Beobachtungsperson (28) in der definierten Entfernung As eine weitere, für die wenigstens eine Blickrichtung (A, B) zusätzliche astigmatische Wirkung hat,
wobei unter der korrigierenden Wirkung der ersten dioptrischen Wirkungskomponente die Eigenschaft verstanden wird, dass der Beitrag der dioptrischen Wirkungskomponente zu der dioptrischen Wirkung der Sehhilfe insgesamt eine Refraktion für eine Korrektur der Fehlsichtigkeit der Beobachtungsperson auf maximale Sehschärfe zumindest bis auf 1/5 D oder bis auf 1/8 D der sphärischen Wirkung genau und zumindest bis auf 1/5 DC oder bis auf 1/8 DC der astigmatischen Wirkung und ± 5° Achslage genau bewirkt,
wobei die weitere, für die wenigstens eine Blickrichtung (A, B) zusätzliche astigmatische Wirkung in der definierten Entfernung As des Objekts (15) die Sehschärfe der Beobachtungsperson um nicht mehr als 0,2 logMAR gegenüber der Sehschärfe reduziert, die mit der ersten dioptrischen Wirkungskomponente erzielt wird und wobei die erste dioptrische Wirkungskomponente ($K_1$) die das Auge (11, 11') der Beobachtungsperson (28) korrigierende Wirkung für die wenigstens eine Blickrichtung (A) für eine Entfernung As $\geq$ 4m des Objekts (15) von dem Hornhautscheitel des Auges (11, 11') hat und wobei die weitere dioptrische Wirkungskomponente ($K_2$) eine zusätzliche negative astigmatische Wirkung für das Auge (11, 11') der Beobachtungsperson (28) für die wenigstens eine Blickrichtung (A) aufweist, wobei die zusätzliche negative astigmatische Wirkung eine Zylinderbrechkraft BK ist mit -1,0 DC $\leq$ BK $\leq$ -0,125 DC oder -0,7 DC $\leq$ BK $\leq$ -0,3 DC oder BK $\approx$ -0,5 DC und mit einer in dem TABO-Schema angegebenen Achslage $\varphi$ mit 70° $\leq$ $\varphi$ $\leq$ 110° oder 80° $\leq$ $\varphi$ $\leq$ 100° oder $\varphi$ $\approx$ 90°, oder mit einer in dem TABO-Schema angegebenen Achslage $\varphi$ mit -20° $\leq$ $\varphi$ $\leq$ 20° oder -10° $\leq$ $\varphi$ $\leq$ 10° oder $\varphi$ $\approx$ 0°.

2. Fertigung oder computergestütztes Bereitstellen oder computergestützte Berechnung oder computergestützte Auswahl einer optischen Sehhilfe (6) mit wenigstens einem Brillenglas (10) zur Verwendung von einer Beobachtungsperson (28) für das Betrachten eines Objekts (15), wobei die optische Sehhilfe (6) für wenigstens eine Blickrichtung (A, B) eine auf ein Auge (11, 11') der Beobachtungsperson (28) abgestimmte dioptrische Wirkung hat, die sich aus mehreren dioptrischen Wirkungskomponenten ($K_1$, $K_2$, $K_3$, $K_4$) zusammensetzt,
**dadurch gekennzeichnet, dass**
eine erste dioptrische Wirkungskomponente ($K_1$, $K_3$) der mehreren dioptrischen Wirkungskomponenten ($K_1$, $K_2$, $K_3$, $K_4$) für das Auge (11, 11') der Beobachtungsperson (28) in einer definierten Entfernung As des Objekts (15) von dem Hornhautscheitel des Auges (11, 11') eine erste für die Blickrichtung (A, B) korrigierende Wirkung; und eine weitere dioptrische Wirkungskomponente ($K_2$, $K_4$) der mehreren dioptrischen Wirkungskomponenten ($K_1$, $K_2$, $K_3$, $K_4$) für das Auge (11, 11') der Beobachtungsperson (28) in der definierten Entfernung As eine weitere, für die wenigstens eine Blickrichtung (A, B) zusätzliche astigmatische Wirkung hat, wobei unter der korrigierenden Wirkung der ersten dioptrischen Wirkungskomponente die Eigenschaft verstanden wird, dass der Beitrag der dioptrischen Wirkungskomponente zu der dioptrischen Wirkung der Sehhilfe insgesamt eine Refraktion für eine Korrektur der Fehlsichtigkeit der Beobachtungsperson auf maximale Sehschärfe zumindest bis auf 1/5 D oder bis auf 1/8 D der sphärischen Wirkung genau und zumindest bis auf 1/5 DC oder bis auf 1/8 DC der astigmatischen Wirkung und $\pm$ 5° Achslage genau bewirkt, und wobei die weitere, für die wenigstens eine Blickrichtung (A, B) zusätzliche astigmatische Wirkung die Sehschärfe der Beobachtungsperson um nicht mehr als 0,2 logMAR gegenüber der Sehschärfe reduziert, die mit der ersten dioptrischen Wirkungskomponente erzielt wird, und wobei die erste dioptrische Wirkungskomponente ($K_3$) die das Auge (11, 11') der Beobachtungsperson (28) korrigierende Wirkung für die wenigstens eine Blickrichtung (B) für eine Entfernung As $\leq$ 1m des Objekts (15) von dem Hornhautscheitel des Auges (11, 11') hat und wobei die weitere dioptrische Wirkungskomponente ($K_4$) eine zusätzliche negative astigmatische Wirkung für das Auge (11, 11') der Beobachtungsperson (28) für die wenigstens eine Blickrichtung (B) aufweist, wobei die zusätzliche negative astigmatische Wirkung eine Zylinderbrechkraft BK ist mit -1,0 DC $\leq$ BK $\leq$ -0,125 DC oder -0,7 DC $\leq$ BK $\leq$ -0,3 DC oder BK $\approx$ -0,5 DC und eine in dem TABO-Schema angegebene Achslage $\varphi$ mit 70° $\leq$ $\varphi$ $\leq$ 110° oder 80° $\leq$ $\varphi$ $\leq$ 100° oder $\varphi$ $\approx$ 90°, oder eine in dem TABO-Schema angegebene Achslage $\varphi$ mit -20° $\leq$ $\varphi$ $\leq$ 20° oder -10° $\leq$ $\varphi$ $\leq$ 10° oder $\varphi$ $\approx$ 0°.

3. Vorgang nach Anspruch 2, **dadurch gekennzeichnet, dass** die erste dioptrische Wirkungskomponente ($K_3$) eine in Bezug auf eine das Auge (11, 11') der Beobachtungsperson (28) für eine Entfernung 25 cm $\leq$ $A_S$ $\leq$ 40 cm oder $A_S$ $\approx$ 33 cm eines Objekts (15) von dem Hornhautscheitel des Auges (11, 11') korrigierende Wirkung um den Wert -1,0 D $\leq$ $\Delta$SBK $\leq$ -0,1 D reduzierte sphärische Brechkraft SBK für die Blickrichtung (B) hat.

4. Vorgang nach einem der Ansprüche 1 bis 3, **dadurch gekennzeichnet, dass** sich die auf das Auge (11, 11') der Beobachtungsperson (28) abgestimmte dioptrische Wirkung aus mindestens zwei ersten und zwei weiteren diopt-rischen Wirkungskomponenten ($K_1$, $K_2$, $K_3$, $K_4$) zusammensetzt, wobei eine der beiden ersten dioptrischen Wir-kungskomponenten ($K_3$) die für das Auge (11, 11') der Beobachtungsperson (28) korrigierende Wirkung für eine Entfernung $A_S$ $\leq$ 1 m des Objekts (15) von dem Hornhautscheitel des Auges (11, 11') für eine erste Blickrichtung (B) hat und die andere der beiden ersten dioptrischen Wirkungskomponenten ($K_1$) die für das Auge (11, 11') der Beobachtungsperson (28) korrigierende Wirkung für eine Entfernung $A_S$ $\geq$ 4 m des Objekts (15) von dem Hornhautscheitel des Auges (11, 11') für eine weitere Blickrichtung (A) hat, und wobei eine der beiden zweiten dioptrischen Wirkungskomponenten ($K_2$) eine zusätzliche negative astigmatische Wirkung für das Auge (11, 11') der Beobachtungsperson (28) mit einer Zylinderbrechkraft BK mit -1,0 DC $\leq$ BK $\leq$ -0,125 DC oder -0,7 DC $\leq$ BK $\leq$ -0,3 DC oder BK $\approx$ -0,5 DC und einer in dem TABO-Schema angegebenen Achslage $\varphi$ mit 70° $\leq$ $\varphi$ $\leq$ 110° oder 80° $\leq$ $\varphi$ $\leq$ 100° oder $\varphi$ $\approx$ 90° für die Blickrichtung (B) oder einer Achslage $\varphi$ mit -20° $\leq$ $\varphi$ $\leq$ 20° oder -10° $\leq$ $\varphi$ $\leq$ 10° oder $\varphi$ $\approx$ 0° für

die Blickrichtung (B) hat, und wobei die andere der beiden zweiten dioptrischen Wirkungskomponenten ($K_4$) eine zusätzliche negative astigmatische Wirkung für das Auge (11, 11') der Beobachtungsperson (28) mit einer Zylinderbrechkraft BK mit -1,0 DC ≤ BK ≤ -0,125 DC oder -0,7 DC ≤ BK ≤ -0,3 DC oder BK ≈ -0,5 DC und einer in dem TABO-Schema angegebene Achslage $\varphi$ mit 70° ≤ $\varphi$ ≤ 110° oder 80° ≤ $\varphi$ ≤ 100° oder $\varphi$ ≈ 90°, oder einer Achslage $\varphi$ mit -20° ≤ $\varphi$ ≤ 20° oder -10° ≤ $\varphi$ ≤ 10° oder $\varphi$ ≈ 0° für die weitere Blickrichtung (A) hat.

5. Vorgang nach Anspruch 4, **dadurch gekennzeichnet, dass** die eine der beiden ersten dioptrischen Wirkungskomponenten ($K_1$) mit der für das Auge (11, 11') für die Entfernung As ≤ 1m korrigierenden Wirkung eine in Bezug auf eine für das Auge (11, 11') der Beobachtungsperson (28) für eine Entfernung 25 cm ≤ As ≤ 40 cm oder As ≈ 33 cm des Objekts (15) von dem Hornhautscheitel des Auges (11, 11') korrigierenden Wirkung um den Wert -1,0D ≤ $\Delta$SBK ≤ -0,1D reduzierte sphärische Brechkraft SBK für die Blickrichtung (B) hat.

6. Verfahren für das Ermitteln einer gesuchten Parametrisierung ($P_E$) der aus mehreren dioptischen Wirkungskomponenten ($K_1$, $K_2$, $K_3$, $K_4$) zusammengesetzten dioptischen Wirkung einer optischen Sehhilfe (6) mit wenigstens einem Brillenglas (10) für ein Auge (11, 11') einer Beobachtungsperson (28),
   **dadurch gekennzeichnet, dass**
   für das Auge (11, 11') der Beobachtungsperson (28) durch Einsetzen von Probiergläsern (31) in eine Probierbrille (30) eine sphärische Brechkraft und eine Zylinderbrechkraft sowie optional eine prismatische Wirkung eingestellt wird und aus der damit erfolgenden Korrektur des Auges (11, 11') der Beobachtungsperson (28) für wenigstens eine definierte Entfernung As eines Objekts (15) von dem Hornhautscheitel des Auges (11, 11') für wenigstens eine Blickrichtung (A, B) eine erste Parametrisierung ($P_A$) der dioptischen Wirkung der optischen Sehhilfe (6) in Form der sphärischen Wirkung, der astigmatische Wirkung und deren Achslage sowie optional die prismatische Wirkung und deren Basis entsprechend einer ersten Wirkungskomponente ($K_1$, $K_2$) bestimmt wird, die eine korrigierende Wirkung hat,
   die ermittelte erste Parametrisierung ($P_A$) um eine zusätzliche weitere dioptrische Wirkungskomponente ($K_2$, $K_4$) korrigiert wird, die in der definierten Entfernung (A3) eine für die wenigstens eine Blickrichtung (A, B) zusätzliche astigmatische Wirkung hat, und
   die korrigierte erste Parametrisierung ($P_A$) als die gesuchte Parametrisierung ($P_E$) festgelegt wird,
   wobei unter der korrigierenden Wirkung der ersten Parametrisierung ($P_A$) der dioptrischen Wirkung der Sehhilfe die Eigenschaft verstanden wird, dass diese Parametrisierung für die wenigstens eine Blickrichtung (A, B) eine Korrektur der Fehlsichtigkeit der Beobachtungsperson auf maximal Sehschärfe zumindest bis auf 1/5 D oder bis auf 1/8 D der sphärischen Wirkung und zumindest bis auf 1/5 DC oder bis auf 1/8 DC der astigmatischen Wirkung und ± 5° Achslage genau bewirkt, und
   wobei die weitere, für die wenigstens eine Blickrichtung (A, B) zusätzliche astigmatische Wirkung in der definierten Entfernung As die Sehschärfe der Beobachtungsperson um nicht mehr als 0,2 logMAR gegenüber der Sehschärfe reduziert, die mit der ersten dioptrischen Wirkungskomponente erzielt wird, wobei
   die Korrektur für das Auge (11, 11') der Beobachtungsperson (28) für die wenigstens eine Blickrichtung (B) eine Korrektur für eine Entfernung As ≤ 1 m oder 25 cm ≤ As ≤ 40 cm oder As ≈ 25 cm oder As ≈ 33 cm oder As ≈ 40 cm eines Objekts (15) von dem Hornhautscheitel (11, 11') des Auges ist und die zusätzliche weitere dioptrische Wirkungskomponente ($K_4$) eine zusätzliche negative astigmatische Wirkung für das Auge (11, 11') der Beobachtungsperson (28) aufweist, wobei die zusätzliche negative astigmatische Wirkung eine Zylinderbrechkraft BK ist mit -1,0 DC ≤ BK ≤ -0,125 DC oder -0,7 DC ≤ BK ≤ -0,3 DC oder BK ≈ -0,5 DC und mit einer in dem TABO-Schema angegebenen Achslage $\varphi$ mit 70° ≤ $\varphi$ ≤ 110° oder 80° ≤ $\varphi$ ≤ 100°, besonders bevorzugt $\varphi$ ≈ 90°, oder einer Achslage $\varphi$ mit -20° ≤ $\varphi$ ≤ 20° oder -10° ≤ $\varphi$ ≤ 10° oder $\varphi$ ≈ 0°.

7. Verfahren für das Ermitteln einer gesuchten Parametrisierung ($P_E$) der aus mehreren dioptischen Wirkungskomponenten ($K_1$, $K_2$, $K_3$, $K_4$) zusammengesetzten dioptischen Wirkung einer optischen Sehhilfe (6) mit wenigstens einem Brillenglas (10) für ein Auge (11, 11') einer Beobachtungsperson (28) mittels eines Computerprogramms mit Programmcodemitteln,
   **dadurch gekennzeichnet, dass**
   aus einer Korrektur des Auges (11, 11') der Beobachtungsperson (28) für wenigstens eine definierte Entfernung As eines Objekts (15) von dem Hornhautscheitel des Auges (11, 11') für wenigstens eine Blickrichtung (A, B) eine erste Parametrisierung ($P_A$) der dioptischen Wirkung der optischen Sehhilfe (6) in Form der sphärischen Wirkung, der astigmatische Wirkung und deren Achslage sowie optional die prismatische Wirkung und deren Basis entsprechend einer ersten Wirkungskomponente ($K_1$, $K_2$) bestimmt wird, die eine korrigierende Wirkung hat,
   die ermittelte erste Parametrisierung ($P_A$) um eine zusätzliche weitere dioptrische Wirkungskomponente ($K_2$, $K_4$) korrigiert wird, die in der definierten Entfernung (A3) eine für die wenigstens eine Blickrichtung (A, B) zusätzliche astigmatische Wirkung hat, und

die korrigierte erste Parametrisierung (P$_A$) als die gesuchte Parametrisierung (P$_E$) festgelegt wird,
wobei unter der korrigierenden Wirkung der ersten Parametrisierung (P$_A$) der dioptrischen Wirkung der Sehhilfe die Eigenschaft verstanden wird, dass diese Parametrisierung für die wenigstens eine Blickrichtung (A, B) eine Korrektur der Fehlsichtigkeit der Beobachtungsperson auf maximal Sehschärfe zumindest bis auf 1/5 D oder bis auf 1/8 D der sphärischen Wirkung und zumindest bis auf 1/5 DC oder bis auf 1/8 DC der astigmatischen Wirkung und ± 5° Achslage genau bewirkt, und
wobei die weitere, für die wenigstens eine Blickrichtung (A, B) zusätzliche astigmatische Wirkung in der definierten Entfernung As die Sehschärfe der Beobachtungsperson um nicht mehr als 0,2 logMAR gegenüber der Sehschärfe reduziert, die mit der ersten dioptrischen Wirkungskomponente erzielt wird, wobei
die Korrektur für das Auge (11, 11') der Beobachtungsperson (28) für die wenigstens eine Blickrichtung (B) eine Korrektur für eine Entfernung As $\leq$ 1 m oder 25 cm $\leq$ As $\leq$ 40 cm oder As $\approx$ 25 cm oder As $\approx$ 33 cm oder A$_S$ $\approx$ 40 cm eines Objekts (15) von dem Hornhautscheitel (11, 11') des Auges ist und die zusätzliche weitere dioptrische Wirkungskomponente (K$_4$) eine zusätzliche negative astigmatische Wirkung für das Auge (11, 11') der Beobachtungsperson (28) aufweist, wobei die zusätzliche negative astigmatische Wirkung eine Zylinderbrechkraft BK ist mit -1,0 DC $\leq$ BK $\leq$ -0,125 DC oder -0,7 DC $\leq$ BK $\leq$ -0,3 DC oder BK $\approx$ -0,5 DC und mit einer in dem TABO-Schema angegebenen Achslage φ mit 70° $\leq$ φ $\leq$ 110° oder 80° $\leq$ φ $\leq$ 100°, besonders bevorzugt φ $\approx$ 90°, oder einer Achslage φ mit -20° $\leq$ φ $\leq$ 20° oder -10° $\leq$ φ $\leq$ 10° oder φ $\approx$ 0°.

8. Verfahren nach Anspruch 6 oder 7, **dadurch gekennzeichnet, dass** die ermittelte erste Parametrisierung (P$_A$) um eine dioptrische Wirkungskomponente mit einer in Bezug auf eine für das Auge (11, 11') für eine Entfernung 25 cm $\leq$ As $\leq$ 40 cm oder As $\approx$ 33cm eines Objekts (15) von dem Hornhautscheitel des Auges (11, 11') korrigierenden Wirkung für maximale Sehschärfe um den Wert -1,0 D $\leq$ $\Delta$SBK $\leq$ -0,1D reduzierten sphärischen Brechkraft SBK korrigiert wird, und/oder **dadurch gekennzeichnet, dass** die Korrektur für das Auge (11, 11') der Beobachtungsperson (28) zusätzlich eine Korrektur für eine Entfernung As $\geq$ 4 m eines Objekts (15) von dem Hornhautscheitel (11, 11') des Auges für eine weitere Blickrichtung (B) ist und auch daraus die erste Parametrisierung (P$_A$) der optischen Sehhilfe (10) ermittelt wird, und die so ermittelte erste Parametrisierung (P$_A$) auch um eine zusätzliche dioptrische Wirkungskomponente (K$_3$) korrigiert wird und die korrigierte erste Parametrisierung (P$_A$) als die gesuchte Parametrisierung (P$_E$) festgelegt wird, wobei die zusätzliche dioptrische Wirkungskomponente (K$_3$) für das Auge (11, 11') der Beobachtungsperson (28) eine negative astigmatische Wirkung ist mit der Zylinderbrechkraft BK mit -1,0 DC $\leq$ BK $\leq$ -0,125 DC oder -0,7 DC $\leq$ BK $\leq$-0,3 DC oder BK $\approx$ -0,5 DC und mit einer in dem TABO-Schema angegebenen Achslage φ mit 70° $\leq$ φ $\leq$ 110° oder 80° $\leq$ φ $\leq$ 100° oder φ $\approx$ 90°, oder mit -20° $\leq$ φ $\leq$ 20° oder -10° $\leq$ φ $\leq$ 10° oder φ $\approx$ 0°.

9. Verfahren für das Ermitteln einer gesuchten Parametrisierung (P$_E$) der aus mehreren dioptischen Wirkungskomponenten (K$_1$, K$_2$, K$_3$, K$_4$) zusammengesetzten dioptischen Wirkung einer optischen Sehhilfe (6) mit wenigstens einem Brillenglas (10) für ein Auge (11, 11') einer Beobachtungsperson (28),
**dadurch gekennzeichnet, dass**
für das Auge (11, 11') der Beobachtungsperson (28) durch Einsetzen von Probiergläsern (31) in eine Probierbrille (30) eine sphärische Brechkraft und eine Zylinderbrechkraft sowie optional eine prismatische Wirkung eingestellt wird und aus der damit erfolgenden Korrektur des Auges (11, 11') der Beobachtungsperson (28) für wenigstens eine definierte Entfernung As eines Objekts (15) von dem Hornhautscheitel des Auges (11, 11') für wenigstens eine Blickrichtung (A, B) eine erste Parametrisierung (P$_A$) der dioptrischen Wirkung der optischen Sehhilfe (6) in Form der sphärischen Wirkung, der astigmatische Wirkung und deren Achslage sowie optional die prismatische Wirkung und deren Basis entsprechend einer ersten Wirkungskomponente (K$_1$, K$_2$) bestimmt wird, die eine korrigierende Wirkung hat,
die ermittelte erste Parametrisierung (P$_A$) um eine zusätzliche weitere dioptrische Wirkungskomponente (K$_2$, K$_4$) korrigiert wird, die in der definierten Entfernung (A3) eine für die wenigstens eine Blickrichtung (A, B) zusätzliche astigmatische Wirkung hat, und
die korrigierte erste Parametrisierung (P$_A$) als die gesuchte Parametrisierung (P$_E$) festgelegt wird,
wobei unter der korrigierenden Wirkung der ersten Parametrisierung (P$_A$) der dioptrischen Wirkung der Sehhilfe die Eigenschaft verstanden wird, dass diese Parametrisierung für die wenigstens eine Blickrichtung (A, B) eine Korrektur der Fehlsichtigkeit der Beobachtungsperson auf maximale Sehschärfe und zumindest bis auf 1/5 D oder bis auf 1/8 D der sphärischen Wirkung und zumindest bis auf 1/5 DC oder bis auf 1/8 DC der astigmatischen Wirkung und ± 5° Achslage genau bewirkt, und
wobei die weitere, für die wenigstens eine Blickrichtung (A, B) zusätzliche astigmatische Wirkung in der definierten Entfernung As die Sehschärfe der Beobachtungsperson um nicht mehr als 0,2 logMAR gegenüber der Sehschärfe reduziert, die mit der ersten dioptrischen Wirkungskomponente erzielt wird, wobei
die Korrektur für das Auge (11, 11') der Beobachtungsperson (28) für die wenigstens eine Blickrichtung (A) eine Korrektur für eine Entfernung As ≥ 4 m eines Objekts (15) von dem Hornhautscheitel (11, 11') des Auges ist und die zusätzliche weitere dioptrische Wirkungskomponente ($K_3$) eine zusätzliche negative astigmatische Wirkung für das Auge (11, 11') der Beobachtungsperson (28) aufweist, wobei die zusätzliche negative astigmatische Wirkung eine Zylinderbrechkraft BK ist mit -1,0 DC ≤ BK ≤ -0,125 DC oder -0,7 DC ≤ BK ≤ -0,3 DC oder BK ≈ -0,5 DC und mit einer in dem TABO-Schema angegebenen Achslage $\varphi$ mit 70° ≤ $\varphi$ ≤ 110° oder 80° ≤ $\varphi$ ≤ 100° oder $\varphi$ ≈ 90°, oder mit -20° ≤ $\varphi$ ≤ 20° oder -10° ≤ $\varphi$ ≤ 10° oder $\varphi$ ≈ 0°.

10. Verfahren für das Ermitteln einer gesuchten Parametrisierung ($P_E$) der aus mehreren dioptischen Wirkungskomponenten ($K_1$, $K_2$, $K_3$, $K_4$) zusammengesetzten dioptrischen Wirkung einer optischen Sehhilfe (6) mit wenigstens einem Brillenglas (10) für ein Auge (11, 11') einer Beobachtungsperson (28) mittels eines Computerprogramms mit Programmcodemitteln,
   **dadurch gekennzeichnet, dass**
   aus der Korrektur des Auges (11, 11') der Beobachtungsperson (28) für wenigstens eine definierte Entfernung As eines Objekts (15) von dem Hornhautscheitel des Auges (11, 11') für wenigstens eine Blickrichtung (A, B) eine erste Parametrisierung ($P_A$) der dioptrischen Wirkung der optischen Sehhilfe (6) in Form der sphärischen Wirkung, der astigmatische Wirkung und deren Achslage sowie optional die prismatische Wirkung und deren Basis entsprechend einer ersten Wirkungskomponente ($K_1$, $K_2$) bestimmt wird, die eine korrigierende Wirkung hat,
   die ermittelte erste Parametrisierung ($P_A$) um eine zusätzliche weitere dioptrische Wirkungskomponente ($K_2$, $K_4$) korrigiert wird, die in der definierten Entfernung (A3) eine für die wenigstens eine Blickrichtung (A, B) zusätzliche astigmatische Wirkung hat, und
   die korrigierte erste Parametrisierung ($P_A$) als die gesuchte Parametrisierung ($P_E$) festgelegt wird,
   wobei unter der korrigierenden Wirkung der ersten Parametrisierung ($P_A$) der dioptrischen Wirkung der Sehhilfe die Eigenschaft verstanden wird, dass diese Parametrisierung für die wenigstens eine Blickrichtung (A, B) eine Korrektur der Fehlsichtigkeit der Beobachtungsperson auf maximale Sehschärfe und zumindest bis auf 1/5 D oder bis auf 1/8 D der sphärischen Wirkung und zumindest bis auf 1/5 DC oder bis auf 1/8 DC der astigmatischen Wirkung und ± 5° Achslage genau bewirkt, und
   wobei die weitere, für die wenigstens eine Blickrichtung (A, B) zusätzliche astigmatische Wirkung in der definierten Entfernung As die Sehschärfe der Beobachtungsperson um nicht mehr als 0,2 logMAR gegenüber der Sehschärfe reduziert, die mit der ersten dioptrischen Wirkungskomponente erzielt wird, wobei
   die Korrektur für das Auge (11, 11') der Beobachtungsperson (28) für die wenigstens eine Blickrichtung (A) eine Korrektur für eine Entfernung As ≥ 4 m eines Objekts (15) von dem Hornhautscheitel (11, 11') des Auges ist und die zusätzliche weitere dioptrische Wirkungskomponente ($K_3$) eine zusätzliche negative astigmatische Wirkung für das Auge (11, 11') der Beobachtungsperson (28) aufweist, wobei die zusätzliche negative astigmatische Wirkung eine Zylinderbrechkraft BK ist mit -1,0 DC ≤ BK ≤ -0,125 DC oder -0,7 DC ≤ BK ≤ -0,3 DC oder BK ≈ -0,5 DC und mit einer in dem TABO-Schema angegebenen Achslage $\varphi$ mit 70° ≤ $\varphi$ ≤ 110° oder 80° ≤ $\varphi$ ≤ 100° oder $\varphi$ ≈ 90°, oder mit -20° ≤ $\varphi$ ≤ 20° oder -10° ≤ $\varphi$ ≤ 10° oder $\varphi$ ≈ 0°.

11. Vorgang nach einem der Ansprüche 1 bis 5,
    **gekennzeichnet durch**
    das Ermitteln einer gesuchten Parametrisierung ($P_E$) der aus mehreren dioptrischen Wirkungskomponenten ($K_1$, $K_2$, $K_3$, $K_4$) zusammengesetzten dioptrischen Wirkung der optischen Sehhilfe (6) mit einem Verfahren nach einem der Ansprüche 6 bis 10.

12. Bereitstellen oder Berechnung oder Fertigung oder Auswahl einer optischen Sehhilfe (6) mit wenigstens einem Brillenglas (10) zur Verwendung von einer Beobachtungsperson (28) für das Betrachten eines Objekts (15),
    **gekennzeichnet durch**
    das Ermitteln einer gesuchten Parametrisierung ($P_E$) einer aus mehreren dioptrischen Wirkungskomponenten ($K_1$, $K_2$, $K_3$, $K_4$) zusammengesetzten dioptrischen Wirkung der optischen Sehhilfe (6) mit einem Verfahren nach einem der Ansprüche 6 bis 10.

13. System für die Fertigung oder das computergestützte Bereitstellen oder die computergestützte Berechnung oder die computergestützte Auswahl einer optischen Sehhilfe (6) mit wenigstens einem Brillenglas (10) zur Verwendung von einer Beobachtungsperson (28) für das Betrachten eines Objekts (15)
    **gekennzeichnet durch**
    Mittel zum Ausführen eines Vorgangs nach einem der Ansprüche 1 bis 5 oder 11.

14. Computerprogramm mit Programmcodemitteln zur Ausführung des Verfahrens nach Anspruch 7 oder Anspruch 10 oder Anspruch 8, soweit dieser auf Anspruch 7 zurückbezogen ist.

**15.** System (92) zur Ermittlung einer gesuchten Parametrisierung (P$_E$) einer optischen Sehhilfe (10) für ein Auge (11, 11') einer Beobachtungsperson (28),
mit einer Messeinrichtung (94) für das Bestimmen einer bestmöglichen Korrektur des auf eine vorgegebene Entfernung (As) fixierenden Auges (11); und
mit einer Rechnereinheit (98), der die mit der Messeinrichtung (94) bestimmte bestmögliche Korrektur des auf eine vorgegebene Entfernung (As) akkommodierten Auges (11) zuführbar ist;
**dadurch gekennzeichnet, dass**
die Rechnereinheit ein Computerprogramm mit Programmcodemitteln für das Ermitteln der gesuchten Parametrisierung (P$_E$) aus der zugeführten bestmöglichen Korrektur ein Verfahren nach Anspruch 7 oder nach Anspruch 10 oder nach Anspruch 8, soweit dieser auf Anspruch 7 zurückbezogen ist, ausführt.


**Claims**

**1.** Manufacture or computer-assisted provision or computer-assisted calculation or computer-assisted selection of an optical visual aid (6) comprising at least one spectacle lens (10), to be used by an observer (28) for looking at an object (15),
wherein the optical visual aid (6) has a dioptric power matched to an eye (11, 11') of the observer (28) for at least one viewing direction (A, B), said dioptric power being composed of a plurality of dioptric power components ($K_1$, $K_2$, $K_3$, $K_4$),
**characterized in that**
a first dioptric power component ($K_1$, $K_3$) of the plurality of dioptric power components ($K_1$, $K_2$, $K_3$, $K_4$) has a first corrective power for the at least one viewing direction (A, B) for the eye (11, 11') of the observer (28) at a defined distance A$_S$ of the object (15) from the corneal vertex of the eye (11, 11'); and
a further dioptric power component ($K_2$, $K_4$) of the plurality of dioptric power components ($K_1$, $K_2$, $K_3$, $K_4$) has a further, additional astigmatic power for the at least one viewing direction (A, B) for the eye (11, 11') of the observer (28) at the defined distance A$_S$,
wherein the corrective power of the first dioptric power component is understood to mean the property that the contribution of the dioptric power component to the dioptric power of the visual aid overall brings about a refraction for correcting the refractive error of the observer to the maximum visual acuity, at least with an accuracy of up to 1/5 D or up to 1/8 D of the spherical power and at least with an accuracy of up to 1/5 DC or up to 1/8 DC of the astigmatic power and an axis location of $\pm$ 5°,
wherein the further, additional astigmatic power for the at least one viewing direction (A, B) at the defined distance A$_S$ of the object (15) reduces the visual acuity of the observer by no more than 0.2 logMAR in relation to the visual acuity obtained by the first dioptric power component,
and wherein
the first dioptric power component ($K_1$) has the power which, for a distance A$_S$ $\geq$ 4m of the object (15) from the corneal vertex of the eye (11, 11'), corrects the eye (11, 11') of the observer (28) for the at least one viewing direction (A) and wherein the further dioptric power component ($K_2$) has an additional negative astigmatic power for the eye (11, 11') of the observer (28) for the at least one viewing direction (A), wherein the additional negative astigmatic power is a cylindrical refractive power BK with -1.0 DC $\leq$ BK $\leq$ -0.125 DC or -0.7 DC $\leq$ BK $\leq$ -0.3 DC or BK $\approx$ -0.5 DC and with an axis location $\varphi$, specified in the TABO scheme, with 70° $\leq$ $\varphi$ $\leq$ 110° or 80° $\leq$ $\varphi$ $\leq$ 100° or $\varphi$ $\approx$ 90°, or with an axis location $\varphi$, specified in the TABO scheme, with -20° $\leq$ $\varphi$ $\leq$ 20° or -10° $\leq$ $\varphi$ $\leq$ 10° or $\varphi$ $\approx$ 0°.

**2.** Manufacture or computer-assisted provision or computer-assisted calculation or computer-assisted selection of an optical visual aid (6) comprising at least one spectacle lens (10), to be used by an observer (28) for looking at an object (15),
wherein the optical visual aid (6) has a dioptric power matched to an eye (11, 11') of the observer (28) for at least one viewing direction (A, B), said dioptric power being composed of a plurality of dioptric power components ($K_1$, $K_2$, $K_3$, $K_4$),
**characterized in that**
a first dioptric power component ($K_1$, $K_3$) of the plurality of dioptric power components ($K_1$, $K_2$, $K_3$, $K_4$) has a first corrective power for the viewing direction (A, B) for the eye (11, 11') of the observer (28) at a defined distance A$_S$ of the object (15) from the corneal vertex of the eye (11, 11'); and
a further dioptric power component ($K_2$, $K_4$) of the plurality of dioptric power components ($K_1$, $K_2$, $K_3$, $K_4$) has a further, additional astigmatic power for the at least one viewing direction (A, B) for the eye (11, 11') of the observer (28) at the defined distance A$_S$,
wherein the corrective power of the first dioptric power component is understood to mean the property that the contribution of the dioptric power component to the dioptric power of the visual aid overall brings about a refraction for correcting the refractive error of the observer to the maximum visual acuity, at least with an accuracy of up to 1/5 D or up to 1/8 D of the spherical power and at least with an accuracy of up to 1/5 DC or up to 1/8 DC of the astigmatic power and an axis location of $\pm$ 5°, and

wherein the further, additional astigmatic power for the at least one viewing direction (A, B) reduces the visual acuity of the observer by no more than 0.2 logMAR in relation to the visual acuity obtained by the first dioptric power component, and wherein

the first dioptric power component ($K_3$) has the power which, for a distance $A_S \leq 1m$ of the object (15) from the corneal vertex of the eye (11, 11'), corrects the eye (11, 11') of the observer (28) for the at least one viewing direction (B) and wherein the further dioptric power component ($K_4$) has an additional negative astigmatic power for the eye (11, 11') of the observer (28) for the at least one viewing direction (B), wherein the additional negative astigmatic power is a cylindrical refractive power BK with -1.0 DC $\leq$ BK $\leq$ -0.125 DC or -0.7 DC $\leq$ BK $\leq$ -0.3 DC or BK $\approx$ -0.5 DC and an axis location $\varphi$, specified in the TABO scheme, with 70° $\leq \varphi \leq$ 110° or 80° $\leq \varphi \leq$ 100° or $\varphi \approx$ 90°, or an axis location $\varphi$, specified in the TABO scheme, with -20° $\leq \varphi \leq$ 20° or -10° $\leq \varphi \leq$ 10° or $\varphi \approx$ 0°.

3. Process according to Claim 2, **characterized in that** the first dioptric power component ($K_3$) has a spherical refractive power SBK for the viewing direction (B) which has been reduced by the value - 1.0 D $\leq \Delta$SBK $\leq$ -0.1 D in relation to a power which, for a distance 25 cm $\leq A_S \leq$ 40 cm or $A_S \approx$ 33 cm of an object (15) from the corneal vertex of the eye (11, 11'), corrects the eye (11, 11') of the observer (28) .

4. Process according to any one of Claims 1 to 3, **characterized in that** the dioptric power matched to the eye (11, 11') of the observer (28) is composed of at least two first and two further dioptric power components ($K_1$, $K_2$, $K_3$, $K_4$), wherein one of the two first dioptric power components ($K_3$) has the power which, for a distance $A_S \leq$ 1 m of the object (15) from the corneal vertex of the eye (11, 11'), corrects the eye (11, 11') of the observer (28) for a first viewing direction (B) and the other one of the two first dioptric power components ($K_1$) has the power which, for a distance $A_S \geq$ 4 m of the object (15) from the corneal vertex of the eye (11, 11'), corrects the eye (11, 11') of the observer (28) for a further viewing direction (A), and wherein one of the two second dioptric power components ($K_2$) has an additional negative astigmatic power for the eye (11, 11') of the observer (28) with a cylindrical refractive power BK with -1.0 DC $\leq$ BK $\leq$ -0.125 DC or -0.7 DC $\leq$ BK $\leq$ -0.3 DC or BK $\approx$ -0.5 DC and an axis location $\varphi$, specified in the TABO scheme, with 70° $\leq \varphi \leq$ 110° or 80° $\leq \varphi \leq$ 100° or $\varphi \approx$ 90° for the viewing direction (B) or an axis location $\varphi$ with -20° $\leq \varphi \leq$ 20° or -10° $\leq \varphi \leq$ 10° or $\varphi \approx$ 0° for the viewing direction (B), and wherein the other one of the two second dioptric power components ($K_4$) has an additional negative astigmatic power for the eye (11, 11') of the observer (28) with a cylindrical refractive power BK with -1.0 DC $\leq$ BK $\leq$ -0.125 DC or -0.7 DC $\leq$ BK $\leq$ -0.3 DC or BK $\approx$ -0.5 DC and an axis location $\varphi$, specified in the TABO scheme, with 70° $\leq \varphi \leq$ 110° or 80° $\leq \varphi \leq$ 100° or $\varphi \approx$ 90° or an axis location $\varphi$ with -20° $\leq \varphi \leq$ 20° or -10° $\leq \varphi \leq$ 10° or $\varphi \approx$ 0° for the further viewing direction (A).

5. Process according to Claim 4, **characterized in that** the one of the two first dioptric power components ($K_1$) with the power which, for the distance $A_S \leq$ 1m, corrects the eye (11, 11') has a spherical refractive power SBK for the viewing direction (B) which has been reduced by the value -1.0 D $\leq \Delta$SBK $\leq$ -0.1 D in relation to a power which, for a distance 25 cm $\leq A_S \leq$ 40 cm or $A_S \approx$ 33 cm of the object (15) from the corneal vertex of the eye (11, 11'), corrects the eye (11, 11') of the observer (28).

6. Method for ascertaining a sought parameterization ($P_E$) of the dioptric power, composed of a plurality of dioptric power components ($K_1$, $K_2$, $K_3$, $K_4$), of an optical visual aid (6) with at least one spectacle lens (10) for an eye (11, 11') of an observer (28),
   **characterized in that**
   by inserting testing lenses (31) into testing spectacles (30) a spherical refractive power and a cylindrical refractive power and optionally a prismatic power are set for the eye (11, 11') of the observer (28) and a first parameterization ($P_A$) of the dioptric power of the optical visual aid (6) in the form of the spherical power, the astigmatic power and the axis location thereof, as well as optionally the prismatic power and the basis thereof, in accordance with a first power component ($K_1$, $K_2$) , which has a corrective power, is determined from the resulting correction of the eye (11, 11') of the observer (28) for at least one defined distance $A_S$ of an object (15) from the corneal vertex of the eye (11, 11') for at least one viewing direction (A, B),
   the ascertained first parameterization ($P_A$) is corrected by an additional further dioptric power component ($K_2$, $K_4$), which has an additional astigmatic power for the at least one viewing direction (A, B) at the defined distance (A3), and
   the corrected first parameterization ($P_A$) is set as the sought parameterization ($P_E$),
   wherein the corrective power of the first parameterization ($P_A$) of the dioptric power of the visual aid is understood to mean the property that, for the at least one viewing direction (A, B), this parameterization brings about a correction of the refractive error of the observer to the maximum visual acuity, at least with an accuracy of up to 1/5 D or up to 1/8 D of the spherical power and at least with an accuracy of up to 1/5 DC or up to 1/8 DC of the astigmatic power and an axis location of $\pm$ 5°, and

wherein the further, additional astigmatic power for the at least one viewing direction (A, B) at the defined distance $A_S$ reduces the visual acuity of the observer by no more than 0.2 logMAR in relation to the visual acuity obtained by the first dioptric power component, wherein

the correction for the eye (11, 11') of the observer (28) for the at least one viewing direction (B) is a correction for a distance $A_S \leq 1$ m or 25 cm $\leq A_S \leq 40$ cm or $A_S \approx 25$ cm or $A_S \approx 33$ cm or $A_S \approx 40$ cm of an object (15) from the corneal vertex (11, 11') of the eye and the additional further dioptric power component (($K_4$) has an additional negative astigmatic power for the eye (11, 11') of the observer (28), wherein the additional negative astigmatic power is a cylindrical refractive power BK with -1.0 DC $\leq$ BK $\leq$ -0.125 DC or -0.7 DC $\leq$ BK $\leq$ -0.3 DC or BK $\approx$ -0.5 DC and with an axis location $\varphi$, specified in the TABO scheme, with 70° $\leq \varphi \leq$ 110° or 80° $\leq \varphi \leq$ 100°, particularly preferably $\varphi \approx$ 90°, or an axis location $\varphi$ with -20° $\leq \varphi \leq$ 20° or -10° $\leq \varphi \leq$ 10° or $\varphi \approx$ 0°.

7. Method for ascertaining a sought parameterization ($P_E$) of the dioptric power, composed of a plurality of dioptric power components ($K_1$, $K_2$, $K_3$, $K_4$), of an optical visual aid (6) with at least one spectacle lens (10) for an eye (11, 11') of an observer (28) by means of a computer program comprising program code means,
**characterized in that**
a first parameterization ($P_A$) of the dioptric power of the optical visual aid (6) in the form of the spherical power, the astigmatic power and the axis location thereof, as well as optionally the prismatic power and the basis thereof, in accordance with a first power component ($K_1$, $K_2$), which has a corrective power, is determined from a correction of the eye (11, 11') of the observer (28) for at least one defined distance $A_S$ of an object (15) from the corneal vertex of the eye (11, 11') for at least one viewing direction (A, B),
the ascertained first parameterization ($P_A$) is corrected by an additional further dioptric power component ($K_2$, $K_4$), which has an additional astigmatic power for the at least one viewing direction (A, B) at the defined distance (A3), and the corrected first parameterization ($P_A$) is set as the sought parameterization ($P_E$),
wherein the corrective power of the first parameterization ($P_A$) of the dioptric power of the visual aid is understood to mean the property that, for the at least one viewing direction (A, B), this parameterization brings about a correction of the refractive error of the observer to the maximum visual acuity, at least with an accuracy of up to 1/5 D or up to 1/8 D of the spherical power and at least with an accuracy of up to 1/5 DC or up to 1/8 DC of the astigmatic power and an axis location of $\pm$ 5°, and
wherein the further, additional astigmatic power for the at least one viewing direction (A, B) at the defined distance $A_S$ reduces the visual acuity of the observer by no more than 0.2 logMAR in relation to the visual acuity obtained by the first dioptric power component, wherein
the correction for the eye (11, 11') of the observer (28) for the at least one viewing direction (B) is a correction for a distance $A_S \leq 1$ m or 25 cm $\leq A_S \leq 40$ cm or $A_S \approx 25$ cm or $A_S \approx 33$ cm or $A_S \approx 40$ cm of an object (15) from the corneal vertex (11, 11') of the eye and the additional further dioptric power component (($K_4$) has an additional negative astigmatic power for the eye (11, 11') of the observer (28), wherein the additional negative astigmatic power is a cylindrical refractive power BK with -1.0 DC $\leq$ BK $\leq$ -0.125 DC or -0.7 DC $\leq$ BK $\leq$ -0.3 DC or BK $\approx$ -0.5 DC and with an axis location $\varphi$, specified in the TABO scheme, with 70° $\leq \varphi \leq$ 110° or 80° $\leq \varphi \leq$ 100°, particularly preferably $\varphi \approx$ 90°, or an axis location $\varphi$ with -20° $\leq \varphi \leq$ 20° or -10° $\leq \varphi \leq$ 10° or $\varphi \approx$ 0°.

8. Method according to Claim 6 or 7, **characterized in that** the ascertained first parameterization ($P_A$) is corrected by a dioptric power component with a spherical refractive power SBK which has been reduced by the value -1.0 D $\leq$ $\Delta$SBK $\leq$ -0.1 D in relation to a power which, for a distance 25 cm $\leq A_S \leq$ 40 cm or $A_S \approx$ 33 cm of an object (15) from the corneal vertex of the eye (11, 11'), corrects the eye (11, 11') for maximum visual acuity, and/or **characterized in that** the correction for the eye (11, 11') of the observer (28) additionally is a correction for a distance $A_S \geq 4$ m of an object (15) from the corneal vertex (11, 11') of the eye for a further viewing direction (B) and the first parameterization ($P_A$) of the optical visual aid (10) is also ascertained therefrom, and the first parameterization ($P_A$) ascertained thus is also corrected by an additional dioptric power component ($K_3$) and the corrected first parameterization ($P_A$) is set as the sought parameterization ($P_E$), wherein the additional dioptric power component ($K_3$) for the eye (11, 11') of the observer (28) is a negative astigmatic power with the cylindrical refractive power BK with -1.0 DC $\leq$ BK $\leq$ -0.125 DC or -0.7 DC $\leq$ BK $\leq$ -0.3 DC or BK $\approx$ -0,5 DC and with an axis location $\varphi$, specified in the TABO scheme, with 70° $\leq \varphi \leq$ 110° or 80° $\leq \varphi \leq$ 100° or $\varphi \approx$ 90°, or with -20° $\leq \varphi \leq$ 20° or -10° $\leq \varphi \leq$ 10° or $\varphi \approx$ 0°.

9. Method for ascertaining a sought parameterization ($P_E$) of the dioptric power, composed of a plurality of dioptric power components ($K_1$, $K_2$, $K_3$, $K_4$), of an optical visual aid (6) with at least one spectacle lens (10) for an eye (11, 11') of an observer (28),

**characterized in that**

by inserting testing lenses (31) into testing spectacles (30) a spherical refractive power and a cylindrical refractive power and optionally a prismatic power are set for the eye (11, 11') of the observer (28) and a first parameterization ($P_A$) of the dioptric power of the optical visual aid (6) in the form of the spherical power, the astigmatic power and the axis location thereof, as well as optionally the prismatic power and the basis thereof, in accordance with a first power component ($K_1$, $K_2$), which has a corrective power, is determined from the resulting correction of the eye (11, 11') of the observer (28) for at least one defined distance $A_S$ of an object (15) from the corneal vertex of the eye (11, 11') for at least one viewing direction (A, B),

the ascertained first parameterization ($P_A$) is corrected by an additional further dioptric power component ($K_2$, $K_4$), which has an additional astigmatic power for the at least one viewing direction (A, B) at the defined distance (A3), and the corrected first parameterization ($P_A$) is set as the sought parameterization ($P_E$),

wherein the corrective power of the first parameterization ($P_A$) of the dioptric power of the visual aid is understood to mean the property that, for the at least one viewing direction (A, B), this parameterization brings about a correction of the refractive error of the observer to the maximum visual acuity and at least with an accuracy of up to 1/5 D or up to 1/8 D of the spherical power and at least with an accuracy of up to 1/5 DC or up to 1/8 DC of the astigmatic power and an axis location of $\pm$ 5°, and

wherein the further, additional astigmatic power for the at least one viewing direction (A, B) at the defined distance $A_S$ reduces the visual acuity of the observer by no more than 0.2 logMAR in relation to the visual acuity obtained by the first dioptric power component, wherein

the correction for the eye (11, 11') of the observer (28) for the at least one viewing direction (A) is a correction for a distance $A_S \geq 4$ m of an object (15) from the corneal vertex (11, 11') of the eye and the additional further dioptric power component ($K_3$) has an additional negative astigmatic power for the eye (11, 11') of the observer (28), wherein the additional negative astigmatic power is a cylindrical refractive power BK with -1.0 DC $\leq$ BK $\leq$ -0.125 DC or -0.7 DC $\leq$ BK $\leq$ -0.3 DC or BK $\approx$ -0.5 DC and with an axis location $\varphi$, specified in the TABO scheme, with 70° $\leq \varphi \leq$ 110° or 80° $\leq \varphi \leq$ 100°, or $\varphi \approx$ 90°, or with -20° $\leq \varphi \leq$ 20° or -10° $\leq \varphi \leq$ 10° or $\varphi \approx$ 0°.

10. Method for ascertaining a sought parameterization ($P_E$) of the dioptric power, composed of a plurality of dioptric power components ($K_1$, $K_2$, $K_3$, $K_4$), of an optical visual aid (6) with at least one spectacle lens (10) for an eye (11, 11') of an observer (28) by means of a computer program comprising program code means,

**characterized in that**

a first parameterization ($P_A$) of the dioptric power of the optical visual aid (6) in the form of the spherical power, the astigmatic power and the axis location thereof, as well as optionally the prismatic power and the basis thereof, in accordance with a first power component ($K_1$, $K_2$), which has a corrective power, is determined from the correction of the eye (11, 11') of the observer (28) for at least one defined distance $A_S$ of an object (15) from the corneal vertex of the eye (11, 11') for at least one viewing direction (A, B),

the ascertained first parameterization ($P_A$) is corrected by an additional further dioptric power component ($K_2$, $K_4$), which has an additional astigmatic power for the at least one viewing direction (A, B) at the defined distance (A3), and the corrected first parameterization ($P_A$) is set as the sought parameterization ($P_E$),

wherein the corrective power of the first parameterization ($P_A$) of the dioptric power of the visual aid is understood to mean the property that, for the at least one viewing direction (A, B), this parameterization brings about a correction of the refractive error of the observer to the maximum visual acuity and at least with an accuracy of up to 1/5 D or up to 1/8 D of the spherical power and at least with an accuracy of up to 1/5 DC or up to 1/8 DC of the astigmatic power and an axis location of $\pm$ 5°, and

wherein the further, additional astigmatic power for the at least one viewing direction (A, B) at the defined distance $A_S$ reduces the visual acuity of the observer by no more than 0.2 logMAR in relation to the visual acuity obtained by the first dioptric power component, wherein

the correction for the eye (11, 11') of the observer (28) for the at least one viewing direction (A) is a correction for a distance $A_S \geq 4$ m of an object (15) from the corneal vertex (11, 11') of the eye and the additional further dioptric power component ($K_3$) has an additional negative astigmatic power for the eye (11, 11') of the observer (28), wherein the additional negative astigmatic power is a cylindrical refractive power BK with -1.0 DC $\leq$ BK $\leq$ -0.125 DC or -0.7 DC $\leq$ BK $\leq$ -0.3 DC or BK $\approx$ -0.5 DC and with an axis location $\varphi$, specified in the TABO scheme, with 70° $\leq \varphi \leq$ 110° or 80° $\leq \varphi \leq$ 100°, or $\varphi \approx$ 90°, or with -20° $\leq \varphi \leq$ 20° or -10° $\leq \varphi \leq$ 10° or $\varphi \approx$ 0°.

11. Process according to any one of Claims 1 to 5,

**characterized by**

ascertaining a sought parameterization ($P_E$) of the dioptric power, composed of a plurality of dioptric power components ($K_1$, $K_2$, $K_3$, $K_4$), of the optical visual aid (6) using a method according to any one of Claims 6 to 10.

**12.** Provision or calculation or manufacture or selection of an optical visual aid (6) comprising at least one spectacle lens (10), to be used by an observer (28) for looking at an object (15),
**characterized by**
ascertaining a sought parameterization ($P_E$) of the dioptric power, composed of a plurality of dioptric power components ($K_1$, $K_2$, $K_3$, $K_4$), of the optical visual aid (6) using a method according to any one of Claims 6 to 10.

**13.** System for the manufacture or the computer-assisted provision or the computer-assisted calculation or the computer-assisted selection of an optical visual aid (6) comprising at least one spectacle lens (10), to be used by an observer (28) for looking at an object (15),
**characterized by**
means for carrying out a process according to any one of Claims 1 to 5 or 11.

**14.** Computer program comprising program code means for carrying out the method according to Claim 7 or Claim 10 or Claim 8, insofar as this refers back to Claim 7.

**15.** System (92) for ascertaining a sought parameterization ($P_E$) of an optical visual aid (10) for an eye (11, 11') of an observer (28),
comprising a measuring device (94) for determining a best possible correction of the eye (11) fixing onto a predetermined distance ($A_S$); and
comprising a computer unit (98), to which the best possible correction of the eye (11) accommodated onto a predetermined distance ($A_S$), determined by the measuring device (94), is suppliable;
**characterized in that**
the computer unit carries out a computer program comprising program code means for ascertaining the sought parameterization ($P_E$) from the supplied best possible correction a method according to Claim 7 or according to Claim 10 or according to Claim 8, insofar as this refers back to Claim 7.

**Revendications**

**1.** Fabrication ou fourniture assistée par ordinateur ou calcul assisté par ordinateur ou sélection assistée par ordinateur d'une aide visuelle optique (6) comprenant au moins un verre de lunettes (10) destinée à être utilisée par une personne observatrice (28) pour l'observation d'un objet (15),
l'aide visuelle optique (6) possédant, pour au moins une direction du regard (A, B), un effet dioptrique accordé à un oeil (11, 11') de la personne observatrice (28) qui est constitué de plusieurs composantes actives dioptriques ($K_1$, $K_2$, $K_3$, $K_4$),
**caractérisée en ce que**
une première composante active dioptrique ($K_1$, $K_3$) des plusieurs composantes actives dioptriques ($K_1$, $K_2$, $K_3$, $K_4$) possède un premier effet correcteur, pour l'au moins une direction du regard (A, B), pour l'oeil (11, 11') de la personne observatrice (28) à une distance $A_S$ définie entre l'objet (15) et le sommet de la cornée de l'oeil (11, 11') ; et
une autre composante active dioptrique ($K_2$, $K_4$) des plusieurs composantes actives dioptriques ($K_1$, $K_2$, $K_3$, $K_4$) possède un autre effet astigmatique supplémentaire, pour l'au moins une direction du regard (A, B), pour l'oeil (11, 11') de la personne observatrice (28) à la distance $A_S$ définie,
l'effet correcteur de la première composante active dioptrique désignant la propriété selon laquelle la contribution de la composante active dioptrique à l'effet correcteur de l'aide visuelle produit dans l'ensemble une réfraction pour une correction de l'amétropie de la personne observatrice à une acuité visuelle maximale précisément au moins jusqu'à 1/5 D ou jusqu'à 1/8 D de l'effet sphérique et précisément au moins jusqu'à 1/5 DC ou jusqu'à 1/8 DC de l'effet astigmatique et ±5° de position d'axe,
l'autre effet astigmatique supplémentaire, pour l'au moins une direction du regard (A, B) , à la distance $A_S$ définie de l'objet (15) ne réduisant pas l'acuité visuelle de la personne observatrice de plus de 0,2 logMAR par rapport à l'acuité visuelle qui est obtenue avec la première composante active dioptrique,
et
la première composante active dioptrique ($K_1$) possédant l'effet correcteur de l'oeil (11, 11') de la personne observatrice (28) pour l'au moins une direction du regard (A) pour une distance $A_S \geq 4$ m entre l'objet (15) et le sommet de la cornée de l'oeil (11, 11'), et l'autre composante active dioptrique ($K_2$) possédant un effet astigmatique négatif supplémentaire pour l'oeil (11, 11') de la personne observatrice (28) pour l'au moins une direction du regard (A),
l'effet astigmatique négatif supplémentaire étant une puissance de réfraction de cylindre BK, avec -1,0 DC $\leq$ BK $\leq$ -0,125 DC ou -0,7 DC $\leq$ BK $\leq$ -0,3 DC ou BK $\approx$ -0,5 DC et avec une position d'axe $\varphi$ indiquée dans le schéma TABO de 70° $\leq \varphi \leq$ 110° ou 80° $\leq \varphi \leq$ 100° ou $\varphi \approx$ 90°, ou avec une position d'axe $\varphi$ indiquée dans le schéma TABO de

-20° ≤ φ ≤ 20° ou -10° ≤ φ ≤ 10° ou φ ≈ 0°.

**2.** Fabrication ou fourniture assistée par ordinateur ou calcul assisté par ordinateur ou sélection assistée par ordinateur d'une aide visuelle optique (6) comprenant au moins un verre de lunettes (10) destinée à être utilisée par une personne observatrice (28) pour l'observation d'un objet (15),
l'aide visuelle optique (6) possédant, pour au moins une direction du regard (A, B), un effet dioptrique accordé à un oeil (11, 11') de la personne observatrice (28) qui est constitué de plusieurs composantes actives dioptriques ($K_1$, $K_2$, $K_3$, $K_4$),
**caractérisée en ce que**
une première composante active dioptrique ($K_1$, $K_3$) des plusieurs composantes actives dioptriques ($K_1$, $K_2$, $K_3$, $K_4$) possède un premier effet correcteur, pour la direction du regard (A, B), pour l'oeil (11, 11') de la personne observatrice (28) à une distance $A_S$ définie entre l'objet (15) et le sommet de la cornée de l'oeil (11, 11') ; et une autre composante active dioptrique ($K_2$, $K_4$) des plusieurs composantes actives dioptriques ($K_1$, $K_2$, $K_3$, $K_4$) possède un autre effet astigmatique supplémentaire, pour l'au moins une direction du regard (A, B), pour l'oeil (11, 11') de la personne observatrice (28) à la distance $A_S$ définie,
l'effet correcteur de la première composante active dioptrique désignant la propriété selon laquelle la contribution de la composante active dioptrique à l'effet correcteur de l'aide visuelle produit dans l'ensemble une réfraction pour une correction de l'amétropie de la personne observatrice à une acuité visuelle maximale précisément au moins jusqu'à 1/5 D ou jusqu'à 1/8 D de l'effet sphérique et précisément au moins jusqu'à 1/5 DC ou jusqu'à 1/8 DC de l'effet astigmatique et ±5° de position d'axe, et
l'autre effet astigmatique supplémentaire, pour l'au moins une direction du regard (A, B), ne réduisant pas l'acuité visuelle de la personne observatrice de plus de 0,2 logMAR par rapport à l'acuité visuelle qui est obtenue avec la première composante active dioptrique, et
la première composante active dioptrique ($K_3$) possédant l'effet correcteur de l'oeil (11, 11') de la personne observatrice (28) pour l'au moins une direction du regard (B) pour une distance $A_S$ ≤ 1 m entre l'objet (15) et le sommet de la cornée de l'oeil (11, 11'), et l'autre composante active dioptrique ($K_4$) possédant un effet astigmatique négatif supplémentaire pour l'oeil (11, 11') de la personne observatrice (28) pour l'au moins une direction du regard (B), l'effet astigmatique négatif supplémentaire étant une puissance de réfraction de cylindre BK, avec -1,0 DC ≤ BK ≤ -0,125 DC ou -0,7 DC ≤ BK ≤ -0,3 DC ou BK ≈ -0,5 DC et avec une position d'axe φ indiquée dans le schéma TABO de 70° ≤ φ ≤ 110° ou 80° ≤ φ ≤ 100° ou φ ≈ 90°, ou une position d'axe φ indiquée dans le schéma TABO de -20° ≤ φ ≤ 20° ou -10° ≤ φ ≤ 10° ou φ ≈ 0°.

**3.** Opération selon la revendication 2, **caractérisée en ce que** la première composante active dioptrique ($K_3$) possède, pour la direction du regard (B), une puissance de réfraction sphérique SBK réduite de la valeur -1,0 D ≤ ΔSBK ≤ -0,1 D en référence à un effet de correction de l'oeil (11, 11') de la personne observatrice (28) pour une distance 25 cm ≤ $A_S$ ≤ 40 cm ou $A_S$ ≈ 33 cm entre un objet (15) et le sommet de la cornée de l'oeil (11, 11').

**4.** Opération selon l'une des revendications 1 à 3, **caractérisée en ce que** l'effet correcteur accordé à l'oeil (11, 11') de la personne observatrice (28) est constitué d'au moins deux premières et de deux autres composantes actives dioptriques ($K_1$, $K_2$, $K_3$, $K_4$), l'une des deux premières composantes actives dioptriques ($K_3$) possédant un effet correcteur pour l'oeil (11, 11') de la personne observatrice (28) pour une distance $A_S$ ≤ 1 m entre un objet (15) et le sommet de la cornée de l'oeil (11, 11') pour une première direction du regard (B) et l'autre des deux premières composantes actives dioptriques ($K_1$) possédant un effet correcteur pour l'oeil (11, 11') de la personne observatrice (28) pour une distance $A_S$ ≥ 4 m entre l'objet (15) et le sommet de la cornée de l'oeil (11, 11') pour une autre direction du regard (A), et l'une des deux deuxièmes composantes actives dioptriques ($K_2$) possédant un effet astigmatique négatif supplémentaire pour l'oeil (11, 11') de la personne observatrice (28) avec une puissance de réfraction de cylindre BK, avec -1,0 DC ≤ BK ≤ -0,125 DC ou -0,7 DC ≤ BK ≤ -0,3 DC ou BK ≈ -0,5 DC et une position d'axe φ indiquée dans le schéma TABO de 70° ≤ φ ≤ 110° ou 80° ≤ φ ≤ 100° ou φ ≈ 90° pour la direction du regard (B) ou une position d'axe φ de -20° ≤ φ ≤ 20° ou -10° ≤ φ ≤ 10° ou φ ≈ 0° pour la direction du regard (B), et l'autre des deux deuxièmes composantes actives dioptriques ($K_3$) possédant un effet astigmatique négatif supplémentaire pour l'oeil (11, 11') de la personne observatrice (28) avec une puissance de réfraction de cylindre BK, avec -1,0 DC ≤ BK ≤ -0,125 DC ou -0,7 DC ≤ BK ≤ -0,3 DC ou BK ≈ -0,5 DC et une position d'axe φ indiquée dans le schéma TABO de 70° ≤ φ ≤ 110° ou 80° ≤ φ ≤ 100° ou φ ≈ 90°, ou une position d'axe φ de -20° ≤ φ ≤ 20° ou -10° ≤ φ ≤ 10° ou φ ≈ 0° pour l'autre direction du regard (A).

**5.** Opération selon la revendication 4, **caractérisée en ce que** l'une des deux premières composantes actives dioptriques ($K_1$) ayant l'effet correcteur pour l'oeil (11, 11') à la distance $A_S$ ≤ 1 m , possède, pour la direction du regard (B), une puissance de réfraction sphérique SBK réduite de la valeur -1,0 D ≤ ΔSBK ≤ -0,1 D en référence à un effet de correction de l'oeil (11, 11') de la personne observatrice (28) pour une distance 25 cm ≤ A$_S$ ≤ 40 cm ou A$_S$ ≈ 33 cm entre un objet (15) et le sommet de la cornée de l'oeil (11, 11').

6. Procédé pour la détermination d'un paramétrage recherché (P$_E$) de l'effet dioptrique constitué de plusieurs composantes actives dioptriques (K$_1$, K$_2$, K$_3$, K$_4$) d'une aide visuelle optique (6) comprenant au moins un verre de lunettes (10) pour un oeil (11, 11') d'une personne observatrice (28),
**caractérisé en ce que**
une puissance de réfraction sphérique et une puissance de réfraction de cylindre et aussi, en option, un effet prismatique sont réglés pour l'oeil (11, 11') de la personne observatrice (28) en insérant des verres d'essai (31) dans des lunettes d'essai (30) puis, à partir de la correction ainsi effectuée de l'oeil (11, 11') de la personne observatrice (28), pour au moins une distance A$_S$ définie entre un objet (15) et le sommet de la cornée de l'oeil (11, 11') et pour au moins une direction du regard (A, B), est déterminé un premier paramétrage (P$_A$) de l'effet dioptrique de l'aide visuelle optique (6), qui a un effet correcteur, sous la forme de l'effet sphérique, de l'effet astigmatique et de sa position d'axe et aussi, en option, de l'effet prismatique et sa base conformément à une première composante active (K$_1$, K$_2$),
le premier paramétrage (P$_A$) déterminé est corrigé d'une autre composante active dioptrique supplémentaire (K$_2$, K$_4$) qui possède un effet astigmatique supplémentaire pour l'au moins une direction du regard (A, B) à la distance définie (A3), et
le premier paramétrage (P$_A$) corrigé étant défini en tant que paramétrage recherché (P$_E$),
l'effet correcteur du premier paramétrage (P$_A$) de l'effet dioptrique de l'aide visuelle désignant la propriété selon laquelle ce paramétrage, pour l'au moins une direction du regard (A, B), produit une correction de l'amétropie de la personne observatrice à une acuité visuelle maximale au moins jusqu'à 1/5 D ou jusqu'à 1/8 D de l'effet sphérique et au moins jusqu'à 1/5 DC ou jusqu'à 1/8 DC de l'effet astigmatique et ±5° de position d'axe,
l'autre effet astigmatique supplémentaire, pour l'au moins une direction du regard (A, B), à la distance A$_S$ définie ne réduisant pas l'acuité visuelle de la personne observatrice de plus de 0,2 logMAR par rapport à l'acuité visuelle qui est obtenue avec la première composante active dioptrique,
la correction pour l'oeil (11, 11') de la personne observatrice (28), pour l'au moins une direction du regard (B), étant une correction pour une distance A$_S$ ≤ 1 m ou 25 cm ≤ A$_S$ ≤ 40 cm ou A$_S$ ≈ 25 cm ou A$_S$ ≈ 33 cm ou A$_S$ ≈ 40 cm entre un objet (15) et le sommet de la cornée de l'oeil (11, 11'), et l'autre composante active dioptrique supplémentaire (K$_4$) possédant un effet astigmatique négatif supplémentaire pour l'oeil (11, 11') de la personne observatrice (28), l'effet astigmatique négatif supplémentaire étant une puissance de réfraction de cylindre BK, avec -1,0 DC ≤ BK ≤ -0,125 DC ou -0,7 DC ≤ BK ≤ -0,3 DC ou BK ≈ -0,5 DC et avec une position d'axe φ indiquée dans le schéma TABO de 70° ≤ φ ≤ 110° ou 80° ≤ φ ≤ 100°, notamment de préférence φ ≈ 90°, ou une position d'axe φ de -20° ≤ φ ≤ 20° ou -10° ≤ φ ≤ 10° ou φ ≈ 0°.

7. Procédé pour la détermination d'un paramétrage recherché (P$_E$) de l'effet dioptrique constitué de plusieurs composantes actives dioptriques (K$_1$, K$_2$, K$_3$, K$_4$) d'une aide visuelle optique (6) comprenant au moins un verre de lunettes (10) pour un oeil (11, 11') d'une personne observatrice (28) au moyen d'un programme informatique comprenant des moyens de code de programme, **caractérisé en ce que**
un premier paramétrage (P$_A$) de l'effet dioptrique de l'aide visuelle optique (6), qui a un effet correcteur, sous la forme de l'effet sphérique, de l'effet astigmatique et de sa position d'axe et aussi, en option, de l'effet prismatique et sa base conformément à une première composante active (K$_1$, K$_2$) est déterminé à partir d'une correction de l'oeil (11, 11') de la personne observatrice (28), pour au moins une distance A$_S$ définie entre un objet (15) et le sommet de la cornée de l'oeil (11, 11') et pour au moins une direction du regard (A, B),
le premier paramétrage (P$_A$) déterminé est corrigé d'une autre composante active dioptrique supplémentaire (K$_2$, K$_4$) qui possède un effet astigmatique supplémentaire pour l'au moins une direction du regard (A, B) à la distance définie (A3), et
le premier paramétrage (P$_A$) corrigé est défini en tant que paramétrage recherché (P$_E$),
l'effet correcteur du premier paramétrage (P$_A$) de l'effet dioptrique de l'aide visuelle désignant la propriété selon laquelle ce paramétrage, pour l'au moins une direction du regard (A, B), produit une correction de l'amétropie de la personne observatrice à une acuité visuelle maximale au moins jusqu'à 1/5 D ou jusqu'à 1/8 D de l'effet sphérique et au moins jusqu'à 1/5 DC ou jusqu'à 1/8 DC de l'effet astigmatique et ±5° de position d'axe,
l'autre effet astigmatique supplémentaire, pour l'au moins une direction du regard (A, B), à la distance A$_S$ définie ne réduisant pas l'acuité visuelle de la personne observatrice de plus de 0,2 logMAR par rapport à l'acuité visuelle qui est obtenue avec la première composante active dioptrique,
la correction pour l'oeil (11, 11') de la personne observatrice (28), pour l'au moins une direction du regard (B), étant une correction pour une distance A$_S$ ≤ 1 m ou 25 cm ≤ A$_S$ ≤ 40 cm ou A$_S$ ≈ 25 cm ou A$_S$ ≈ 33 cm ou A$_S$ ≈ 40 cm entre un objet (15) et le sommet de la cornée de l'oeil (11, 11'), et l'autre composante active dioptrique supplémentaire

($K_4$) possédant un effet astigmatique négatif supplémentaire pour l'oeil (11, 11') de la personne observatrice (28), l'effet astigmatique négatif supplémentaire étant une puissance de réfraction de cylindre BK, avec -1,0 DC ≤ BK ≤ -0,125 DC ou -0,7 DC ≤ BK ≤ -0,3 DC ou BK ≈ -0,5 DC et avec une position d'axe φ indiquée dans le schéma TABO de 70° ≤ φ ≤ 110° ou 80° ≤ φ ≤ 100°, notamment de préférence φ ≈ 90°, ou une position d'axe φ de -20° ≤ φ ≤ 20° ou -10° ≤ φ ≤ 10° ou φ ≈ 0°.

8. Procédé selon la revendication 6 ou 7, **caractérisé en ce que** le premier paramétrage ($P_A$) déterminé est corrigé d'une composante active dioptrique ayant une puissance de réfraction sphérique SBK réduite de la valeur -1,0 D ≤ ΔSBK ≤ -0,1 D en référence à un effet de correction pour l'acuité visuelle maximale de l'oeil (11, 11') pour une distance 25 cm ≤ $A_S$ ≤ 40 cm ou $A_S$ ≈ 33 cm entre un objet (15) et le sommet de la cornée de l'oeil (11, 11') et/ou **caractérisé en ce que** la correction pour l'oeil (11, 11') de la personne observatrice (28) est en plus une correction pour une distance $A_S$ ≥ 4 m entre un objet (15) et le sommet de la cornée de l'oeil (11, 11') pour une autre direction du regard (B) et le premier paramétrage ($P_A$) est également déterminée à partir de celle-ci, et le premier paramétrage ($P_A$) ainsi déterminé est également corrigé d'une composante active dioptrique supplémentaire ($K_3$) et le premier paramétrage ($P_A$) corrigé est défini en tant que paramétrage recherché ($P_E$), la composante active dioptrique supplémentaire ($K_3$) possédant pour l'oeil (11, 11') de la personne observatrice (28) un effet astigmatique négatif supplémentaire avec la puissance de réfraction de cylindre BK, avec -1,0 DC ≤ BK ≤ -0,125 DC ou -0,7 DC ≤ BK ≤ -0,3 DC ou BK ≈ -0,5 DC et avec une position d'axe φ indiquée dans le schéma TABO de 70° ≤ φ ≤ 110° ou 80° ≤ φ ≤ 100°, notamment de préférence φ ≈ 90°, ou une position d'axe φ de -20° ≤ φ ≤ 20° ou -10° ≤ φ ≤ 10° ou φ ≈ 0°.

9. Procédé pour la détermination d'un paramétrage recherché ($P_E$) de l'effet dioptrique constitué de plusieurs composantes actives dioptriques ($K_1$, $K_2$, $K_3$, $K_4$) d'une aide visuelle optique (6) comprenant au moins un verre de lunettes (10) pour un oeil (11, 11') d'une personne observatrice (28),
   **caractérisé en ce que**
   une puissance de réfraction sphérique et une puissance de réfraction de cylindre et aussi, en option, un effet prismatique sont réglés pour l'oeil (11, 11') de la personne observatrice (28) en insérant des verres d'essai (31) dans des lunettes d'essai (30) puis, à partir de la correction ainsi effectuée de l'oeil (11, 11') de la personne observatrice (28), pour au moins une distance $A_S$ définie entre un objet (15) et le sommet de la cornée de l'oeil (11, 11') et pour au moins une direction du regard (A, B), est déterminé un premier paramétrage ($P_A$) de l'effet dioptrique de l'aide visuelle optique (6), qui a un effet correcteur, sous la forme de l'effet sphérique, de l'effet astigmatique et de sa position d'axe et aussi, en option, de l'effet prismatique et sa base conformément à une première composante active ($K_1$, $K_2$),
   le premier paramétrage ($P_A$) déterminé est corrigé d'une autre composante active dioptrique supplémentaire ($K_2$, $K_4$) qui possède un effet astigmatique supplémentaire pour l'au moins une direction du regard (A, B) à la distance définie (A3), et
   le premier paramétrage ($P_A$) corrigé étant défini en tant que paramétrage recherché ($P_E$),
   l'effet correcteur du premier paramétrage ($P_A$) de l'effet dioptrique de l'aide visuelle désignant la propriété selon laquelle ce paramétrage, pour l'au moins une direction du regard (A, B), produit une correction de l'amétropie de la personne observatrice à une acuité visuelle maximale et au moins jusqu'à 1/5 D ou jusqu'à 1/8 D de l'effet sphérique et au moins jusqu'à 1/5 DC ou jusqu'à 1/8 DC de l'effet astigmatique et ±5° de position d'axe,
   l'autre effet astigmatique supplémentaire, pour l'au moins une direction du regard (A, B), à la distance $A_S$ définie ne réduisant pas l'acuité visuelle de la personne observatrice de plus de 0,2 logMAR par rapport à l'acuité visuelle qui est obtenue avec la première composante active dioptrique,
   la correction pour l'oeil (11, 11') de la personne observatrice (28), pour l'au moins une direction du regard (A), étant une correction pour une distance $A_S$ ≥ 4 m entre un objet (15) et le sommet de la cornée de l'oeil (11, 11'), et l'autre composante active dioptrique supplémentaire ($K_3$) possédant un effet astigmatique négatif supplémentaire pour l'oeil (11, 11') de la personne observatrice (28), l'effet astigmatique négatif supplémentaire étant une puissance de réfraction de cylindre BK, avec -1,0 DC ≤ BK ≤ -0,125 DC ou -0,7 DC ≤ BK ≤ -0,3 DC ou BK ≈ -0,5 DC et avec une position d'axe φ indiquée dans le schéma TABO de 70° ≤ φ ≤ 110° ou 80° ≤ φ ≤ 100° ou φ ≈ 90°, ou une position d'axe φ de -20° ≤ φ ≤ 20° ou -10° ≤ φ ≤ 10° ou φ ≈ 0°.

10. Procédé pour la détermination d'un paramétrage recherché ($P_E$) de l'effet dioptrique constitué de plusieurs composantes actives dioptriques ($K_1$, $K_2$, $K_3$, $K_4$) d'une aide visuelle optique (6) comprenant au moins un verre de lunettes (10) pour un oeil (11, 11') d'une personne observatrice (28) au moyen d'un programme informatique comprenant des moyens de code de programme,
   **caractérisé en ce que**
   un premier paramétrage ($P_A$) de l'effet dioptrique de l'aide visuelle optique (6), qui a un effet correcteur, sous la forme de l'effet sphérique, de l'effet astigmatique et de sa position d'axe et aussi, en option, de l'effet prismatique et sa base conformément à une première composante active ($K_1$, $K_2$) est déterminé à partir de la correction de l'oeil (11, 11') de la personne observatrice (28) pour au moins une distance $A_S$ définie entre un objet (15) et le sommet de la cornée de l'oeil (11, 11') et pour au moins une direction du regard (A, B),

le premier paramétrage ($P_A$) déterminé est corrigé d'une autre composante active dioptrique supplémentaire ($K_2$, $K_4$) qui possède un effet astigmatique supplémentaire pour l'au moins une direction du regard (A, B) à la distance définie (A3), et

le premier paramétrage ($P_A$) corrigé est défini en tant que paramétrage recherché ($P_E$),

l'effet correcteur du premier paramétrage ($P_A$) de l'effet dioptrique de l'aide visuelle désignant la propriété selon laquelle ce paramétrage, pour l'au moins une direction du regard (A, B), produit précisément une correction de l'amétropie de la personne observatrice à une acuité visuelle maximale et au moins jusqu'à 1/5 D ou jusqu'à 1/8 D de l'effet sphérique et au moins jusqu'à 1/5 DC ou jusqu'à 1/8 DC de l'effet astigmatique et $\pm 5°$ de position d'axe, l'autre effet astigmatique supplémentaire, pour l'au moins une direction du regard (A, B), à la distance $A_S$ définie ne réduisant pas l'acuité visuelle de la personne observatrice de plus de 0,2 logMAR par rapport à l'acuité visuelle qui est obtenue avec la première composante active dioptrique,

la correction pour l'oeil (11, 11') de la personne observatrice (28), pour l'au moins une direction du regard (A), étant une correction pour une distance $A_S \geq 4$ m entre un objet (15) et le sommet de la cornée de l'oeil (11, 11'), et l'autre composante active dioptrique supplémentaire ($K_3$) possédant un effet astigmatique négatif supplémentaire pour l'oeil (11, 11') de la personne observatrice (28), l'effet astigmatique négatif supplémentaire étant une puissance de réfraction de cylindre BK, avec -1,0 DC $\leq$ BK $\leq$ -0,125 DC ou -0,7 DC $\leq$ BK $\leq$ -0,3 DC ou BK $\approx$ -0,5 DC et avec une position d'axe $\varphi$ indiquée dans le schéma TABO de 70° $\leq \varphi \leq$ 110° ou 80° $\leq \varphi \leq$ 100° ou $\varphi \approx$ 90°, ou de -20° $\leq \varphi \leq$ 20° ou -10° $\leq \varphi \leq$ 10° ou $\varphi \approx$ 0°.

11. Opération selon l'une des revendications 1 à 5,
   **caractérisée par**
   la détermination d'un paramétrage recherché ($P_E$) de l'effet dioptrique constitué de plusieurs composantes actives dioptriques ($K_1$, $K_2$, $K_3$, $K_4$) de l'aide visuelle optique (6) avec un procédé selon l'une des revendications 6 à 10.

12. Fourniture ou calcul ou sélection d'une aide visuelle optique (6) comprenant au moins un verre de lunettes (10) destinée à être utilisée par une personne observatrice (28) pour l'observation d'un objet (15),
   **caractérisée par**
   la détermination d'un paramétrage recherché ($P_E$) d'un effet dioptrique constitué de plusieurs composantes actives dioptriques ($K_1$, $K_2$, $K_3$, $K_4$) de l'aide visuelle optique (6) avec un procédé selon l'une des revendications 6 à 10.

13. Système pour la fabrication ou la fourniture assistée par ordinateur ou le calcul assisté par ordinateur ou la sélection assistée par ordinateur d'une aide visuelle optique (6) comprenant au moins un verre de lunettes (10) destinée à être utilisée par une personne observatrice (28) pour l'observation d'un objet (15)
   **caractérisé par**
   des moyens destinés à accomplir une opération selon l'une des revendications 1 à 5 ou 11.

14. Programme informatique comprenant des moyens de code de programme destinés à mettre en oeuvre le procédé selon la revendication 7 ou la revendication 10 ou la revendication 8, dans la mesure où celle-ci se réfère à la revendication 7.

15. Système (92) de détermination d'un paramétrage recherché ($P_E$) d'une aide visuelle optique (6) d'un oeil (11, 11') d'une personne observatrice (28),
   comprenant un dispositif de mesure (94) pour la détermination d'une meilleure correction possible de l'oeil (11) observant fixement à une distance ($A_S$) prédéfinie ; et
   comprenant une unité de calcul (98) à laquelle peut être acheminée la meilleure correction possible de l'oeil (11), accommodé à une distance ($A_S$) prédéfinie, déterminée par le dispositif de mesure (94) ;
   **caractérisé en ce que**
   l'unité de calcul exécute un programme informatique comprenant des moyens de code de programme un procédé selon la revendication 7 ou la revendication 10 ou la revendication 8, dans la mesure où celle-ci se réfère à la revendication 7, pour la détermination du paramétrage recherché ($P_E$) à partir de la meilleure correction possible acheminée.

Fig.1

Fig.2

Fig.3

Fig.4a

Fig.4b

Fig.5

Fig.6

Fig.7

Fig.8

Fig.9

Fig.10

Fig.11

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- EP 1880663 A1
- EP 0857993 A2
- US 20090210054 A1
- US 20110116037 A1
- EP 0632308 A1
- WO 2010083546 A2
- DE 102007032001 B4

**In der Beschreibung aufgeführte Nicht-Patentliteratur**

- **SAWUSCH M.R. et al.** Optimal Astigmatism to Enhance Depth of Focus after Cataract Surgery. *Ophtalmology,* 1991, vol. 98, 1025
- **G. M. SPITZLBERGER.** *Änderung der optischen Aberrationen des menschlichen Auges durch Laser in situ Keratomileusis,* 2004